# EUROPEAN PATENT APPLICATION

(11) **EP 2 347 653 A2**
(43) Date of publication of application: **27.07.2011**
(21) Application number: 10197065.5
(22) Date of filing: 27.12.2010
(51) Int. Cl.: A01N 37/04, A01N 37/34, A01N 43/40, A01P 7/02, A01P 7/04

(54) **Animal ectoparasite control composition**

(30) Priority: 28.12.2009 JP 2009296828
(71) Applicant: Sumitomo Chemical Company, Limited, Tokyo 104-8260 (JP)
(72) Inventor: Nishiguchi, Naonobu, Takarazuka-shi Hyogo 665-0875 (JP); Hirayama, Takahisa, Toyonaka-shi Osaka 561-0802 (JP); Ikari, Kaori, Nishinomiya-shi Hyogo 662-0871 (JP)
(74) Representative: Vossius & Partner

(57) **Abstract**

The present invention provides an animal ectoparasite control composition containing an insecticidal component and an adipate, and a method of controlling an animal ectoparasite which comprises administering an effective amount of the animal ectoparasite control composition to an animal.

## Description

### FIELD OF THE INVENTION

The present invention relates to an animal ectoparasite control composition and a method of controlling an animal ectoparasite.

### BACKGROUND OF THE INVENTION

Conventionally, as methods for controlling an ectoparasite parasitizing animals, methods which comprise administering an insecticidal component to an animal are known (see JP-A 2003-313104, and JP-A 2007-534714).

### SUMMARY OF THE INVENTION

An object of the present invention is to provide an animal ectoparasite control composition having excellent efficacy.

The present inventors intensively studied, and as a result, found that a composition containing an insecticidal component and an adipic acid ester has an excellent controlling effect against an animal ectoparasite. Thus, the present invention was completed.

The present invention provides:
(1) An animal ectoparasite control composition containing an insecticidal component and an adipate (hereinafter, referred to as the present composition);
(2) The composition according to (1), wherein the adipate is at least one selected from the group consisting of diisopropyl adipate and diisobutyl adipate;
(3) The composition according to (1) or (2), wherein the weight ratio of the insecticidal component to the adipate is 1 _{:} 0.2 to 1 _{:} 500;
(4) The composition according to (1) or (2), wherein the weight ratio of the insecticidal component to the adipate is 1 _{:} 0.2 to 1 : 50;
(5) The composition according to (1) or (2), wherein the weight ratio of the insecticidal component to the adipate is 1 : 1 0.5 to 1 : 1 50;
(6) The composition according to any one of (1) to (5), wherein the total content of the insecticidal component and the adipate is 0.1 to 100% by weight;
(7) The composition according to any one of (1) to (5), wherein the total content of the insecticidal component and the adipate is 10 to 100% by weight;
(8) The composition according to any one of (1) to (5), wherein the total amount of the insecticidal component and the adipate is 30 to 100% by weight;
(9) The composition according to any one of (1) to (8), wherein the insecticidal component is at least one compound selected from the group consisting of halogen-containing organosulfur compounds, pyrethroid compounds, neonicotinoid compounds, organophosphorus compounds, insect growth regulation active compounds, phenylpyrazole compounds, carbamate compounds, and benzoylurea compounds;
(10) The composition according to (9), wherein the halogen-containing organosulfur compound is represented by the following formula (I): 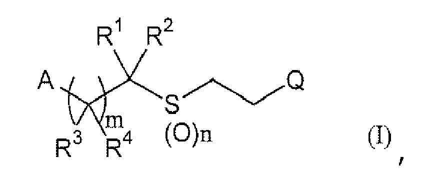 wherein m represents 0 or 1, n represents 0, 1 or 2,
   A represents a C3-C7 cycloalkyl group optionally substituted with a group selected from the groups E1 to E3, or a C5-C7 cycloalkenyl group optionally substituted with a group selected from the groups E1 to E3;
   Q represents a C1-C3 haloalkyl group containing at least one fluorine atom, or a fluorine atom;
   R¹ and R³ are the same as or different from each other, and represent a C1-C4 chain hydrocarbon group optionally substituted with a halogen atom, a halogen atom, or a hydrogen atom;
   R² and R⁴ are the same as or different from each other, and represent a C1-C4 chain hydrocarbon group optionally substituted with a halogen atom, -C(=G)R⁵, a cyano group, a halogen atom, or a hydrogen atom;
   G represents an oxygen atom or a sulfur atom;
   R⁵ represents a C1-C4 alkyl group optionally substituted with a halogen atom, a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkyl)amino group optionally substituted with a halogen atom, a C2-C5 cyclic amino group, or a hydrogen atom;
   the group E1 is a group of monovalent substituents consisting of a C1-C6 chain hydrocarbon group optionally substituted with a group selected from the group L, a C3-C6 cycloalkyl group optionally substituted with a halogen atom, -OR⁶, -SR⁶, -S(=O)R⁶, -S(=O)₂R⁶, -C(=O)R⁷, -OC(=O)R⁸, a halogen atom, a cyano group, and a hydroxyl group;
   the group E2 is a group of bivalent substituents of which two valences are derived from one atom, consisting of =O, =NO-R⁶, =C=CH₂, and =C (R¹¹) R¹²;
   the group E3 is a group of bivalent substituents of which two valences are derived from different atoms, consisting of a C2-C6 alkylene group optionally substituted with a group selected from the group L, a C4-C6 alkenylene group optionally substituted with a group selected from the group L, -G-T¹-G-, and -G-T¹-G-T²-; wherein T¹ and T² are the same as or different from each other, and represent a methylene group or an ethylene group;
   the group L consists of a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, -N (R⁹) R¹⁰, a C2-C5 cyclic amino group, -C(=O)R⁷, -OC (=O) R⁸ and a halogen atom;
   R⁶ represents a C1-C4 chain hydrocarbon group optionally substituted with a halogen atom, or a C3-C6 cycloalkyl group optionally substituted with a halogen atom;
   R⁷ represents a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkyl)amino group optionally substituted with a halogen atom, a C2-C5 cyclic amino group, a C1-C4 alkyl group optionally substituted with a halogen atom, or a hydrogen atom;
   R⁸ represents a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkyl)amino group optionally substituted with a halogen atom, a C2-C5 cyclic amino group, a C1-C4 alkyl group optionally substituted with a halogen atom, or a hydrogen atom;
   R⁹ and R¹⁰ are the same as or different from each other, and represent a C1-C4 alkyl group optionally substituted with a halogen atom, a C3-C6 alkenyl group optionally substituted with a halogen atom, a C3-C6 alkynyl group optionally substituted with a halogen atom, a C3-C6 cycloalkyl group optionally substituted with a halogen atom, a phenyl group optionally substituted with a halogen atom, or a hydrogen atom; and
   R¹¹ and R¹² are the same as or different from each other, and represent a C1-C4 alkoxy group optionally substituted with a halogen atom, a C1-C4 chain hydrocarbon group optionally substituted with a halogen atom, a halogen atom, or a hydrogen atom;
(11) The composition according to (10), wherein m is 0;
(12) The composition according to (10) or (11), wherein n is 2;
(13) The composition according to any one of (10) to (12), wherein R² is a hydrogen atom;
(14) The composition according to any one of (10) to (12), wherein R² is a C1-C4 alkyl group;
(15) The composition according to any one of (10) to (12), wherein R² is a cyano group;
(16) The composition according to any one of (10) to (12), wherein R² is -C (=G) R⁵; G is an oxygen atom; and R⁵ is an amino group, a C1-C4 alkylamino group optionally substituted with a halogen atom, a di(C1-C4 alkyl)amino group optionally substituted with a halogen atom, or a C2-C5 cyclic amino group;
(17) The composition according to any one of (10) to (12), wherein R² is -C(=G)R⁵; G is an oxygen atom; and R⁵ is an amino group;
(18) The composition according to any one of (10) to (17), wherein R¹ is a hydrogen atom or a C1-C4 alkyl group optionally substituted with a halogen atom;
(19) The composition according to any one of (10) to (17), wherein R¹ is a halogen atom;
(20) The composition according to any one of (10) to (19), wherein A is a cyclohexyl group optionally substituted with a group selected from the groups E1 to E3;
(21) The composition according to any one of (10) to (20), wherein the group selected from the groups E1 to E3 is =NOR⁶ and R⁶ is a C1-C4 chain hydrocarbon group optionally substituted with a halogen atom;
(22) The composition according to any one of (10) to (20), wherein A is a cyclohexyl group substituted with one or more of halogen atoms;
(23) The composition according to any one of (1) to (22), which is in the form of a liquid formulation;
(24) The composition according to any one of (1) to (23), which is in the form of an oral formulation, an external formulation for skin, or an injectable formulation;
(25) The composition according to any one of (1) to (24), which is for controlling an animal ectoparasite of Siphonaptera, Anoplura, or Acarina;

(26) A method of controlling an animal ectoparasite, which comprises administering an effective amount of the composition according to any one of (1) to (25) to an animal;
(27) The method according to (26), wherein the administration of the composition to the animal is carried out by a spot-on treatment or a pour-on treatment;
(28) The method according to (26) or (27), wherein the composition according to any one of (1) to (25) is administered in an amount of 1 to 5000 mg per kg of the living body weight of the target animal;
(29) The method according to (26) or (27), wherein the composition according to any one of (1) to (25) is administered in an amount of 10 to 1000 mg per kg of the living body weight of the target animal;
(30) The method according to (26) or (27), wherein the composition according to any one of (1) to (25) is administered in an amount of 50 to 500 mg per kg of the living body weight of the target animal;
(31) The method according to (26) or (27), wherein the target animal is a dog, a cat, a cow, a horse, a pig, or a sheep;
(32) The method according to (26) or (27), wherein the target animal is a dog or a cat; and
(33) The method according to (26) or (27), wherein the target animal is a cow, a horse, a pig, or a sheep.

### DETAILED DESCRIPTION OF THE INVENTION

Examples of an adipate contained in the present composition include bis(2-methoxyethyl) adipate, bis(2-ethoxyethyl) adipate, dimethyl adipate, diethyl adipate, diisopropyl adipate, di-n-butyl adipate, diisobutyl adipate, dioctyl adipate, diisooctyl adipate, diisononyl adipate, diisodecyl adipate, diisooctadecyl adipate, monomethyl adipate, monoethyl adipate, monobenzyl adipate, ethylene glycol adipate, polyethylene glycol adipate, and their mixtures. The adipate is preferably a dibasic acid ester with lower alcohol having 1 to 5 carbon atoms, and specific examples thereof include diisopropyl adipate and diisobutyl adipate, and preferably diisopropyl adipate. In the present composition, the weight ratio of the insecticidal component to the adipate is usually 1 : 0.1 to 1 : 1000, preferably 1 : 0.2 to 1 : 1 500, more preferably 1 : 0.2 to 1 : 50, and even more preferably 1 : 0.5 to 1 : 50.

As the insecticidal component contained in the present composition, a known insecticidal component can be used.
Examples of the insecticidal component include halogen-containing organosulfur compounds, pyrethroid compounds, neonicotinoid compounds, organophosphorus compounds, insect growth regulation active compounds, phenylpyrazole compounds, carbamate compounds, and benzoylurea compounds.

Examples of the halogen-containing organosulfur compound include compounds described in JP-A 2009-1550, JP-A 2009-1551, JP-A 2009-1552, and JP-A 2009-256302 and can be produced by a method described in these publications.
Examples of the pyrethroid compound include metofluthrin, fenpropathrin, permethrin, allethrin, d-allethrin, prallethrin, cyphenothrin, phenothrin, resmethrin, empenthrin, fenvalerate, cyhalothrin, cyfluthrin, etofenprox, tralomethrin, esbiothrin, transfluthrin, terallethrin and profluthrin.
Examples of the neonicotinoid compound include acetamiprid, nitenpyram, thiacloprid, thiamethoxam, imidacloprid, dinotefuran and clothianidin.
Examples of the insect growth regulation active compound include pyriproxyfen, methoprene, fenoxycarb, hydroprene, diflubenzuron, chlorfluazuron, triflumuron, flufenoxuron, hexaflumuron, cyromazine and lufenuron.
Examples of the organophosphorus compound include fenitrothion, naled, fenthion, ciafos, chlorpyrifos, diazinon, calcrofos, salithion, tetrachlorvinphos and dichlorvos.
Examples of the phenylpyrazole compound include acetoprole, ethiprole, fipronil, vaniliprole, pyriprole and pyrafluprole.
Examples of the carbamate compound include alanycarb, bendiocarb, benfuracarb, BPMC, carbaryl, carbofuran, carbosulfan, cloethocarb, ethiofencarb, fenobucarb, fenothiocarb, fenoxycarb, furathiocarb, isoprocarb, metolcarb, methomyl, methiocarb, NAC, oxamyl, pirimicarb, propoxur, XMC, thiodicarb, xylylcarb and aldicarb.
Examples of the benzoylurea compound include bistrifluron, diafenthiuron, fluazuron, flucycloxuron, novaluron, noviflumuron, teflubenzuron and triazuron.

Preferable examples of the halogen-containing organosulfur compound include halogen-containing organosulfur compounds represented by the following formula (I): 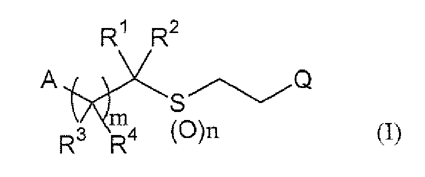 wherein m represents 0 or 1, n represents 0, 1 or 2,
A represents a C3-C7 cycloalkyl group optionally substituted with a group selected from the groups E1 to E3, or a C5-C7 cycloalkenyl group optionally substituted with a group selected from the groups E1 to E3;
Q represents a C1-C3 haloalkyl group containing at least one fluorine atom, or a fluorine atom;
R¹ and R³ are the same as or different from each other, and represent a C1-C4 chain hydrocarbon group optionally substituted with a halogen atom, a halogen atom, or a hydrogen atom;
R² and R⁴ are the same as or different from each other, and represent a C1-C4 chain hydrocarbon group optionally substituted with a halogen atom, -C (=G) R⁵, a cyano group, a halogen atom, or a hydrogen atom;
G represents an oxygen atom or a sulfur atom;
R⁵ represents a C1-C4 alkyl group optionally substituted with a halogen atom, a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di (C1-C4 alkyl)amino group optionally substituted with a halogen atom, a C2-C5 cyclic amino group, or a hydrogen atom;
the group E1 is a group of monovalent substituents consisting of a C1-C6 chain hydrocarbon group optionally substituted with a group selected from the group L, a C3-C6 cycloalkyl group optionally substituted with a halogen atom, -OR⁶, -SR⁶, -S(=O)R⁶, -S(=O)₂R⁶, -C(=O)R⁷, -OC(=O)R⁸, a halogen atom, a cyano group, and a hydroxyl group;
the group E2 is a group of bivalent substituents of which two valences are derived from one atom, consisting of =O, =NO-R⁶, =C=CH₂, and =C(R¹¹)R¹²_{;}
the group E3 is a group of bivalent substituents of which two valences are derived from different atoms, consisting of a C2-C6 alkylene group optionally substituted with a group selected from the group L, a C4-C6 alkenylene group optionally substituted with a group selected from the group L, -G-T¹-G-, and -G-T¹-C-T²-; wherein T¹ and T² are the same as or different from each other, and represent a methylene group or an ethylene group;
the group L consists of a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, -N(R⁹)R¹⁰, a C2-C5 cyclic amino group, -C(=O)R⁷, -OC(=O)R⁸ and a halogen atom;
R⁶ represents a C1-C4 chain hydrocarbon group optionally substituted with a halogen atom, or a C3-C6 cycloalkyl group optionally substituted with a halogen atom;
R⁷ represents a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkyl)amino group optionally substituted with a halogen atom, a C2-C5 cyclic amino group, a C1-C4 alkyl group optionally substituted with a halogen atom, or a hydrogen atom;
R⁸ represents a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di (C1-C4 alkyl)amino group optionally substituted with a halogen atom, a C2-C5 cyclic amino group, a C1-C4 alkyl group optionally substituted with a halogen atom, or a hydrogen atom;
R⁹ and R¹⁰ are the same as or different from each other, and represent a C1-C4 alkyl group optionally substituted with a halogen atom, a C3-C6 alkenyl group optionally substituted with a halogen atom, a C3-C6 alkynyl group optionally substituted with a halogen atom, a C3-CF cycloalkyl group optionally substituted with a halogen atom, a phenyl group optionally substituted with a halogen atom, or a hydrogen atom; and
R¹¹ and R¹² are the same as or different from each other, and represent a C1-C4 alkoxy group optionally substituted with a halogen atom, a C1-C4 chain hydrocarbon group optionally substituted with a halogen atom, a halogen atom, or a hydrogen atom.

The halogen-containing organosulfur compound represented by the formula (I) may have isomers. In the present invention, the isomers of the halogen-containing organosulfur compound containing can be used in any ratios.
The "haloalkyl group", as used herein, means an alkyl group substituted with one or more of halogen atoms selected from the group consisting of fluorine, chlorine, bromine and iodine. The expression "C1-C6" or the like, as used herein, means the total number of carbon atoms constituting each substituent group.

The C3-C7 cycloalkyl group of the "C3-C7 cycloalkyl group optionally substituted with a group selected from the groups El to E3" in the formula (I) is a 3- to 7-membered saturated carbocyclic group, and examples thereof include a cyclopropyl group, a cyclobutyl group, a cyclopentyl group, a cyclohexyl group, a cycloheptyl group and a cyclooctyl group.
The C5-C7 cycloalkenyl group of the "C5-C7 cycloalkenyl group optionally substituted with a group selected from the groups E1 to E3" in the formula (I) is a 5- to 7-membered unsaturated carbocyclic group not containing the maximum number of double bonds, and examples thereof include a 1-cyclopentenyl group, a 2-cyclopentenyl group, a 3-cyclopentenyl group, a 1-cyclohexenyl group, a 2-cyclohexenyl group, a 3-cyclohexenyl group, a 1-cycloheptenyl group, a 2-cycloheptenyl group, a 3-cycloheptenyl group and a 4-cycloheptenyl group.

The C3-C7 cycloalkyl group or the C5 to C7 cycloalkenyl group may be substituted with two or more monovalent groups selected from the group E1 at different carbon atoms or the same carbon atom on the ring, and the two or more monovalent substituents selected from the group E1 may be the same as or different from each other. Examples of a cyclohexyl group substituted with two groups selected from the group E1 are shown below. 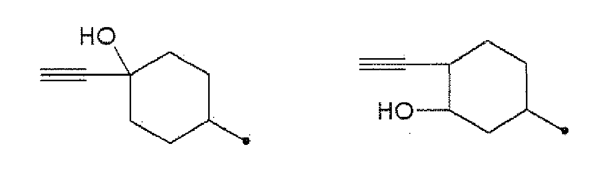 In addition, the C3-C7 cycloalkyl group or the C5 to C7 cycloalkenyl group may be substituted with a bivalent group selected from the group E2 or E3 at different carbon atoms or the same carbon atom on the ring. Examples of a cyclohexyl group substituted with a group selected from the group E3 are shown below.

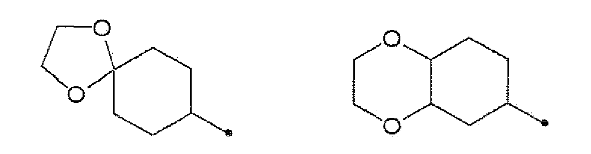

In the group E1, examples of the "C1-C6 chain hydrocarbon group optionally substituted with a group selected from the group L" include a C1-C6 alkyl group optionally substituted with a group selected from the group L, such as a methyl group, a fluoromethyl group, a difluoromethyl group, a trifluoromethyl group, a 2-propynyloxymethyl group, and a 2-butynyloxymethyl group, a hydroxymethyl group; a C2-C6 alkenyl group optionally substituted with a group selected from the group L, such as a vinyl group, a 2,2-difluorovinyl group, and a 1-propenyl group, a 2-propenyl group; and a C2-C6 alkynyl group optionally substituted with a group selected from the group L, such as a 3-methoxy-l-propynyl group, a 3-methoxy-l-butynyl group, a 4-methaxy-1-butynyl group, a 4-methoxy-2-butynyl group, a 3-methoxy-l-pentynyl group, a 4-methoxy-1-pentynyl group, a 5-methoxy-1-pentynyl group, a 4-methoxy-2-pentynyl group, a 5-methoxy-2-pentynyl group, 5-methoxy-3-pentynyl group, a 3-hydroxy-l-propynyl group, a 3-hydroxy-1-butynyl group, a 4-hydroxy-l-butynyl group, a 4-hydroxy-2-butynyl group, a 3-hydroxy-l-pentynyl group, a 4-hydroxy-1-pentynyl group, a 5-hydroxy-l-pentynyl group, a 4-hydroxy-2-pentynyl group, a 5-hydroxy-2-pentynyl group, a 5-hydroxy-3-pentynyl group, a 3-methylamino-l-propynyl group, a 3-methylamino-l-butynyl group, a 4-methylamino-l-butynyl group, a 4-methylamino-2-butynyl group, a 3-methylamino-1-pentynyl group, a 4-methylamino-l-pentynyl group, a 5-methylamino-l-pentynyl group, a 4-methylamino-2-pentynyl group, a 5-methylamino-2-pentynyl group, a 5-methylamino-3-pnetynyl group, a 3-dimethylamino-l-propynyl group, a 3-dimethylamino-l-butynyl group, a 4-dimethylamino-1-butynyl group, a 4-dimethylamino-2-butynyl group, a 3-dimethylamino-1-pentynyl group, a 4-dimethylamino-1-pentynyl group, a 5-dimethylamino-l-pentynyl group, a 4-dimethylamino-2-pentynyl group, a 5-diinethylamino-2-pentynyl group, a 5-dimethylamino-3-pentynyl group, a 3-phenylamino-l-propynyl group, a 3-phenylamino-1-butynyl group, a 4-phenylamino-l-butynyl group, a 4-phenylamino-2-butynyl group, a 3-phenylamino-1-pentynyl group, a 4-phenyla-nino-1-pentynyl group, a 5-phenylamino-1-pentynyl group, a 4-phenylamino-2-pentynyl group, a 5-phenylamino-2-pentynyl group, a 5-phenylamino-3-pentynyl group, a 3-methylphenyLamino-l-propynyl group, a 3-methylphenylamino-l-butynyl group, a 4-methylphenylamino-1-butynyl group, a 4-methylphenylamino-2-butynyl group, a 3-methylphenylamino-l-pentynyl group, a 4-methylphenylamino-1-pentynyl group, a 5-methylphenylamino-1-pentynyl group, a 4-methylphenylamino-2-pentynyl group, a 5-methylphenylamino-2-pentynyl group, a 5-methylphenylamino-3-pentynyl group, a 3-(1-pyrrolidinyl)-1-propynyl group, a 3-(1-pyrrolidinyl)-1-butynyl group, a 4-(1-pyrrolidinyl)-l-butynyl group, a 4-(1-pyrrolidinyl)-2-butynyl group, a 3-(1-pyrrolidinyl)-1-pentynyl group, a 4-(1-pyrrolidinyl)-l-pentynyl group, a 5-(1-pyrrolidinyl)-1-pentynyl group, a 4-(1-pyrrolidinyl)-2-pentynyl group, a 5-(1-pyrrolidinyl)-2-pentynyl group, a 5-(1-pyrrolidinyl)-3-pentynyl group, a 3-(1-piperidinyl)-1-prapynyl group, a 3-(1-piperidinyl)-1-butynyl group, a 4-(1-piperidinyl)-1-butynyl group, a 4-(1-piperidinyl)-2-butynyl group, a 3-(1-piperidinyl)-1-pentynyl group, a 4-(1-piperidinyl)-1-pentynyl group, a 5-(1-piperidinyl)-1-pentynyl group, a 4-(1-piperidinyl)-2-pentynyl group, a 5-(1-piperidinyl)-2-pentynyl group, a 5-(1-piperidinyl)-3-pentynyl group, a 3-(1-morpholinyl)-1-popynyl group, a 3-(1-morpholinyl)-1-butynyl group, a 4-(l-morpholinyl)-l-butynyl group, a 4-(1-morpholinyl)-2-butynyl group, a 3-(l-morpholinyl)-l-pentynyl group, a 4-(1-morpholinyl)-l-pentynyl group, a 5-(1-morpholinyl)-1-pentynyl group, a 4-(1-morpholinyl)-2-pentynyl group, a 5-(1-morpholinyl)-2-pentynyl group, a 5-(1-morpholinyl)-3-pentynyl group, a 3-methoxycarbony1-1-propynyl group, a 3-methoxycarbonyl-1-butynyl group, a 4-methoxycarbonyl-1-butynyl group, a 4-methoxycarbonyl-2-butynyl group, a 3-methoxycarbonyl-1-pentynyl group, a 4-methoxycarbonyl-1-pentynyl group, a 5-methoxycarbonyl-1-pentynyl group, a 4-methoxycarbonyl-2-pentynyl group, a 5-methoxycarbonyl-2-pentynyl group, a 5-methoxycarbonyl-3-pentynyl group, a 3-dimethylaminocarbonyl-1-propynyl group, a 3-dimethylaminocarbonyl-l-butynyl group, a 4-dimethylaminocarbonyl-1-butynyl group, a 4-dimethylaminocarbonyl-2-butynyl group, a 3-dimethylaminocarbonyl-1-pentynyl group, a 4-dimethylaminocarbonyl-1-pentynyl group, a 5-dimethylaminocarbonyl-1-pentynyl group, a 4-dimethylaminocarbonyl-2-pentynyl group, a 5-dimethylaminocarbonyl-2-pentynyl group, a 5-dimethylaminocarbonyl-3-pentynyl group, a 3-(1-Pyrrolidinyl)carbonyl-1-prapynyl group, a 3-(1-pyrrolidinyl)carbonyl-1-butynyl group, a 4-(1-pyrrolidinyl)carbonyl-l-butynyl group, a 4-(1-pyrrolidinyl)carbonyl-2-butynyl group, a 3-(1-pyrrolidinyl)carbonyl-1-pentynyl group, a 4-(1-pyrrolidinyl)carbonyl-l-pentynyl group, a 5-(1-pyrrolidinyl)carbonyl-1-pentynyl group, a 4-(1-pyrralidinyl)carbanyl-2-pentynyl group, a 5-(1-pyrrolidinyl)carbonyl-2-pentynyl group, a 5-(1-pyrrolidinyl)carbonyl-3-pentynyl group, a 3-(1-piperidinyl)carbonyl-1-propynyl group, a 3-(1-piperidinyl)carbonyl-1-butynyl group, a 4-(1-piperidinyl)carbonyl-1-butynyl group, a 4-(1-piperidirxyl)carbonyl-2-butynyl group, a 3-(1-piperidirzyl)carbonyl-1-pentynyl group, a 4-(1-piperidinyl)carbonyl-1-pentynyl group, a 5-(1-piperidinyl)carbonyl-1-pentynyl group, a 4-(1-piperidinyl)carbonyl-2-pentynyl group, a 5-(1-piperidinyl)carbonyl-2-pentynyl group, a 5-(1-piperidinyl)carbonyl-3-pentynyl group, a 3-(1-morpholinyl)carbonyl-1-propynyl group, a 3-(1-morpholiryl)carbonyl-1-butynyl group, a 4-(1-morpholinyl)carbonyl-1-butynyl group, a 4-(1-morpholinyl)carbonyl-2-butynyl group, a 3-(1-morpholinyl)carbonyl-1-pentynyl group, a 4-(1-morpholinyl)carbonyl-1-pentynyl group, a 5-(1-morpholinyl)carbonyl-1-pentynyl group, a 4-(1-morpholinyl)carbonyl-2-pentynyl group, a 5-(1-morpholinyl)carbonyl-2-pentynyl group, a 5-(1-morpholinyl)carbonyl-3-pentynyl group, a 3-carboxy-1 propynyl group, a 3-carboxy-1-butynyl group, a 4-carboxy-1-butynyl group, a 4-carboxy-2-butynyl group, a 3-carboxy-1-pentynyl group, a 4-oarboxy-1-pentynyl group, a 5-carboxy-1-pentynyl group, a 4-carboxy-2-pentynyl group, a 5-carboxy-2-pentynyl group, a 5-carboxy-3-pentynyl group, a 3-acetoxy-1-propynyl group, a 3-acetoxy-1-butynyl group, a 4-acetoxy-1-butynyl group, a 4-acetoxy-2-butynyl group, a 3-acetoxy-1-pentynyl group, a 4-acetoxy-1-pentynyl group, a 5-acetoxy-1-pentynyl group, a 4-acetoxy-2-pentynyl group, a 5-acetoxy-2-pentynyl group, a 5-acetoxy-3-pentynyl group, a 3-methoxycarbonyloxy-1-propynyl group, a 3-methoxycarbonyloxy-1-butynyl group, a 4-methoxycarbonyloxy-1-butynyl group, a 4-methoxycarbonyloxy-2-butynyl group, a 3-methoxycarbonyloxy-1-pentyny group, a 4-methoxycarbonyloxy-1-pentynyl group, a 5-methoxycarbonyloxy-1-pentynyl group, a 4-methoxycarbonyloxy-2-pentynyl group, a 5-methoxycarbonyloxy-2-pentynyl group, a 5-methoxycarbonyloxy-3-pentynyl group, a 2-bromoethynyl group, a 2-iodoethynyl group, a 3-fluoro-1-propynyl group, a 3,3-difluoro-1-propynyl group, a 3,3,3-tridifluoro-1-propynyl group, a 3-fluoro-1-propynyl group, a 3,3-difluoro-l-propynyl group, a 3,3,3-trifluoro-1-propynyl group, a 1-fluoro-2-propynyl group, a 1,1-difluoro-2-propynyl group, a 3-fluoro-l-butynyl group, a 4-fluoro-1-butynyl group, a 3-fluoro-1-pentynyl group, a 4-fluoro-1-pentynyl group, a 5-fluoro-1-pentynyl group, an ethynyl group, a 1-propynyl group, a 2-propynyl group, a 1-butynyl group, a 2-butynyl group, a 3-butynyl group, a 1-pentynyl group, a 2-pentynyl group, and a 3-pentynyl group.

Examples of the "C3-C6 cycloalkyl group optionally substituted with a halogen atom" in the group E1 include a cyclopropyl group.

Examples of a group represented by "-OR⁶" in the group E1 include a C1-C4 alkoxy group optionally substituted with a group selected from the group L, a C2-C6 alkenyloxy group optionally substituted with a group selected from the group L, a C2-C4 alkynyloxy group optionally substituted with a group selected from the group L, and a C3-C6 cycloalkoxy group optionally substituted with a group selected from the group L, and specific examples thereof include a C1-C4 alkoxy group optionally substituted with a halogen atom such as a 2-propynyloxy group, and a 2-butynyloxy group; a C3-C4 alkenyloxy group optionally substituted with a halogen atom; a C3-C4 alkynyloxy group optionally substituted with a halogen atom; and a C3-C6 cycloalkoxy group optionally substituted with a halogen atom.

Examples of a group represented by "-SR⁶" in the group E1 include a C1-C4 alkylthio group optionally substituted with a group selected from the group L.
Examples of a group represented by "-S(=O)R⁶" in the group E1 include a C1-C4 alkylsulfinyl group optionally substituted with a group selected from the group L.
Examples of a group represented by "-S(=O)₂R⁶" in the group E1 include a C1-C4 alkylsulfonyl group optionally substituted with a group selected from the group L.

Examples of a group represented by "-C(=O)R⁷" in the group E1 include a group in which R⁷ is a C1-C4 alkyl group optionally substituted with a halogen atom; a group in which R⁷ is a C1-C4 alkoxy group optionally substituted with a halogen atom; a group in which R⁷ is a C3-C6 alkenyloxy group optionally substituted with a halogen atom; a group in which R⁷ is a C3-C6 alkynyloxy group optionally substituted with a halogen atom; a group in which R⁷ is an amino group, a C1-C4 alkylamino group optionally substituted with a halogen atom, or a di(C1-C4 alkyl)amino group optionally substituted with a halogen atom; a group in which R⁷ is a C2-C5 cyclic amino group; a group in which R⁷ is a hydroxyl group; and a group in which R⁷ is a hydrogen atom.

Examples of a group represented by "-OC(=O)R⁸" in the group E1 include a group in which R⁸ is a C1-C4 alkyl group optionally substituted with a halogen atom; a group in which R⁶ is a C1-C4 alkoxy group optionally substituted with a halogen atom; a group in which R⁸ is a C3-C6 alkenyloxy group optionally substituted with a halogen atom; a group in which R⁸ is a C3-C6 alkynyloxy group optionally substituted with a halogen atom; a group in which R⁸ is an amino group, a C1-C4 alkylamino group optionally substituted with a halogen atom, or a di C1-C4 alkyl)amino group optionally substituted with a halogen atom; a group in which R⁸ is a C2-C5 cyclic amino group; and a group in which R⁸ is a hydrogen atom.

The bivalent substituent of the group E2 is a bivalent substituent of which two valences are derived from one atom.
The bivalent substituent of the group E3 is a bivalent substituent of which two valences are derived from different atoms.

In the group E2, examples of the "=NO-R⁶" include a group in which R⁶ is a C1-C4 alkyl group optionally substituted with a group selected from the group L, a group in which R⁶ is a C2-C4 alkenyl group optionally substituted with a group selected from the group L, a group in which R⁶ is a C2-C4 alkynyl group optionally substituted with a group selected from the group L, and a group in which R⁶ is a C3-C6 cycloalkyl group optionally substituted with a group selected from the group L, and specific examples thereof include a methoxyimino group, an ethoxyimino group, an isopropoxyimino group, a cyclopropylimino group, a 2,2,2-trifluoroethoxyimino group, an allylimino group and a 3-propynylimino group.
Examples of the "=C(R¹¹R¹²") in the group E2 include a methylidene group, an ethylidene group, a 1-methylethylidene group, a propylidene group and a dichloromethylidene group.

In the group E3, examples of the "C2-C6 alkylene group optionally substituted with a group selected from the group L" include an ethane-1,2-diyl group, a propane-1,2-diyl group, a propane-1,3-diyl group, a butane-1,4-diyl group, a 2,3-dichlorobutane-1,4-diyl group and a pentane-1,5-diyl group.
In the group E3 examples of the "C4-C6 alkenylene group optionally substituted with a group selected from the group L" include a 2-butene-1,4-diyl group and a 3-pentene-1,5-diyl group.

Examples of a group represented by "G-T¹-G-" in the group E3 include -OCH₂O-, -SCH₂S-, -OCH₂CH₂O- and -SCH₂CH₂S-.
Examples of the group represented by "-G-T¹-G-T²-" in the group E3 include -OCH₂OCH₂-, -SCH₂SCH₂-, -OCH₂CH₂OCH₂-and -SCH₂CH₂SCH₂-.

Examples of the "C1-C5 haloalkyl group containing at least one fluorine atom" include a C1-C5 alkyl group substituted with only fluorine atom(s), such as a fluoromethyl group, a difluoromethyl group, a trifluoromethyl group, a 1,1,2,2,2-pentafluoroethyl group, a 2,2,2-trifluoroethyl group, a 1,1-difluoroethyl group, a 1,1,2,2,3,3,3-heptafluoropropyl group, a 1,1-difluoropropyl group, a 2,2-difluoroprapyl group, a 3,3,3-trifluoropropyl group, a 1,1,2,2,3,3,4,4,4-nonafluorobutyl group, a 1,1-difluorobutyl group, a 2,2-difluorobutyl group, a 1,1,2,2,3,3,4,4,5,5,5-undecafluoropentyl group, a 1,1-difluropentyl group, and a 2,2-difluoropentyl group; a C1-C5 alkyl group substituted with fluorine atom(s) and chlorine atom(s), such as a chlorodifluoromethyl group, a 1,2-dichloro-1,2,2-trifluoroethyl group, a 1,1-dichloro-2,2,2-trifluoroethyl group, a 1-chloro-1,3,3,3-tetrafluoropropyl group, a 2,3-dichloro-2,3,3-trifluoropropyl group, and a 2,2-dchloro-3,3,3-trifluoropropyl group; and a C1-C5 alkyl group substituted with fluorine atom(s) and bromide atom(s), such as a 2,2-dibromo-3,3,3-trifluoropropyl group, a 2-bromo-3,3,3-trifluoropropyl group, a 2,3-dibrnma-3,3-difluaropropyl group, a 3-bromo-3,3--difluarapropyl group, a 1-bromo-1,3,3,3-tetrafluoropropyl group, a 1-brozno-2,2,3,3,3-pentafluoroprapyl group, a 1,3-dibromo-2,2,3,3-tetrafluoropropyl group, a 3-brorno-2,3,3-trifluoropropyl group, a 3-broma-2,2,3,3-tetrafluaraprapyl group, a 2,3-dibromo-2,3,3-trifluoropropyl group, and a 3-bromo-3,3-difluoropropyl group.

Examples of the "C1-C3 haloalkyl group containing at least one fluorine atom" include a C1-C3 alkyl group substituted with only fluorine atom(s), such as a trifluoromethyl group, a 1,1,2,2,2-pentafluoroethyl group, a 1,1-difluoroethyl group, and a 1,1,2,2,3,3,3-heptaflucropropyl group; a C1-C3 alkyl group substituted with fluorine atom(s) and chlorine atom(s), such as a chlorodifluoromethyl group, a 1,2-dichloro-1,2,2-trifluoroethyl group, a 1,1-dichloro-2,2,2-trifluoroethyl group, a 1-chloro-1,3,3,3-tetrafluoropropyl group, a 2,3-dichloro-2,3,3-trifluoropropyl group, and a 2,2-dichloro-3,3,3-trifluoropropyl group; and a C1-C3 alkyl group substituted with fluorine atom(s) and bromine atom(s), such as a 2,2-dibromo-3,3,3-trifluoropropyl group, a 2-bromo-3,3,3-trifluoropropyl group, a 2,3-dibromo-3,3-difluoropropyl group, a 3-bromo-3,3-difluoropropyl group, a 1-bromo-1,3,3,3-tetrafluoropropyl group, a 1-bromo-2,2,3,3,3-pentafluoropropyl group, a 1,3-dibramo-2,2,3,3-tetrafluoropropyl group, a 3-bromo-2,3,3-trifluoropropyl group, a 3-broma-2,2,3,3-tetrafluoropropyl group, a 2,3-dibromo-2,3,3-trifluoropropyl group, and a 3-bromo-3,3-difluoropropyl group.

Examples of the "C1-C5 fluoroalkyl group" include a fluoromethyl group, a trifluoromethyl group, a 1,1,2,2,2-pentafluoroethyl group, a 2,2,2-trifluoroethyl group, a 1,1-difluoroethyl group, a 1,1,2,2,3,3,3-heptafluoropropyl group, a 1,1-difluoropropyl group, a 2,2-difluoropropyl group, a 3,3,3-trifluoraprapyl group, a 1,1,2,2,3,3,4,4,4-nonafluorobutyl group, a 1,1-difluorobutyl group, a 2,2-difluorobutyl group, a 1,1,2,2,3,3,4,4,5,3,5-undecafluoropentyl group, a 1,1-difluoropentyl group and a 2,2-difluoropentyl group.
Examples of the "C1-C3 fluoroalkyl group" include a fluoromethyl group, a trifluoromethyl group, a 1,1,2,2,2-pentafluoroethyl group, a 1,1-difluoroethyl group and a 1,1,2,2,3,3,3-heptafluoropropyl group.

Examples of the "halogen atom" include a fluorine atom, a chlorine atom and a bromine atom.
Examples of the "C1-C4 chain hydrocarbon group optionally substituted with a halogen atom" include a C1-C4 alkyl group optionally substituted with a halogen atom, a C2-C4 alkenyl group optionally substituted with a halogen atom, and a C2-C4 alkynyl group optionally substituted with a halogen atom.

Examples of the "C1-C4 alkyl group optionally substituted with a halogen atom" include a methyl group, an ethyl group, a propyl group, a 1-methylethyl group (hereinafter referred to as an i-propyl group in some cases), a 2,2-dimethylpropyl group, a chloromethyl group, a fluoromethyl group, a difluoromethyl group, a trifluoromethyl group, a 2,2,2-trifluoromethyl group, a 1,1,2,2-tetrafluoroethyl group, a 1,1,2,2,2-pentafluoroethyl group and a 1,1-dimethylethyl group (hereinafter, referred to as a t-butyl group in some cases).

Examples of the "C2-C4 alkenyl group optionally substituted with a halogen atom" include a vinyl group, a 2,2-difluorovinyl group, a 1,2,2-trifluorovinyl group, a 1-propenyl group, a 2-propenyl group, a 3,3-difluoro-2-propenyl group, a 1-methyl-2-propenyl group, a 2-methyl-2-propenyl group, a 1-butenyl group and a 2-butenyl group.
Examples of the "C2-C4 alkynyl group optionally substituted with a halogen atom" include an ethynyl group, a 1-propenyl group, a 3,3,3-trifluoro-l-propynyl group, a 2-propynyl group, a 1-methyl-2-propynyl group, a 1-butynyl group, a 2-butynyl group, and a 3-butynyl group.

Examples of the "C1-C4 alkoxy group optionally substituted with a halogen atom" include a methoxy group, an ethoxy group, a propoxy group, a trifluoromethoxy group, a bromodifluoromethoxy group, a difluoromethoxy group, a chlorodifluoromethoxy group, a pentafluoroethoxy group, a 2,2,2-trifluoroethoxy group and a 1,1,2,2-tetrafluoroethoxy group.
Examples of the "C3-C6 alkenyloxy group optionally substituted with a halogen atom" include a 1-propenyloxy group, a 2-propenyloxy group, a 1-methyl-2-propenyloxy group, a 1,1-dimethyl-2-propenyloxy group and a 2,2-difluoro-2-propenyloxy group.

Examples of the "C3-C6 alkynyloxy group optionally substituted with a halogen atom" include a 2-propynyloxy group, a 1-methyl-2-propynyloxy group, a 1,1-dimethyl-2-propynyloxy group, a 2-butynyloxy group, a 1-methyl-2-butynyloxy group, a 1,1-dimethyl-2-butynyloxy group and a 3,3,3-trifluoro-1-propynyloxy group.
Examples of the "C1-C4 alkylamino group optionally substituted with a halogen atom" include a N-methylamino group, a N-ethylamino group, a N-propylamino group, a N-(1-methylethyl)amino group and a N-(2,2,2-trifluoroethyl)amino group.

Examples of the "di(C1-C4 alkyl)amino group optionally substituted with a halogen atom" include a N,N-dimethylamino group, a N-ethyl-N-methylamino group, a N,N-diethylamino group, a N-methyl-N-propylamino group, a N-ethyl-N-propylamino group, a N,N-dipropylamino group, a N-methyl-N-(l-methylethyl)amino group, a N-ethyl-N-(l-methylethyl)amino group, a N,N-di(1-methylethyl)amino group, a N-methyl-N-(2,2,2-trifluoroethyl)amino group and a N-methyl-N-ethyl-N-(2,2,2-trifluoroethyl)amino group.
Examples of the "C2-C5 cyclic amino group" include a 1-aziridino group, a 1-azetidinyl group, a 1-pyrralidinyl group, a piperidino group, and a morpholino group.

Examples of the "C1-C6 chain hydrocarbon group optionally substituted with a group selected from the group L" include a C1-C6 alkyl group optionally substituted with a group selected from the group L, a C2-C6 alkenyl group optionally substituted with a group selected from the group L, and a C2-C6 alkynyl group optionally substituted with a group selected from the group L.

Examples of the "C1-C6 alkyl group optionally substituted with a group selected from the group L" include a C1-C6 alkyl group optionally substituted with a halogen atom, such as a methyl group, an ethyl group, a propyl group, a 1-methylethyl group, a 2,2-dimethylpropyl group, a chloromethyl group, a fluoromethyl group, a difluoromethyl group, a trifluoromethyl group, a 2,2,2-trifluoroethyl group, a 1,1,2,2-tetraflucroethyl group, a 1,1,2,2,2-pentafluoroethyl group, and a 1,1-dimethylethyl group; a (C1-C4 alkoxy)C1-C4 alkyl group optionally substituted with a halogen atom, such as a methoxymethyl group, an ethoxymethyl group, a 1-methoxyethyl group, a I-ethoxyethyl group, and a trifluoromethoxymethyl group; a (C3-C6 alkenyloxy)C1-C4 alkyl group optionally substituted with a halogen atom, such as a (1-propenyloxy)methyl group, a (2-propenyloxy)methyl group, a (1-methyl-2-propenyloxy)methyl group, a (1,1-dimethyl-2-propenyloxy)methyl group, a (2,2-difluoro-2-propenyloxy)methyl group, a 1-(1-propenyloxy)ethyl group, a 1-(2-propenyloxy ethyl group, a 1-(1-methyl-2-propenyloxy)ethyl group, a 1-(1,1-dimethyl-2-propenyloxy)ethyl group, a 1-(2,2-difluoro-2-propenyloxy)ethyl group, a 2-(1-propenyloxy)ethyl group, a 2-(2-propenyloxy)ethyl group, a 2-(-methyl-2-propenyloxy)ethyl group, a 2-(1,1-dimethyl-2-propenyloxy)ethyl group and a 2-(2,2-difluoro-2-propenylaxy)ethyl group; a (C3-C6 alkynyloxy)C1-C4 alkyl group optionally substituted with a halogen atom such as a (2-propynyloxy)methyl group, a (1-methyl-2-propynyloxy)methyl group, a (1,1-dimethyl-2-propynyloxy)methyl group, a (2-butynyloxy)methyl group, a (1-methyl-2-butynyloxy)methyl group, a (1,1-dimethyl-2-butynyloxy)methyl group, a (3,3,3-trifluoro-1-propynyloxy)methyl group, a 1-(2-propynyloxy)ethyl group, a 1-(1-methyl-2-propynyloxy)ethyl group, a 1-(1,1-dimethyl-2-propynyloxy)ethyl group, a 1-(2-butynyloxy)ethyl group, a 1-(1-methyl-2-butynyloxy)ethyl group, a 1-(1,1-dimethyl-2-butynyloxy)ethyl group, a 1-(3,3,3-trifluoro-1-propynyloxy)ethyl group, a 2-(2-propynyloxy)ethyl group, a 2-(1-methyl-2-prapynylaxy)ethyl group, a 2-(1,1-dimethyl-2-propynyloxy)ethyl group, a 2-(2-butynyloxy)ethyl group, a 2-(1-methyl-2-butynyloxy)ethyl group, a 2-(1,1-dimethyl-2-butynyloxy)ethyl group, and a 2-(3,3,3-trifluoro-1-propynyloxy)ethyl group; and a (hydroxy)C1-C4 alkyl group optionally substituted with a halogen atom, such as a hydroxymethyl group, a 1-hydroxyethyl group, a 1-hydroxy-1-methylethyl group, a 2-hydroxyethyl group, and a 2-hydroxy-1-methylethyl group.

Examples of the "C2-C6 alkenyl group optionally substituted with a group selected from the group L" include a C2-C6 alkenyl group optionally substituted with a halogen atom, such as a vinyl group, a 2,2-difluorovinyl group, a 1,2,2-trifluorovinyl group, a 1-propenyl group, a 2-propenyl group, a 3,3-difluoro-2-propenyl group, and a 1-methyl-2-propenyl group.

Examples of the "C2-C6 alkynyl group optionally substituted with a group selected from the group L" include an ethynyl group, such as a I-ethynyl group, a 2-bromoethynyl group, a 2-iodoethynyl group, and a 2-(methoxycarbonyl)ethynyl group; a 1-propynyl group, such as a 1-propynyl group, a 3-fluoro-1-propynyl group, a 3,3-difluoro-1-propynyl group, a 3-(dimethylamino)-1-propynyl group, a 3,3,3-trifluoro-1-propynyl group, a 3-methoxy-1-propynyl group, and a 3-(methoxycarbonyl)-1-propynyl group; a 2-propynyl group, such as a 2-propynyl group, a 1-fluoro-2-propynyl group and a 1,1-difluoro-2-propynyl group; a 1-butynyl group such as a 1-butynyl group, a 4-fluoro-1-butynyl group, a 4-methoxy-l-butynyl group, a 4-(dimethylamino)-1-butynyl group, and a 4-(methoxycarbonyl)-1-butynyl group; a 2-butynyl group, such as a 2-butynyl group, a 4-fluoro-2-butynyl group, a 4-methoxy-2-butynyl group, a 4-(dimethylamino)-2-butynyl group, and a 4-(methoxycarbonyl)-2-butynyl group; a 3-butynyl group, such as a 3-butynyl group, and a 1,1-difluoro-3-butynyl group; a 1-pentynyl group, such as a 1-pentynyl group, a 5-fluoro-1-pentynyl group, a 5-methoxy-1-pentynyl group, a 5-(dimethylamino)-1-pentynyl group, and a 5-(methoxycarbonyl)-1-pentynyl group; and a 2-pentynyl group, such as a 2-pentynyl group, a 5-fluoro-2-pentynyl group, a 5-methoxy-2-pentynyl group, a 5-(dimethylamino)-2-pentynyl group, and a 5-(methoxycarbonyl)-2-pentynyl group.

Examples of the "C3-C6 cycloalkyl group optionally substituted with a halogen atom" include a cyclopropyl group, a 1-methylcyclopropyl group, a 2,2-dichlorocyclopropyl group, a 2,2-dichloro-1-methylcyclcpropyl group, a 2,2-difluorocyclopropyl group, a 2,2-difluoro-l-methylcyclopropyl group, a cyclobutyl group, a cyclopentyl group and a cyclohexyl group.

Examples of the "phenyl group optionally substituted with a halogen atom" include a phenyl group, a 2-chlorophenyl group, a 3-chlorophenyl group, a 4-chlorophenyl group, a 2,3-dichlorophenyl group, a 2,4-dichlorophenyl group, a 2,5-dichlorophenyl group, a 2,6-dichlorophenyl group, a 3,4-dichlorophenyl group, a 3,5-dichlorophenyl group, a 2-fluorophenyl group, a 3-fluorophenyl group, a 4-fluorophenyl group, a 2,3-difluorophenyl group, a 2,4-difluorophenyl group, a 2,5-difluorophenyl group, a 2,6-difluorophenyl group, a 3,4-difluorophenyl group, a 3,5-difluorophenyl group, a 2-bromophenyl group, a 3-bromophenyl group, a 4-bromophenyl group, a 2,3-dibromophenyl group, a 2,4-dibromophenyl group, a 2,5-dibromophenyl group, a 2,6-dibromophenyl group, a 3,4-dibromophenyl group, and a 3,5-dibromophenyl group.

Abbreviations used herein have the following meanings. Me: methyl group, Et: ethyl group, Bn: benzyl group, Ph: phenyl group, Ts: p-toluenesulfonnyl group, Ac: acetyl group.

Preferable examples of the halogen-containing organosulfur compound represented by the formula (I) include:
a compound represented by the formula (I) wherein m is 0;
a compound represented by the formula (I) wherein n is 2;
a compound represented by the formula (I) wherein R² is a hydrogen atom;
a compound represented by the formula (I) wherein R² is a C1-C4 alkyl group;
a compound represented by the formula (I) wherein R² is a cyano group;
a compound represented by the formula (I) wherein R¹ is a bromine atom;
a compound represented by the formula (I) wherein R² is - C(=G) R⁵, G is an oxygen atom, and R⁵ is an amino group, a C1-C4 alkylamino optionally substituted with a halogen atom, a di(C1-C4 alkyl)amino optionally substituted with a halogen atom or a C2-C5 cyclic amino group;
a compound represented by the formula (I) wherein R² is - C (=G) R⁵, G is an oxygen atom, and R⁵ is an amino group;
a compound represented by the formula (I) wherein R¹ is a hydrogen atom or a C1-C4 alkyl group optionally substituted with a halogen atom;
a compound represented by the formula (I) wherein R¹ is a halogen atom;
a compound represented by the formula (I) wherein A is a cyclohexyl group optionally substituted with a group selected from the groups E1 to E3;
a compound represented by the formula (I) wherein the group selected from the groups E1 to E3 is =NO-R⁶ and R⁶ is a C1-C4 chain hydrocarbon group optionally substituted with a halogen atom; and
a compound represented by the formula (I) wherein A is a cyclohexyl group substituted with one or more of halogen atoms.

Specific examples of the animal ectoparasite control composition of the present invention include:
a composition containing one compound selected from halogen-containing organosulfur compounds (1) to (68) shown in Production Examples described below, and diisopropyl adipate;
a composition containing one compound selected from the halogen-containing organosulfur compounds (1) to (68), and diisobutyl adipate;

a composition containing one compound selected from the halogen-containing organosulfur compounds (1) to (68) and diisopropyl adipate, wherein the weight ratio of the halogen-containing organosulfur compound to the diisopropyl adipate is 1 _{:} 0.2 to 1 _{:} 500;
a composition containing one compound selected from the halogen-containing organosulfur compounds (1) to (68) and diisobutyl adipate, wherein the weight ratio of the halogen-containing organosulfur compound to the diisobutyl adipate is 1 _{:} 0.2 to 1 _{:} 500;

a composition containing one compound selected from the halogen-containing organosulfur compounds (1) to (68) and diisopropyl adipate, wherein the weight ratio of the halogen-containing organosulfur compound to the diisopropyl adipate is 1 : 0.5 to 1 _{:} 50;
a composition containing one compound selected from the halogen-containing organosulfur compounds (1) to (68) and diisobutyl adipate, wherein the weight ratio of the halogen-containing organosulfur compound to the diisobutyl adipate is 1 _{:} 0.5 to 1 : 50;

a composition containing one compound selected from the halogen-containing organosulfur compounds (1) to (68), pyriproxyfen, and diisopropyl adipate;
a composition containing one compound selected from the halogen-containing organosulfur compounds (1) to (68), pyriproxyfen, and diisobutyl adipate;

a composition containing one compound selected from the halogen-containing organosulfur compounds (1) to (68), pyriproxyfen, and diisopropyl adipate, wherein the weight ratio of (halogen-containing organosulfur compound) : (pyriproxyfen) : (diisopropyl adipate) is 1 _{:} 0.005 _{:} 0.2 to 1 _{:} 0.25 _{:} 500;
a composition containing one compound selected from the halogen-containing organosulfur-based compounds (1) to (68), pyriproxyfen, and diisobutyl adipate, wherein the weight ratio of (halogen-containing organosulfur compound) : (pyriproxyfen) : (diisobutyl adipate) is 1 : 0.005 _{:} 0.2 to 1 : 0.25 _{:} 500;

a composition containing one compound selected from the halogen-containing organosulfur compounds (1) to (68), pyriproxyfen, and diisopropyl adipate, wherein the weight ratio of (halogen-containing organosulfur compound) : (pyriproxyfen) : (diisopropyl adipate) is 1 _{:} 0.01 _{:} 0.5 to 1 : 0.015 : 50;
a composition containing one compound selected from the halogen-containing organosulfur-based compounds (1) to (68), pyriproxyfen, and diisobutyl adipate, wherein the weight ratio of (halogen-containing organosulfur compound) : (pyriproxyfen) : (diisobutyl adipate) is 1 : 0.01 _{:} 0.5 to 1 : 0.015 : 50;

a composition containing one compound selected from the halogen-containing organosulfur compounds (1) to (68), and diisopropyl adipate, wherein A is =NO-R⁶ and R⁶ is a C1-C4 chain hydrocarbon group optionally substituted with a halogen atom;
a composition containing one compound selected from the halogen-containing organosulfur compounds (1) to (68), and diisobutyl adipate, wherein A is =NO-R⁶ and R⁶ is a C1-C4 chain hydrocarbon group optionally substituted with a halogen atom;

a composition containing one compound selected from the halogen-containing organosulfur compounds (1) to (68), and diisopropyl adipate, wherein A is =NO-R⁶ and R⁶ is a C1-C4 chain hydrocarbon group optionally substituted with a halogen atom and the weight ratio of the halogen-containing organosulfur compound to the diisopropyl adipate is 1 _{:} 0.2 to 1 : 500;
a composition containing one compound selected from the halogen-containing organosulfur compounds (1) to (68), and diisobutyl adipate, wherein A is =NO-R⁶ and R⁶ is a C1-C4 chain hydrocarbon group optionally substituted with a halogen atom and the weight ratio of the halogen-containing organosulfur compound to the diisobutyl adipate is 1 : 0.2 to 1 _{:} 500;

a composition containing one compound selected from the halogen-containing organosulfur compounds (1) to (68), and diisopropyl adipate, wherein A is =NO-R⁶ and R⁶ is a C1-C4 chain hydrocarbon group optionally substituted with a halogen atom and the weight ratio of the halogen-containing organosulfur compound to the diisopropyl adipate is 1 _{:} 0.5 to 1 : 50;
a composition containing one compound selected from the halogen-containing organosulfur compounds (1) to (68), and diisobutyl adipate, wherein A is =NO-R⁶ and R⁶ is a C1-C4 chain hydrocarbon group optionally substituted with a halogen atom and the weight ratio of the halogen-containing organosulfur compound to the diisobutyl adipate is 1 : 0.5 to 1 _{:} 50;

a composition containing one compound selected from the halogen-containing organosulfur compounds (1) to (68), pyriproxyfen, and diisopropyl adipate, wherein A is =NO-R⁶ and R⁶ is a C1-C4 chain hydrocarbon group optionally substituted with a halogen atom;
a composition containing one compound selected from the halogen-containing organosulfur compounds (1) to (68), pyriproxyfen, and diisobutyl adipate, wherein A is =NO-R⁶ and R⁶ is a C1-C4 chain hydrocarbon group optionally substituted with a halogen atom;

a composition containing one compound selected from the halogen-containing organosulfur compounds (1) to (68), pyriproxyfen, and diisopropyl adipate, wherein A is =NO-R⁶ and R⁶ is a C1-C4 chain hydrocarbon group optionally substituted with a halogen atom and the weight ratio of (halogen-containing organosulfur compound) (pyriproxyfen) : (diisopropyl adipate) is 1 : 0.005 : 0.2 to 1 : 0.25 : 500;
a composition containing one compound selected from the halogen-containing organosulfur compounds (1) to (68), pyriproxyfen, and diisobutyl adipate, wherein A is =NO-R⁶ and R⁶ is a C1-C4 chain hydrocarbon group optionally substituted with a halogen atom and the weight ratio of (halogen-containing organosulfur compound) : (pyriproxyfen) : (diisobutyl adipate) is 1 : 0.005 : 0.2 to 1 _{:} 0.25 _{:} 500;

a composition containing one compound selected from the halogen-containing organosulfur compounds (1) to (68), pyriproxyfen, and diisopropyl adipate, wherein A is =NO-R⁶ and R⁶ is a C1-C4 chain hydrocarbon group optionally substituted with a halogen atom and the weight ratio of (halogen-containing organosulfur compound) : (pyriproxyfen) : (diisopropyl adipate) is 1 _{:} 0.01 : 0.5 to 1 _{:} 0.015 : 50;
a composition containing one compound selected from the halogen-containing organosulfur compounds (1) to (68), pyriproxyfen, and diisobutyl adipate, wherein A is =NO-R⁶ and R⁶ is a C1-C4 chain hydrocarbon group optionally substituted with a halogen atom and the weight ratio of (halogen-containing organosulfur compound) : (pyriproxyfen) : (diisobutyl adipate) is 1 _{:} 0.01 _{:} 0.5 to 1 _{:} 0.015 _{:} 50.

Specific examples of the halogen-containing organosulfur compound represented by the formula (I) include halogen-containing organosulfur compounds (1) to (68) described in the following Reference Production Examples.

### Reference Production Example 1

### Step 1-1

To a solution of 5.45 g of 1,4-dioxaspiro[4.5]decane-8-methanol in 30 ml of pyridine was added 6.64 g of p-toluenesulfonyl chloride, and the mixture was stirred at room temperature for 6 hours. To the reaction mixture was added 100 ml of water, and then extracted with 100 ml of ethyl acetate twice. An organic layer was washed successively with 100 ml of an aqueous 1N hydrochloric acid solution twice, 100 ml of an aqueous saturated sodium hydrogen carbonate solution once, and 100 ml of an aqueous saturated sodium chloride solution once. The organic layer was dried over sodium sulfate, filtered, and then concentrated under reduced pressure. The residue was subjected to silica gel column chromatography to obtain 9.86 g of 1,4-doxaspiro[4.5]dec-8-ylmethyl p-toluenesulfonate represented by the formula: 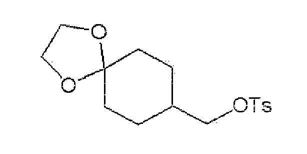 ¹H-NMR (CDCl₃, TMS, δ (ppm) ): 1.17-1.28 (2H, m), 1.43-1.57 (3H, m), 1.71-1.73 (4H, m), 2.45 (3H, S), 3. 83 (2H, d) , 3.88-3.95 (4H, m), 7.33 (2H, d), 7.77 (2H, d) .

### Step 1-2

To a solution of 9.86 g of 1,4-dioxaspiro[4.5]dec-8-ylmethyl p-toluenesulfonate in 40 ml of dimethyl sulfoxide was added 4.00 g of potassium thioacetate, and the mixture was stirred at 70°C for 8 hours. The reaction mixture was cooled to room temperature, and 100 ml of water was added thereto. The reaction mixture was extracted with 100 ml of ethyl acetate twice. An organic layer was washed with 100 ml of an aqueous saturated sodium chloride solution, dried over sodium sulfate, filtered, and then concentrated under reduced pressure. The residue was subjected to silica gel column chromatography to obtain 5.41 g of 1,4-dioxaspiro[4.5]dec-8-ylmethyl thioacetate represented by the formula: 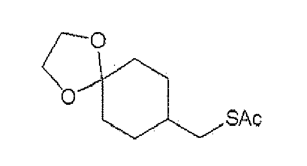 ¹H-NMR (CDCl₃, TMS, δ(ppm)): 1.29-1.36 (2H, m), 1.49-1.52 (3H, m), 1.73-1.80 (4H, m), 2.33 (3H, s), 2.82 (2H, d) , 3.93 (4H, s).

### Step 1-3

To a solution of 5.41 g of 1,4-dioxaspiro[4.5]deca-8-ylmethyl thioacetate in 20 ml of methanol was added 6.75g of a 28% solution of sodium methoxide in methanol at 0°C under a nitrogen atmosphere. To the mixture was added 7.81 g of 3,3,3-trifluoro-1-iodopropane, and the mixture was stirred at room temperature for 1 hour and then at 70°C for 1 hour. After the reaction mixture was cooled to room temperature, 100 ml of water was added and the mixture was concentrated under reduced pressure. An aqueous layer was extracted with 100 ml of ethyl acetate twice. An organic layer was washed with 100 ml of an aqueous saturated sodium chloride solution, dried over sodium sulfate, filtered, and then concentrated under reduced pressure. The residue was subjected to silica gel column chromatography to obtain 3.69 g of 8-(3,3,3-trifluoropropylmethyl)-1,4-dioxaspiro[4.5]decane (hereinafter, referred to as the halogen-containing organosulfur compound (1)) represented by the formula: 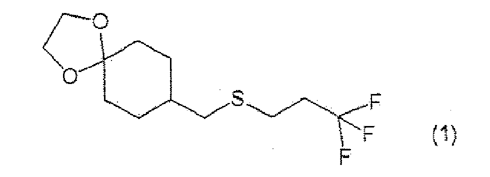 ¹H-NMR (CDCl₃, TMS, δ(ppm)): 1.25-1.35 (2H, m), 1.48-1.58 (3H, m), 1.73-1.79 (2H, m), 1.84-1.88 (2H, m), 2.33-2.43 (2H, m), 2.46 (2H, d), 2.65-2.69 (2H, m), 3.94 (4H, s).

### Reference Production Example 2

To a solution of 3.69 g of the halogen-containing organosulfur compound (1) in 60 ml of chloroform was added 6.56 g of m-chloroperbenzoic acid at 0°C, and the mixture was stirred at room temperature for 1 hour and then at 50°C for 3 hours. The reaction mixture was cooled to 0°C, and 50 ml of a 5% aqueous sodium sulfite solution was added. After the mixture was stirred for 1 hour, an organic layer was separated. An aqueous layer was extracted with 50 ml of chloroform. Organic layers were combined, and washed with 50 ml of an aqueous saturated sodium hydrogen carbonate solution twice and then with 100 ml of an aqueous saturated sodium chloride solution. The resulting organic layer was dried over sodium sulfate, filtered, and then concentrated under reduced pressure. The residue was subjected to silica gel column chromarography to obtain 4.10 g of 8-(3,3,3-trifluoropropylsulfonylmethyl)-1,4-dioxaspiro[4.5]decane (hereinafter, referred to as the halogen-containing organosulfur compound (2)) represented by the formula: 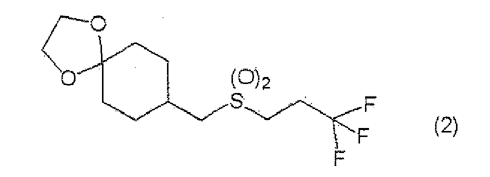 ¹H-NMR (CDC1₃, TMS, δ (ppm) ) : 1.43-1.53 (2H, m), 1.58-1.66 (2H, m), 1.73-1.79 (2H, m), 1.97-2.01 (2H, m), 2.15-2.17 (1H, m), 2.64-2.74 (2H, m), 2.95 (2H, d), 3.16-3.20 (2H, m), 3.92-3.97 (4H, m).

### Reference Production Example 3

To a solution of 4.00 g of the halogen-containing organosulfur compound (2) in 30 ml of acetone was added 0.43 g of toluenesulfonic acid, and the mixture was stirred at 50°C for 8 hours under a nitrogen atmosphere. The reaction solution was concentrated under reduced pressure, and the residue was subjected to silica gel column chromatography to obtain 3.35 g of 4-(3,3,3-trifluoropropylsulfonylmethyl)cyclohexanone (hereinafter, referred to as the halogen-containing organosulfur compound (3)) represented by the formula: 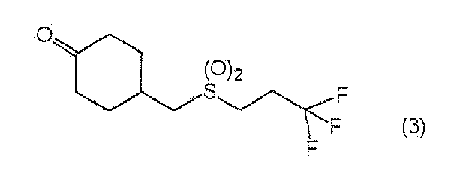 ¹H-NMR, (CDCl₃, TMS, δ (ppm) 1.59-1.70 (2H, m), 2.33-2.36 (2H, m), 2.43-2.46 (4H, m), 2.58-2.67 (1H, m), 2.69-2.73 (2H, m), 3.03 (2H, d), 3.20-3.25 (2H, m).

### Reference Production Example 4

To a solution of 0.82 g of the halogen-containing organosulfur compound (3) in 6 ml of chloroform was added 1.06 g of diethylaminosulfur trifluoride at 0°C under a nitrogen atmosphere, and the mixture was stirred at room temperature for 5 hours. The reaction solution was diluted with 30 ml of chloroform. Thereto 30 ml of water was added, and an organic layer was separated. An aqueous layer was extracted with 30 ml of chloroform twice, and organic layers were combined and washed with 50 ml of an aqueous saturated sodium chloride solution. The resulting organic layer was dried over sodium sulfate, filtered, and then concentrated under reduced pressure. The residue was subjected to silica gel column chromatography to obtain 0.30 g of 1,1-difluoro-4-(3,3,3,-trifluoropropylsulfonylmethyl)cyclohexanone (hereinafter, referred to as the halogen-containing organosulfur compound (4)) and 0.27 g of 1-fluoro-4-(3,3,3,-trifluoropropylsulfonylmethyl) cyclohexanone (hereinafter, referred to as the halogen-containing organosulfur compound (5)), which compounds are represented by the formulas: 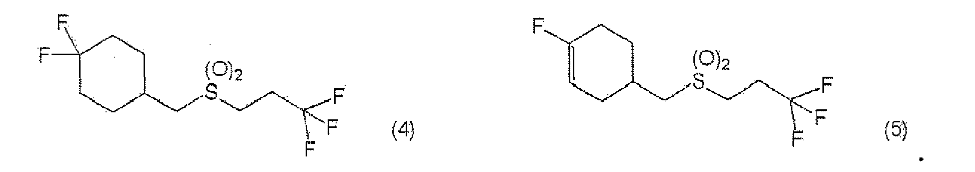 The halogen-containing organosulfur compound (4) : ¹H-NMR (CDC1₃, TMS, δ (ppm) 1.45-1.56 (2H, m), 1.72-1.88 (2H, m), 2.05-2.23 (5H, m), 2.63-2.75 (2H, m), 2.97 (2H, d), 3.02-3.22 (2H, m).
The halogen-containing organosulfur compound (5):
¹H-NMR (CDC1₃, TMS, δ (ppm) ) : 1.65-1.73 (1H, m), 1.99-2.10 (2H, m), 2.19-2.25 (1H, m), 2.33-2.43 (3H, m), 2.63-2.75 (2H, m), 3.02 (2H, d), 3.18-3.22 (2H, m), 5.11-5.19 (1H, m).

### Reference Production Example 5

To a solution of 0.83 g of the halogen-containing organosulfur compound (3) in 10 ml of tetrahydrofuran was added 7.2 ml of a 0.5M solution of ethynylmagnesium bromide in tetrahydrofuran at 0°C under a nitrogen atmosphere, and the mixture was stirred at 0°C for 5 hours. To the reaction solution was added 30 ml of an aqueous IN hydrochloric acid solution, and the mixture was extracted with 30 ml of ethyl acetate twice. Organic layers were combined, and washed with 30 ml of an aqueous saturated sodium hydrogen carbonate solution, and 30 ml of an aqueous saturated sodium chloride solution. The resulting organic layer was dried over sodium sulfate, filtered, and then concentrated under reduced pressure. The residue was subjected to silica gel column chromatography to obtain 0.43 g of 1-ethynyl-4-(3,3,3-trifluoropropyisulfonylmethyl)cyclohexanol (hereinafter, referred to as the halogen-containing organosulfur compound (6)) represented by the formula: 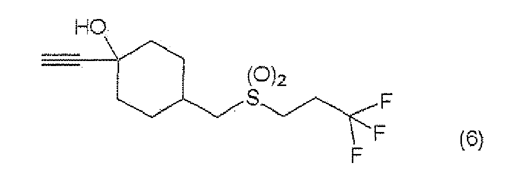 ¹H-NMR (CDCl₃, TMS, δ(ppm)): 1.50-1.59 (4H, m), 2.03-2.11 (6H, m), 2.53 (1H, s), 2.63-2.74 (2H, m), 2.97 (2H, d), 3 .16-3.20 (2H, m).

### Reference Production Example 6

To a solution of 0.29 g of the halogen-containing organosulfur compound (6) in 2 ml of chloroform was added 0.32 g of diethylaminosulfur trifluoride at 0°C under a nitrogen atmosphere, and the mixture was stirred at room temperature for 5 hours. The reaction solution was diluted with 20 ml of chloroform, and 20 ml of water was added thereto. Then an organic layer was separated. An aqueous layer was extracted with 20 ml of chloroform twice. Organic layers were combined, and washed with 50 ml of an aqueous saturated sodium chloride solution. The resulting organic layer was dried over sodium sulfate, filtered, and concentrated under reduced pressure. The residue was subjected to silica gel column chromatography to obtain 0.14 g of 1-ethynyl-1-fluoro-4-(3,3,3-trifluoropropyisulfanylmethyl)cyclohexane (hereinafter, referred to as the halogen-containing organosulfur compound (7)) represented by the formula: 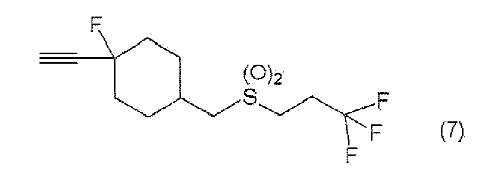 ¹H-NMP,(CDC1₃, TMS, δ (ppm) ): 1.52-1.59 (2H, m), 1.72-1.94 (4H, m), 2.17-2.28 (3H, m), 2.62-2.72 (3H, m), 2.95 (2H, d), 3.17-3.22 (2H, m).

### Reference Production Example 7

### Step 7-1

A solution of 4.86 g of diisopropylamine in 50 ml of tetrahydrofuran was cooled to -50°C under a nitrogen atmosphere. To the solution was added 30 ml of a 1.6M n-butyllithium/n-hexane solution, and stirred at -50°C for 30 minutes. To the solution, a solution of 9.28 g of methyl 2-(1,4-dioxaspiro[4.5]dec-8-yl) acetate in 40 ml of tetrahydrofuran was added dropwise over 15 minutes. The mixture was stirred at 0°C for 30 minutes, and then cooled to -50°C. Thereto a solution of 8.54 g of N-bromosuccinimide in 30 ml of tetrahydrofuran was added, and the mixture was stirred at 0°C for 2 hours and then at room temperature for 5 hours. To the reaction mixture was added 100 ml of water, and an organic layer was separated. An aqueous layer was extracted with 100 ml of ethyl acetate twice. Organic layers were combined, and washed with 100 ml of an aqueous IN hydrochloric acid solution twice, with 100 ml of an aqueous saturated sodium hydrogen carbonate solution, and 100 ml of an aqueous saturated sodium chloride solution. The organic layer was dried over sodium sulfate, filtered, and concentrated under reduced pressure. The residue was dissolved in 50 ml of dimethyl sulfoxide. Thereto 5.04 g of potassium thioacetate was added, and the mixture was stirred at 50°C for 4 hours. After cooling to room temperature, 100 ml of water was added to the reaction mixture, followed by extraction with 100 ml of ethyl acetate twice. Organic layers were combined, washed with 100 ml of an aqueous IN hydrochloric acid solution, 100 ml of an aqueous saturated sodium hydrogen carbonate solution and 100 ml of an aqueous saturated sodium chloride solution, dried over sodium sulfate, filtered, and then concentrated under reduced pressure. The residue was subjected to silica gel column chromatography to obtain 4.27 g of methyl 2-(acetylthio)-2-(1,4-dioxaspiro[4.5]dec-8-yl) acetate represented by the formula: 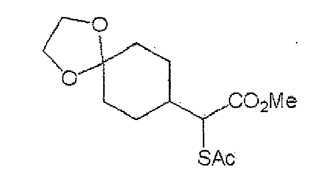 ¹H-NMR (CDCl₃, TMS, δ (ppm) ) : 1.36-1.59 (4H, m), 1.65-1.81 (4H, m), 1.83-1.93 (1H, m), 2.33 (3H, s), 3.73 (3H, s), 3.93 (4H, s), 4.17 (1H d).

### Step-7-2

To a solution of 4.27 g of methyl 2-(acetylthio)-2-(1,4-dioxaspiro[4.5]dec-8-yl)acetate in 30 ml of methanol was added 3.14 g of a 28% solution of sodium methoxide in methanol at 0°C under a nitrogen atmosphere. To the mixture was added 4.31 g of 3,3,3-trifluoro-1-iodopropane, and the mixture was stirred at room temperature for 1 hour and then at 70°C for 1 hours. The reaction mixture was cooled to room temperature, and 100 ml of water was added thereto. The mixture was concentrated to a total amount of about 100 ml under reduced pressure, followed by extraction with 100 ml of ethyl acetate twice. Organic layers were combined, washed with 100 ml of an aqueous saturated sodium chloride solution, dried over sodium sulfate, filtered, and then concentrated under reduced pressure. The residue was subjected to silica gel column chromatography to obtain 3.60 g of methyl 2-(1,4-dioxaspiro[4.5]dec-8-yl)-2-(3,3,3-trifluoropropylthio)acetate (hereinafter, referred to as the halogen-containing organosulfur compound (8)) represented by the formula: 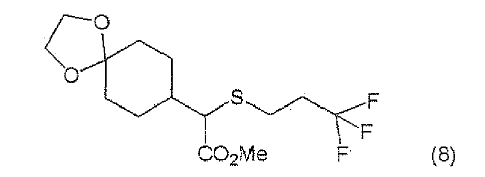 ¹H-NMR (CDCl₃, TMS, δ (ppm) ) : 1.31-1.81 (8H, m), 2.11-2.16 (1H, m), 2.34-2.41 (2H, m), 2.71-2.78 (2H, m), 3.04 (1H, d), 3.75 (3H, s), 4.91-4.96 (4H, m).

### Reference Production Example 8

To a solution of 3.60 g of the halogen-containing organosulfur compound (8) in 20 ml of chloroform was added 4.75 g of m-chloroperbenzoic acid at 0°C under a nitrogen atmosphere. The mixture was stirred at room temperature for 1 hour and then at 50°C for 3 hours. The reaction mixture was cooled to 0°C, and 50 ml of a 5% aqueous sodium sulfite solution was added. The mixture was stirred for 1 hour, and an organic layer was then separated. An aqueous layer was extracted with 50 ml of chloroform, and organic layers were combined and washed with 50 ml of an aqueous saturated sodium hydrogen carbonate solution twice and 100 ml of an aqueous saturated sodium chloride solution. The resulting organic layer was dried over sodium sulfate, filtered, and concentrated under reduced pressure. The residue was subjected to silica gel column chromatography to obtain 2.41 g of methyl 2-(1,4-dioxaspiro[4.5]dec-8-yl)-2-(3,3,3-trifluoropropylsulfonyl)acetate (hereinafter, referred to as the halogen-containing organosulfur compound (9)) represented by the formula: 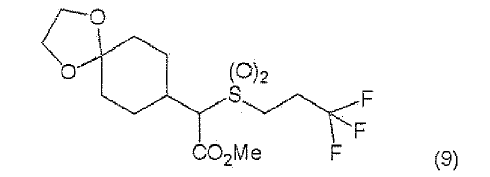 ¹H-NMR (CDCl₃, TMS, δ(ppm)): 1.50-1.83 (7H, m), 2.14-2.17 (1H, m), 2.26-2.29 (1H, m), 2.66-2.74 (2H, m), 3.19-3.27 (1H, m), 3.48-3.53 (1H, m), 3.75 (1H, d), 3.84 (3H, s), 3.91-3.96 (4H, m).

### Reference Production Example 9

To a solution of 2.41 g of the halogen-containing organosulfur compound (9) in 25 ml of acetone was added 0.11 g of toluenesulfonic acid, and the mixture was stirred at 50°C for 8 hours under a nitrogen atmosphere. The reaction mixture was concentrated under reduced pressure. The residue was subjected to silica gel column chromatography to obtain 1.24 g of methyl 2-(4-oxocyclohexyl)-2-(3,3,3-trifluoropropylsulfonyl)acetate (hereinafter referred to as the halogen-containing organosulfur compound (10)) represented by the formula: 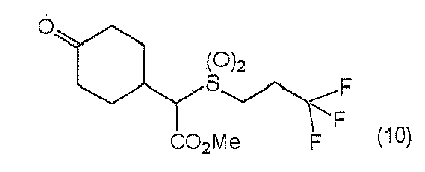 ¹H-NMR (CDC1₃, TMS, δ (ppm) ) : 1.73-1.86 (5H, m) , 2.10-2.13 (1H m), 2.40-2.49 (5H, m), 2.68-2.77 (3H, m), 3.28-3.35 (1H, m), 3.47-3.53 (1H m), 3.85 (2H, d), 3.87 (3H, s).

### Reference Production Example 10

To a solution of 1.24 g of the halogen-containing organosulfur compound (9) in 17 ml of chloroform was added 1.37 g of diethylaminosulfur trifluoride at 0°C under a nitrogen atmosphere, and the mixture was stirred at room temperature for 5 hours. The reaction solution was diluted with 40 ml of chloroform, and 30 ml of water was added thereto. An organic layer was then separated. An aqueous layer was extracted with 30 ml of chloroform twice. Organic layers were combined, and washed with 50 ml of an aqueous saturated sodium chloride solution. The resulting organic layer was dried over sodium sulfate, filtered, and then concentrated under reduced pressure. The residue was subjected to silica gel column chromatography to obtain 0.87 g of methyl 2-(4,4-difluorocyclohexyl)-2-(3,3,3-trifluoropropylsulfonyl)acetate (hereinafter, referred to as the halogen-containing organosulfur compound (11)) represented by the formula: 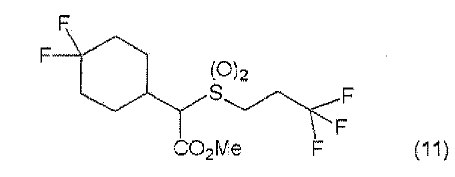 ¹H-NMR (CDC1₃, TMS, δ (ppm) ) : 1.67-1.83 (6H, m), 2.14-2.37 (3H, m), 2.66-2.74 (2H, m), 3.22-3.29 (1H, m), 3.45-3.52 (1H, m), 3.77 (1H, d), 3. 86 (3H, s).

### Reference Production Example 11

To a solution of 0.50 g of the halogen-containing organosulfur compound (11) in 5 ml of tetrahydrofuran was added 0.07 g of 60% sodium hydride at 0°C under a nitrogen atmosphere. Thereto 0.50 g of N-fluorobenzenesulfonimide was further added, and the mixture was stirred at room temperature for 5 hours. To the reaction solution was added 30 ml of water, and an organic layer was separated. An aqueous layer was extracted with 30 ml of ethyl acetate twice. Organic layers were combined, and washed with 50 ml of an aqueous saturated sodium chloride solution. The resulting organic layer was dried over sodium sulfate, filtered, and then concentrated under reduced pressure. The residue was subjected to silica gel column chromatography to obtain 0.43 g of methyl 2-(4,4-difluorocyclohexyl)-2-fluoro-2-(3,3,3-trifluoropropylsulfonyl)acetate (hereinafter referred to as the halogen-containing organosulfur compound (12)) represented by the formula: 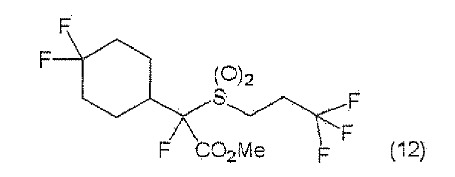 ¹H-NMR (CDC1₃, TMS, δ (ppm) ): 1.67-1.89 (5H, m), 2.12-2.43 (3H, m), 2.55-2.74 (3H, m), 3.21-3.40 (2H, m), 3.98 (3H, s).

### Reference Production Example 12

To a solution of 0.33 g of the halogen-containing organosulfur compound (12) in 3 ml of methanol was added 3 ml of a 2.0M solution of ammonia in methanol at 0°C, and the mixture was then stirred at room temperature for 18 hours. To the reaction solution was added 30 ml of water, and then extracted with 30 ml of ethyl acetate twice. An organic layers were combined, and washed with 50 ml of an aqueous saturated sodium chloride solution. The resulting organic layer was dried over sodium sulfate, filtered, and then concentrated under reduced pressure. The residue was subjected to silica gel column chromatography to obtain 0.43 g of 2-(4,4-difluorocyclohexyl)-2-fluoro-2-(3,3,3-trifluoropropylsulfonyl)acetamide (hereinafter, referred to as the halogen-containing organosulfur compound (13)) represented by the formula: 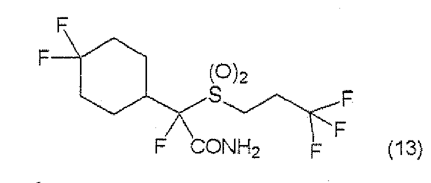 ¹H-NMR (CDC1₃, TMS, δ (ppm) 1.73-1.88 (4H, m), 2.21-2.61 (4H, m), 2.61-2.77 (3H, m), 3.28-3.35 (1H, m), 3.40-3.48 (1H, m), 5.90 (1H, s), 6.53 (1H, s).

### Reference Production Example 13

To a mixture of 22.8 g of cyclohexane-1,4-dimethanol monotosylate (trans/cis=6/4) and 100 ml of dimethyl sulfoxide was added 8.68 g of potassium thioacetate, and the mixture was stirred at room temperature for 1 hour and then at 60°C for 6 hours. After the mixture was cooled to room temperature, thereto 100 ml of an aqueous saturated sodium chloride solution was added and then extracted with 200 ml of t-butyl methyl ether twice. Organic layers were combined, washed with 100 ml of an aqueous saturated sodium chloride solution and 100 ml of water, dried over sodium sulfate, filtered, and then concentrated under reduced pressure. To the residue was added 100 ml of methanol. Under cooling with an ice bath, to the mixture was added dropwise a 28% dilution of 15.43 g of sodium methoxide with 50 ml of methanol over 30 minutes. The mixture was stirred for 30 minutes. To the mixture was added 17.92 g of 3,3,3-trifluoro-1-iodopropane, and then stirred at 60°C for 6 hours. After the reaction mixture was cooled to room temperature, 150 ml of an aqueous saturated sodium chloride solution was added. Methanol was distilled off under reduced pressure. The resulting concentrate was extracted with 200 ml of t-butyl methyl ether twice, and subjected to silica gel column chromatography to obtain 8.46 g of 4-(3,3,3-trifluoropropylthiomethyl)cyclohexanemethanol (hereinafter referred to as the halogen-containing organosulfur compound (14)) represented by the formula: 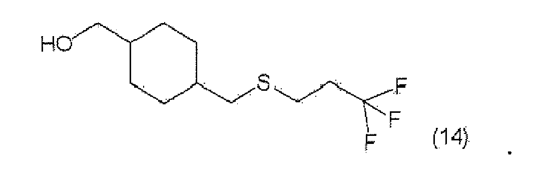 The resulting present compound (14) was a mixture of trans form/cis form = 6/4.
Trans-4-(3,3,3-trifluoropropylthiomethyl)cyclohexanemethanol ¹H-NMR(CDCl₃, TMS, δ(ppm)): 0.96-1.01 (4H, m), 1.40-1.47 (4H, m), 1.58-1.83 (1H, m), 1.92-1.94 (1H, m), 2.34-2.40 (2H, m), 2.43 (2H, d), 2.64-2.68 (2H, m), 3.45 (2H, dd). Cis-4-(3,3,3-trifluoropropylthiomethyl)cyclohexanemethanol ¹H -NMR (CDC1₃, TMS, δ (ppm) ) : 1.22-1.27 (4H, m), 1.40-1.62 (6H, m), 2.34-2.40 (2H, m), 2.52 (2H, d), 2.64-2.68 (2H, m), 3.53 (2H, dd).

### Reference Production Example 14

To a solution of 7.4 g of the halogen-containing organosulfur compound (14) (trans form/cis form = 6/4) in 60 ml of chloroform was added 10.85 g of m-chloroperbenzoic acid at 0°C under a nitrogen atmosphere, and the mixture was stirred at room temperature for 1 hour and then at 50°C for 3 hours. The reaction mixture was cooled to 0°C, and 50 ml of a 5% aqueous sodium sulfite solution was added. The mixture was stirred for 1 hour. An organic layer was separated, and an aqueous layer was extracted with 50 ml of chloroform twice. Organic layers were combined, and washed with 50 ml of an aqueous saturated sodium hydrogen carbonate solution twice, and 100 ml of an aqueous saturated sodium chloride solution. The resulting organic layer was dried over sodium sulfate, filtered, and then concentrated under reduced pressure. The residue was subjected to silica gel column chromatography, and then crystallized from t-butyl methyl ether to obtain 4.13 g of a trans form (hereinafter referred to as the halogen-containing organosulfur compound (15t)) and 3.83 g of a cis form (hereinafter, referred to as the halogen-containing organosulfur compound (15c)) of 4-(3,3,3-trifluoropropylsulfonylmethyl)cyclohexanemethanol (hereinafter, referred to as the halogen-containing organosulfur compound (15)) (trans form/cis form = 1/9), represented by the formula: 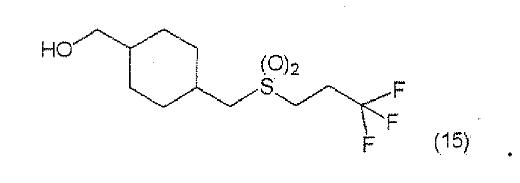 The present compound (15t)
¹H-NMR (CDCl₃, TMS, δ (ppm))1.04-1.19 (4H, m), 1.30 (1H, t), 1.45-1.49 (1H m), 1.84-1.89 (2H, m), 2.04-2.10 (3H, m), 2.62-2.74 (2H, m), 2.93 (2H, d), 3.15-3.19 (2H, m), 3.45 (2H, dd).
The present compound (15c)
¹H-NMR (CDCl₃, TMS, δ(ppm)): 1.04-1.19 (4H, m), 1.30 (1H, t), 1.45-1.49 (1H, m), 1.84-1.89 (2H, m), 2.04-2.10 (3H, m), 2.62-2.74 (2H, m), 2.93 (2H, d), 3.15-3.19 (2H, m), 3.45 (2H, dd).

### Reference Production Example 15

A solution of 7.46 g of oxalyl chloride in 50 ml of dichloromethane was cooled to -78°C under a nitrogen atmosphere. To the solution was added dropwise a solution of 9.53 g of dimethyl sulfoxide in 50 ml of dichloromethane over 20 minutes, and the mixture was stirred at -50°C for 30 minutes. To the reaction mixture was added dropwise a solution of 13.51 g of the halogen-containing organosulfur compound (15t) in 150 ml of dichloromethane over 30 minutes, and then stirred at -50°C for 40 minutes. To the mixture was added dropwise 15.70 g of triethylamine over 40 minutes. The reaction mixture was stirred at room temperature for 18 hours. To the reaction mixture was added 100 ml of water, and an organic layer was separated, followed by extraction with 100 ml of chloroform twice. Organic layers were combined, washed successively with 150 ml of an aqueous 1N hydrochloric acid solution, 150 ml of an aqueous saturated sodium hydrogen carbonate solution, and 150 ml of water, dried over sodium sulfate, filtered and then concentrated under reduced pressure. The residue was subjected to silica gel column chromatography to obtain 10.04 g of a trans form (hereinafter referred to as the halogen-containing organosulfur compound (16t)) of 4-(3,3,3-trifluoropropanesulfonylmethyl)cyclohexane carbaldehyde (hereinafter referred to as the halogen-containing organosulfur compound (16)) represented by the formula: 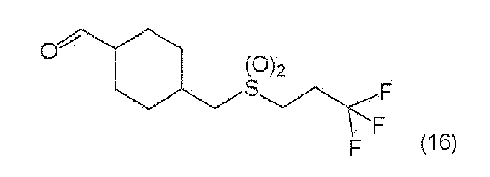 ¹H-NMR(CDCl₃, TMS, δ(ppm)): 1.18-1.27 (2H, m), 1.33-1.43 (2H, m), 2.06-2.24 (6H, m), 2.63-2.74 (2H, m) , 2.95 (2H, d), 3.16-3.21 (2H, m), 9.62 (1H, s).

### Reference Production Example 16

A solution of 23.21 g of carbon tetrabromide in 100 ml of dichloromethane was cooled to 0°C under a nitrogen atmosphere, and 36.72 g of triphenylphosphine was added thereto over 30 minutes. The mixture was stirred for 30 minutes. To the solution was added dropwise a solution of 10.4 g of the halogen-containing organosulfur compound (16t) in 50 ml of dichloromethane over 30 minutes, and then stirred at room temperature for 6 hours. To the reaction mixture was added 150 ml of t-butyl methyl ether. A solid was filtered, and the filtrate was concentrated under reduced pressure. The residue was subjected to silica gel column chromatography to obtain 12.9 g of a trans form (hereinafter, referred to as the halogen-containing organosulfur compound (17t)) of 1.- (2, 2-dibromovinyl) -4-(3,3,3-trifluoropropylsulfonylmethyl)cyclohexane (hereinafter, referred to as the halogen-containing organosulfur compound (17)) represented by the formula: 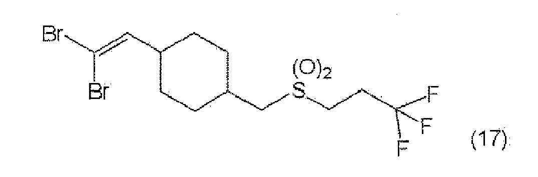 ¹H-NMR(CDC1₃, TMS, δ(ppm) ): 1.16-1.29 (4H, m), 1.84-1.86 (2H, m), 2.04-2.08 (2H, m), 2.21-2.26 (1H, m), 2.62-2.74 (2H, s), 2.92 (2H, d), 3.15-3.22 (2H, m) , 6.19 (2H, d).

### Reference Production Example 17

A solution of 12.90 g of the halogen-containing organosulfur compound (17t) in 60 ml of tetrahydrofuran was cooled to -78°C under a nitrogen atmosphere. To the solution was added dropwise a 1.6M n-butyllithium/n-hexane solution over 30 minutes, and the mixture was stirred at - 50°C for 1 hour and then at 0°C for 2 hours. The reaction mixture was poured into 100 ml of an aqueous IN hydrochloric acid solution which had been cooled with an ice bath, followed by extraction with 200 ml of t-butyl methyl ether twice. Organic layers were combined, washed with 100 ml of an aqueous saturated sodium hydrogen carbonate solution and 100 ml of an aqueous saturated sodium chloride solution, filtered, and then concentrated under reduced pressure. The residue was subjected to silica gel column chromatography to obtain 5.59 g of a trans form (hereinafter, referred to as the halogen-containing organosulfur compound (18t)) of 1-ethynyl-4-(3,3,3-trifluoropropylsulfonylmethyl)cyclohexane (hereinafter, referred to as the halogen-containing organosulfur compound (18)) represented by the formula: 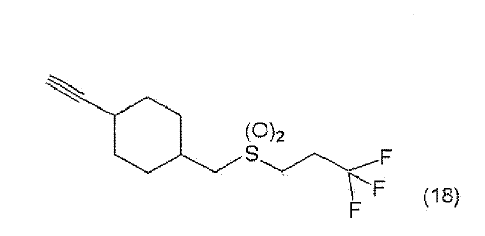 ¹H-NMR (CDC1₃, TMS, δ(ppm)): 1.11-1.20 (2H, m), 1.43-1.53 (2H, m), 2.02-2.13 (6H, m), 2.19-2.23 (1H, m), 2.62-2.73 (2H, m), 2.91 (2H, d), 3.15-3.19 (2H, m).

### Reference Production Example 17

### Step 17-1

A solution of 7.87 g of oxalyl chloride in 50 ml of dichloromethane was cooled to -78°C under a nitrogen atmosphere. To the solution was added dropwise a solution of 4.85 g of dimethyl sulfoxide in 50 ml of dichloromethane over 20 minutes, and the mixture was stirred at -50°C for 30 minutes. To the reaction mixture was added dropwise a solution of 9.28 g of the halogen-containing organosulfur compound (15) (trans form/cis form = 6/4) in 150 ml of dichloromethane over 30 minutes, and then stirred at -50°C for 40 minutes. Thereto 18.22 g of triethylamine was added dropwise over 40 minutes. The reaction mixture was stirred at room temperature for 18 hours. To the reaction mixture was added 100 ml of water, and an organic layer was separated, followed by extraction with 100 ml of chloroform twice. Organic layers were combined, washed successively with 150 ml of an aqueous IN hydrochloric acid solution, 150 ml of an aqueous saturated sodium hydrogen carbonate solution and 150 ml of water, dried over sodium sulfate, filtered, and then concentrated under reduced pressure. The residue was subjected to silica gel column chromatography to obtain 8.41 g of the halogen-containing organosulfur compound (16). The resulting halogen-containing organosulfur compound (16) was a mixture of trans form/cis form = 6/4.

### Step 17-2

A solution of 19.90 g of carbon tetrabromide in 100 ml of dichloromethane was cooled to 0°C under a nitrogen atmosphere. Thereto 31.48 g of triphenylphosphine was added over 30 minutes, and the mixture was stirred for 30 minutes. To the solution was added dropwise a solution of 8.41 g of the halogen-containing organosulfur compound (16) (trans form/cis form =6/4) in 50 ml of dichloromethane over 30 minutes, and the mixture was stirred at room temperature for 6 hours. To the reaction mixture was added 150 ml of t-butyl methyl ether. A solid was filtered and the filtrate was concentrated under reduced pressure. The residue was subjected to silica gel column chromatography to obtain 12.7 g of the halogen-containing organosulfur compound (17). The resulting halogen-containing organosulfur compound (17) was a mixture of trans form/cis form = 6/4.

### Step 17-3

A solution of 12.7 g of the halogen-containing organosulfur compound (17) (trans/cis = 6/4) in 60 ml of tetrahydrofuran was cooled to -78°C under a nitrogen atmosphere. To the solution was added dropwise 40 ml of a 1.6M solution of n-butyllithium in n-hexane over 30 minutes. The mixture was stirred at -50°C for 1 hour and then at 0°C for 2 hours. The reaction mixture was poured into 100 ml of an aqueous IN hydrochloric acid solution which had been cooled with an ice bath, followed by extraction with 200 ml of n-butyl methyl ether twice. Organic layers were combined, washed with 100 ml of an aqueous saturated sodium hydrogen carbonate solution and 100 ml of an aqueous saturated sodium chloride solution, dried over sodium sulfate, filtered and then concentrated under reduced pressure. The residue was subjected to silica gel column chromatography to obtain 1.68 g of a cis form (hereinafter, referred to as the halogen-containing organosulfur compound (18c)) of the halogen-containing organosulfur compound (18). ¹H-NMR (CDC1₃, TMS, δ (ppm)) : 1.58-1.67 (2H, m), 1.82-1.84 (3H, m), 2.01-2.17 (5H, m), 2.62-2.74 (2H, m), 2.78 (1H, br.s), 2.97 (2H, d), 3.15-3.19 (2H, m).

### Reference Production Example 18

A solution of 1.88 g of the halogen-containing organosulfur compound (17) in 10 ml of tetrahydrofuran was cooled to -78°C under a nitrogen atmosphere. To the solution was added dropwise 2.8 ml of a 1.6M solution of n-butyllithium in n-hexane over 10 minutes, and the mixture was stirred at -50°C for 1 hour. Thereto 0.37 g of methyl acrylate was added, and the mixture was stirred at 0°C for 2 hours. The reaction mixture was poured into 30 ml of an aqueous IN hydrochloric acid solution which had been cooled with an ice bath, followed by extraction with 30 ml of t-butyl methyl ether twice. Organic layers were combined, washed with 30 ml of an aqueous saturated sodium hydrogen carbonate solution and 30 ml of an aqueous saturated sodium chloride solution, dried over sodium sulfate, filtered and then concentrated under reduced pressure. The residue was subjected to silica gel column chromatography to obtain 0.70 g of 5-[4-(3,3,3-trifluoropropylsulfonylmethyl)cycohexenyl]-4-pentynic acid methyl ester (hereinafter, referred to as the halogen-containing organosulfur compound (19)) represented by the formula: 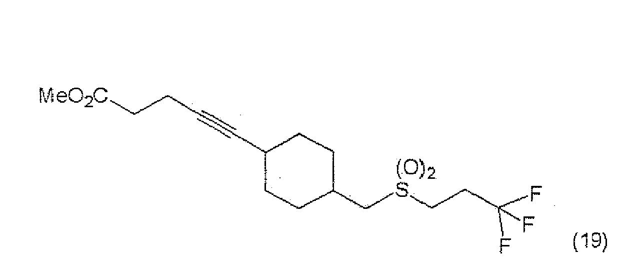 ¹H-NMR (CDC1₃, TMS, δ(ppm)): 1.14-1.20 (2H, m), 1.43-1.56 (2H, m), 2.02-2.22 (8H, m), 2.31-2.40 (2H, m), 2.54-2.60 (1H, m), 2.60-2.71 (1H, m), 2.89-2.96 (3H, m), 3.2a-3.26 (1H, m), 3.70 (3H, s).

### Reference Production Example 19

To 0.57 g of the halogen-containing organosulfur compound (16) were added 0.32 g of pyridine and 0.14 g of hydroxylamine hydrochloride, and the mixture was stirred for 1 hour. To the mixture was added 1 ml of acetic anhydride, and then stirred at 100°C for 2 hours. To the reaction mixture was added 30 ml of an aqueous saturated sodium hydrogen carbonate solution, followed by extraction with 30 ml of ethyl acetate twice. Organic layers were combined, washed with 30 ml of an aqueous saturated sodium chloride solution, dried over sodium sulfate, filtered, and then concentrated under reduced pressure. The residue was subjected to silica gel column chromatography to obtain 0.32 g of 1-cyano-4-(3,3,3-trifluoropropylsulfonylmethyl)cyclorhexane (hereinafter, referred to as the halogen-containing organosulfur compound (20)) represented by the formula: 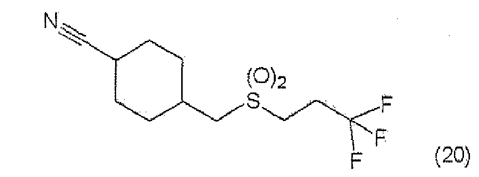 ¹H-NMR (CDCl₃, TMS, δ (ppm)) : 1.14-1.26 (2H, m), 1.63-1.73 (1H m), 2.03-2.43 (7H, m), 2. 64-2. 74 (2H, m), 2.92 (2H, d), 3.16-3.20 (2H, m).

### Reference Production Example 20

To a solution of 0.19 g of the halogen-containing organosulfur compound (3) in 1 ml of pyridine was added 0.08 g of O-methylhydroxylamine hydrochloride, and the mixture was stirred at room temperature for 5 hours. To the reaction solution was added 30 ml of water, followed by extraction with 30 ml of ethyl acetate twice. Organic layers were combined, and washed with 50 ml of an aqueous saturated sodium chloride solution. The resulting organic layer was dried over sodium sulfate, filtered, and then concentrated under reduced pressure. The residue was subjected to silica gel column chromatography to obtain 0.19 g of 4- (3, 3, 3-trifluoropropylsulfonylmethyl)-cyclohexanone O-methyloxime (hereinafter, referred to as the halogen-containing organosulfur compound (21)) represented by the formula: 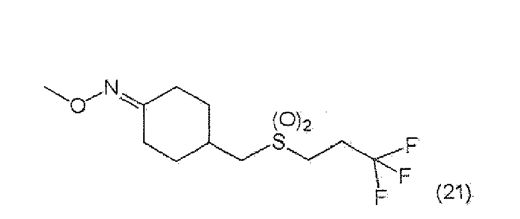 ¹H-NMR (CDCl₃, TMS, δ (ppm) ) : 1.30-1.39 (2H, m), 1.84-1.92 (1H, m), 2.12-2.23 (3H, m), 2.37-2.46 (2H, m), 2.65-2.72 (2H, m), 2.96 (2H, d), 3.17-3.24 (2H, m).

### Reference Production Example 21

To a solution of 0.58 g of the halogen-containing organosulfur compound (7) in 4 ml of tetrahydrofuran was added 2.2 ml of a 0.9M solution of methylmagnesium bromide in tetrahydrofuran at 0°C under a nitrogen atmosphere, and the mixture was stirred at room temperature for 5 hours. To the reaction solution was added 20 ml of water, followed by extraction with 20 ml of ethyl acetate. Organic layers were combined, and washed with 50 ml of an aqueous saturated sodium chloride solution. The resulting organic layer was dried over sodium sulfate, filtered, and then concentrated under reduced pressure. The residue was subjected to silica gel column chromatography to obtain 0.21 g of 1-ethynyl-4-(3,3,3-triflucropropyl-1-sulfonylmethyl)cyclohexene (hereinafter referred to as the halogen-containing organosulfur compound (22)) represented by the formula: 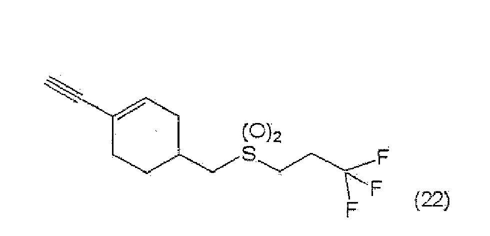 ¹H-NMR (CDCl₃, TMS, δ (ppm)): 1.47-1.61 (1H, m), 1.97-2.06 (2H, m), 2.24-2.30 (2H, m), 2. 92-2. 47 (2H, m), 2. 55-2 .75 (2H, m), 2.84(1H, s), 3.00-3.03 (2H, m), 3.17-3.21 (2H, m), 6.14 (1H, br.s) .

### Reference Production Example 22

A solution of 62.83 g of oxalyl chloride in 250 ml of dichloromethane was was cooled to -78°C under a nitrogen atmosphere. To the solution was added dropwise a solution of 77.35 g of dimethyl sulfoxide in 250 ml of dichloromethane over 60 minutes, and the mixture was stirred at -50°C for 60 minutes. To the reaction mixture was added dropwise a solution of 84.54 g of the halogen-containing organosulfur compound (14) (a mixture of trans form/cis form = 6/4) in 250 ml of dichloromethane, and then stirred at -50°C for 90 minutes. Thereto 100.18 g of triethylamine was added dropwise over 90 minutes. The reaction mixture was stirred at room temperature for 18 hours. To the reaction mixture was added 300 ml of water, and an organic layer was separated. An aqueous layer was then extracted with 200 ml of chloroform twice. Organic layers were combined, washed successively with 300 ml of an aqueous IN hydrochloric acid solution, 300 ml of an aqueous saturated sodium hydrogen carbonate solution and 300 ml of water, dried over sodium sulfate, filtered, and then concentrated under reduced pressure. The residue was subjected to silica gel column chromatography to obtain 23.03 g of a cis form (hereinafter, referred to as the halogen-containing organosulfur compound (23c)) and 38.51 g of a trans form (hereinafter, referred to as the halogen-containing organosulfur compound (23t)) of 4-(3,3,3-trifluoropropylthiomethyl)cyclohexanecarbaldehyde (hereafter, referred to as the halogen-containing organosulfur compound (23)) represented by the formula: 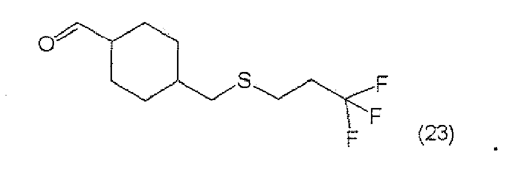 The halogen-containing organosulfur compound (23c): ¹H-NMR (CDCl₃, TMS, δ (ppm)): 1.05-1.16 (2H, m), 1.49-1.66 (3H, m), 1.72-1.80 (2H, m), 2.07-2.16 (2H, m), 2.30-2.47 (5H, m), 2.63-2.67 (2H, m), 9.62 (1H d).
The halogen-containing organosulfur compound (23t) :
¹H-NMR (CDCl₃, TMS, δ (ppm) ) : 0.99-1.10 (2H, m), 1.24-1.34 (2H, m), 1.40-1.53 (1H, m), 1.97-2.08 (4H, m), 2.13-2.25 (1H, m), 2.31-2.43 (2H, m), 2. 46 ( 2H, m), 2.65-2.69 (2H, m), 9.69 (1H d).

### Reference Production Example 23

A solution of 100.48 g of carbon tetrabromide in 300 ml of dichloromethane was cooled to 0°C under a nitrogen atmosphere. Thereto 158.86 g of triphenylphosphine was added over 90 minutes. The mixture was stirred for 30 minutes. To the solution was added dropwise a solution of 38.51 g of the halogen-containing organosulfur compound (23t) in 100 ml of dichloromethane over 30 minutes, and the mixture was stirred at room temperature for 6 hours. To the reaction mixture was added 500 ml of t-butyl methyl ether. A solid was filtered, and the filtrate was concentrated under reduced pressure. The residue was subjected to silica gel column chromatography to obtain 66.56 g of a trans form (hereinafter refereed to as the halogen-containing organosulfur compound (24t)) of 1-(2,2-dibromovinyl)-4-(3,3,3-trifluoropropylthiomethy) cyclohexane (hereinafter, referred to as the halogen-containing organosulfur compound (24)) represented by the formula: 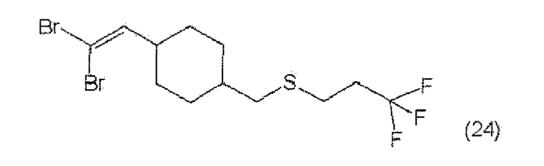 ¹H-NMR (CDCl₃, TMS, δ (ppm) ) : 0.98-1.08 (2H, m), 1.10-1.20 (2H, m), 1.37-1.49 (1H, m), 1.78-1.85 (2H, m), 1.88-1.95 (2H, m), , 2.17-2.29 (1H, m), 2.31-2.41 (2H, m), 2.43 (2H, m), 2.64-2.68 (2H, m), 6.19 (1H, d).

### Reference Production Example 24

A solution of 53.78 g of the halogen-containing organosulfur compound (24t) in 300 ml of tetrahydrofuran was cooled to -78°C under a nitrogen atmosphere. To the solution was added dropwise 180 ml of a 1.6M solution of n-butyllithium in hexane over 60 minutes, and the mixture was stirred at -50°C for 1 hour and then at 0°C for 2 hours. The reaction mixture was poured into 300 ml of an aqueous IN hydrochloric acid solution which had been cooled with an ice bath, followed by extraction with 300 ml of t-butyl methyl ether twice. Organic layers were combined, washed with 300 ml of an aqueous saturated sodium hydrogen carbonate solution and 300 ml of an aqueous saturated sodium chloride solution, dried over sodium sulfate, filtered, and then concentrated under reduced pressure. The residue was subjected to silica gel column chromatography to obtain 31.54 g of a trans form (hereinafter, referred to as the halogen-containing organosulfur compound (25t)) of 1-ethynyl-4-(3,3,3-trifluoropropyl-1-sulfonylmethyl)cyclohexane (hereinafter, referred to as the halogen-containing organosulfur compound (25)) represented by the formula: 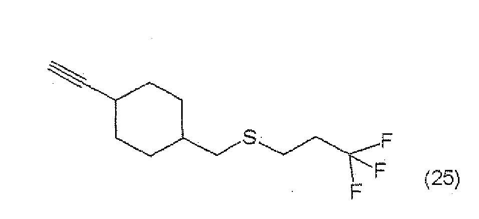 ¹H-NMR(CDCl₃, TMS, δ (ppm) 0.92-1.03 (2H, m), 1.34-1.53 (3H, m), 1.86-1.94 (2H, m), 1.98-2.06 (3H, m), 2.15-2.24 (1H, m), 2.30-2.41 (2H, m), 2.42 (2H, d), 2.64-2.68 (2H, m) .

### Reference Production Example 25

A cis form of the halogen-containing organosulfur compound (25) (hereinafter, referred to as the halogen-containing organosulfur compound (25c)) was produced in the same manner as Reference Production Example 23 and Reference Production Example 24 except that the halogen-containing organosulfur compound (23c) was used in place of the halogen-containing organosulfur compound (23t). ¹H-NMR(CDCl₃, TMS, δ (ppm) 1.33-1.53 (5H, m), 1.67-1.75 (2H, m), 1.78-1.86 (2H, m), 2.05 (1H, d), 2.31-2.44 (2H, m), 2.47 (2H, d), 2.65-2.69 (2H, m), 2.74-2.79 (1H, m).

### Reference Production Example 26

To a suspension of 20.30 g of a double salt of 2KHSOₛ·KHSO₄·K₂SO₄ (Oxone, registered trade mark) in 60 ml of water was added dropwise a solution of 7.51 g of the halogen-containing organosulfur compound (24t) in 60 ml of methanol over 60 minutes at -20°C under a nitrogen atmosphere. The mixture was stirred for 2 hours. To the reaction mixture was added 50 ml of a 10% aqueous sodium sulfite solution, followed by extraction with 100 ml of ethyl acetate twice. Organic layers were combined, washed with 50 ml of a 10% aqueous sodium sulfite solution and 50 ml of an aqueous saturated sodium chloride solution, dried over sodium sulfate, filtered, and then concentrated under reduced pressure. The residue was subjected to silica gel column chromatography to obtain 4.88 g of a trans form (hereinafter, referred to as the halogen-containing organosulfur compound (26t)) of 1-ethynyl-4-(3,3,3-trifluoropropylsulfinylmethyl) cyclohexane (hereinafter, referred to as the halogen-containing organosulfur compound (26)) represented by the formula: 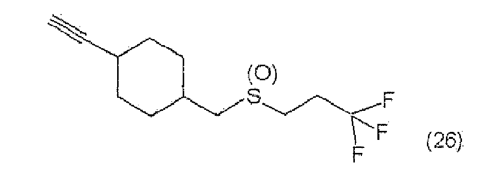 ¹H-NMR (CDCl₃, TMS, δ (ppm) ) : 1.02-1.21 (2H, m), 1.41-1.53 (2H, m), 1.82-2.00 (2H, m), 2.00-2.09 (4H, m), 2.20-2.27 (1H, m), 2.40-2.46 (1H, m), 2.57-2.68 (2H, m), 2.71-2.92 (3H, m).

### Reference Production Example 27

### Step 27-1

To a suspension of 7.35 g of potassium thioacetate in 30 ml of N-methyl-2-pyrrolidone was added dropwise 11.39 g of 3-bromo-1,1,1-trifluoropropane over 15 minutes at 0°C under a nitrogen atmosphere, and the mixture was stirred at room temperature for 1 hour. The reaction mixture was heated to 80°C, followed by distillation under reduced pressure to obtain 9.99 g of 3,3,3-trifluoropropyl thioacetate represented by the formula: 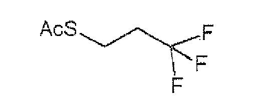 ¹H-NMR (CDCl₃, TMS, δ (ppm) ) : 2.35-2.43 (2H,m), 2.36 (3H, s), 3.01-3.06 (2H, m).
When 3-iodo-1,1,1-trifluoropropane is used in place of 3-bromo-1,1,1-trifluoropropane, 3,3,3-trifluoropropyl thioacetate is obtained in the same manner.

### Step 27-2

A solution of 9.99 g of 3,3,3-trifluoropropyl thioacetate in 60 ml of tetrahydrofuran was cooled to 0°C. Thereto 11.2 g of a 28% solution of sodium methoxide in methanol was added dropwise over 15 minutes, and then stirred at room temperature for 1 hours. To the mixture was added 4.38 g of chloroacetonitrile at 0°C, and then stirred at room temperature for 3 hours. A reaction vessel was cooled in an ice bath. To the reaction mixture was added an aqueous saturated sodium chloride solution, and the mixture was extracted with 100 ml of t-butyl methyl ether twice. Organic layers were combined, washed with an aqueous saturated sodium chloride solution, dried over sodium sulfate, filtered, and then concentrated under reduced pressure. The residue was subjected to silica gel column chromatography to obtain 7.56 g of (3,3,3-trifluoropropylthio) acetonitrile represented by the formula: 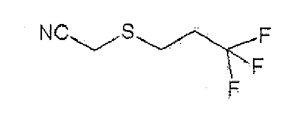 ¹H-NMR (CDCl₃, TMS, δ (ppm) ) : 2.44-2.55 (2H,m), 2.92-2.98 (2H, m), 3.36 (2H, s).

### Step 27-3

To a suspension of 4.97 g of (3,3,3-trifluoropropylthio)acetonitrile and 0.07 g of sodium tungstate dihydrate in 7 ml of water was added 2.3 ml of 31% aqueous hydrogen peroxide while the suspension was stirred. In the middle of the reaction, a part of solids formed in the reaction solution were taken out, purified by thin layer chromatography and then subjected to ¹H-NMR to confirm the formation of (3,3,3-trifluoropropylsulfinyl)acetonitrile. The reaction mixture was heated to 65°C, and 2.3 ml of 31% aqueous hydrogen peroxide was added thereto. The mixture was stirred at 70°C for 1 hour, and then cooled to room temperature. To the mixture was added 5 ml of a 10% aqueous sodium sulfite solution, followed by extraction with 30 ml of ethyl acetate three times. Organic layers were combined, washed with an aqueous saturated sodium chloride solution, dried over sodium sulfate, filtered, and then concentrated under reduced pressure. The residue was crystallized from chloroform: hexane=1:2 to obtain 5.44 g of (3,3,3-trifluoropropylsulfonyl)acetonitrile represented by the formula: 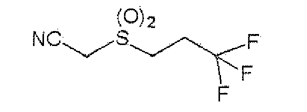 ¹H-NMR (CDCl₃, TMS, δ (ppm)): 2.73-2.85 (2H,m), 3.50-3.56 (2H, m), 4.07 (2H, s).
(3,3,3-Trifluoropropylsulfinyl)acetonitrile: 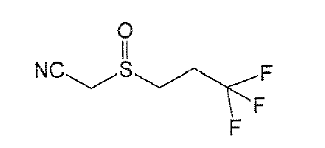 ¹H-NMR (CDCl₃, TMS, δ (pom)): 2.66-2.73 (2H,m), 3.15-3.23 (2H, m), 3.67-3.81 (2H, m).

### Step 27-4

A mixture of 2.01 g of (3,3,3-trifluoropropylsulfonyl)acetonitrile, 50 ml of toluene, 0.12 g of DL-proline and 1.56 g of 1,4-cyclohexanedione monoethylene ketal was heated and stirred for 1 hour under the reflux condition. After 20 ml of toluene was distilled off under normal pressure, the reaction mixture was concentrated under reduced pressure. The residue was subjected to silica gel column chromatography to obtain 1.55 g of 2-(1,4-dioxaspiro[4.5]dec-8-ylidene)-2-(3,3,3-trifluoropropylsulfonyl) acetonitrile. 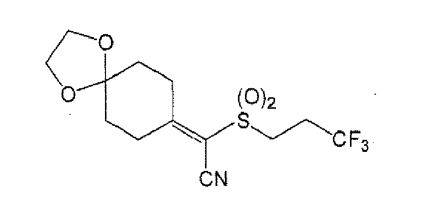 ¹H-NMR (CDCl₃, TMS, δ (ppm)): 1.84-1.90 (2H, m), 1.94-1.97 (2H, m), 2.80-2.89 (2H, m), 2.89-2.92 (2H, m), 3.12-3.16 (2H, m), 3.40-3.44 (2H, m), 4.01 (4H, br.s).

### Step 27-5

To 1.55 g of 2-(1,4-dioxaspiro[4,5]deca-8-ylidene)-2-(3,3,3-trifluoropropylsulfonyl) acetonitrile was added 20 ml of tetrahydrofuran and then cooled to 0°C. Thereto was added 0.19 g of sodium borohydride. The mixture was stirred at room temperature for 6 hours and then cooled to 0°C, and thereto were added 50 ml of water and 50 ml of ethyl acetate. The solution was added dropwise to 50 ml of 1N hydrochloric acid while stirring and extracted with 50 ml of ethyl acetate twice. Organic layers were combined and washed with 50 ml of an aqueous saturated sodium hydrogen carbonate solution and 50 ml of an aqueous saturated sodium chloride solution, dried over sodium sulfate, filtered, and then concentrated under reduced pressure. The residue was subjected to silica gel column chromatography to obtain 1.38 g of 2-(1,4-dioxaspiro[4,5]deca-8-yl)-2-(3,3,3-trifluoropropylsulfonyl)acetonitrile (hereinafter, referred to as the halogen-containing organosulfur compound (27)) represented by the following formula: 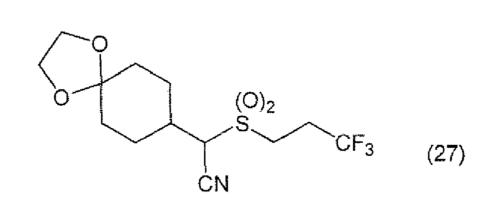 ¹H-NMR (CDCl₃, TMS, δ (ppm)): 1.61-1.90 (7H,m), 2.13-2.23 (1H,m), 2.39-2.51 (1H, m), 2.67-2.86 (2H, m), 3.39-3.47 (1H, m), 3.51-3.60 (1H, m), 3.85 (1H, d), 3.92-3.99 (4H, m).

### Reference Production Example 28

A mixture of 3.20 g of the halogen-containing organosulfur compound (27), 7 ml of acetic acid and 3 ml of water was heated to 70°C and stirred for 10 hours. After the reaction mixture was cooled to room temperature, 100 ml of ethyl acetate was added thereto. The mixture was added slowly into 100 ml of an aqueous saturated sodium hydrogen carbonate solution. The solution was stirred for 1 hour, followed by extraction with 100 ml of ethyl acetate twice. Organic layers were combined, washed with 100 ml of an aqueous saturated sodium hydrogen carbonate solution and 100 ml of an aqueous saturated sodium chloride solution, dried over sodium sulfate, filtered, and concentrated under reduced pressure. The residue was subjected to silica gel column chromatography to obtain 2.59 g of 2-(4-oxocyclohexyl)-2-(3,3,3-trifluoropropylsulfonyl)acetonitrile (hereinafter, referred to as the halogen-containing organosulfur compound (28)) represented by the formula: 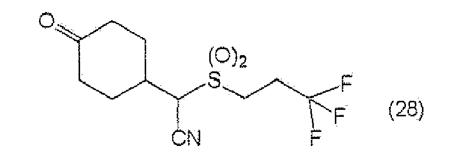 ¹H-NMR (CDCl₃, TMS, δ (ppm) 1.87-1.97 (2H,m), 2.18-2.25 (1H,m) 2.42-2.60 (5H, m) ,2.73-2.95 (3H, m), 3.41-3.51 (1H, m), 3.55-3.66 (1H, m), 3.97 (1H, d).

### Reference Production Example 29

To a solutiono of 0.15 g of the halogen-containing organosulfur compound (28) in 5 ml of dichloromethane was added 0.21 g of diethylaminosulfur trifluoride at -20°C under a nitrogen atmosphere. The mixture was stirred at room temperature for 5 hours. The reaction solution was diluted with 30 ml of chloroform. Thereto 30 ml of water was added, and an organic layer was separated. An aqueous layer was extracted with 30 ml of chloroform twice, and organic layers were combined and washed with 50 ml of an aqueous saturated sodium chloride solution. The resulting organic layer was dried over sodium sulfate, filtered, and then concentrated under reduced pressure. The residue was subjected to silica gel column chromatography to obtain 0.16 g of 2-(4,4-difluorocyclohexyl)-2-(3,3,3-trifluoropropylsulfonyl)acetonitrile (hereinafter, referred to as the halogen-containing organosulfur compound (29)) represented by the formula: 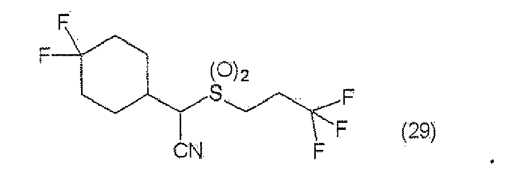 ¹H-NMR (CDCl₃, TMS, δ (ppm) ) : 1.41-1.99 (5H, m), 2.16-2.32 (3H, m), 2.42-2.58 (1H, m), 2.70-2.86 (2H, m), 3.38-3.49 (1H, m), 3.54-3.68 (1H, m), 3.87 (1H, d).

### Reference Production Example 30

To a solution of 1.49 g of the halogen-containing organosulfur compound (28) in 20 ml of tetrahydrofuran was added 30 ml of a 0.5M solution of ethynylmagnesium bromide in tetrahydrofuran at 0°C under a nitrogen atmosphere, and the mixture was stirred at 0°C for 5 hours. To the reaction solution was added 50 ml of an aqueous 1N hydrochloric acid solution, followed by extraction with 50 ml of ethyl acetate twice. Organic layers were combined, and washed with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was subjected to silica gel column chromatography to obtain 1.69 g of 2-(4-ethynyl-4-hydroxycyclohexyl)-2-(3,3,3-trifluoropropylsulfonyl)acetonitrile (hereinafter, referred to as the halogen-containing organosulfur compound (30)) represented by the formula: 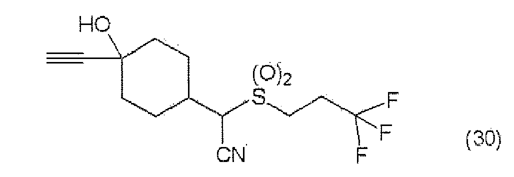 ¹H-NMR (CDCl₃, TMS, δ (ppm) ) : 1.59-2.28 (9H,m), 2.28-2.47 (1H, m), 2.61 (1H, s), 2.71-2.84 (2H, m), 3.40-3.48 (1H, m), 3.52-3.60 (1H, m), 3.87 (1H, d).

### Reference Production Example 31

To a solution of 0.65 g of the halogen-containing organosulfur compound (30) in 6 ml of dichloromethane was added 0.48 g of diethylaminosulfur trifluoride at 0°C under a nitrogen atmosphere, and the mixture was stirred at room temperature for 5 hours. The reaction solution was diluted with 20 ml of chloroform. Thereto 20 ml of water was added, and an organic layer was separated. An aqueous layer was extracted with 20 ml of chloroform twice. Organic layers were combined, and washed with 50 ml of an aqueous saturated sodium chloride solution. The resulting organic layer was dried over sodium sulfate, filtered, and then concentrated under reduced pressure. The residue was subjected to silica gel column chromatography to obtain 0.31 g of 2-(4-ethynyl-4-fluorocyclohexyl)-2-(3,3,3-trifluoropropylsulfonyl)acetonitrile (hereinafter, referred to as the halogen-containing organosulfur compound (31)) and 0.23 g of 2-(4-ethynyl-cyclohexen-3-yl)-2-(3,3,3-trifluoropropylsulfonyl)acetonitrile (hereinafter, referred to as the halogen-containing organosulfur compound (32)), which are represented by the formulas: 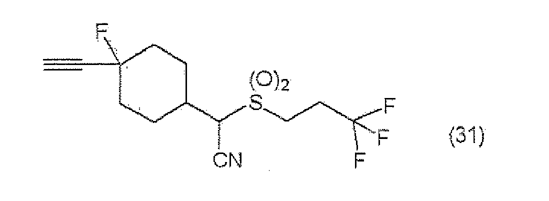 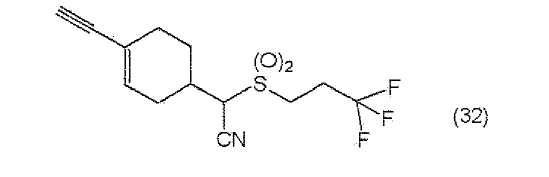 The resulting halogen-containing organosulfur compound (32) was a 1:1 isomer mixture.
The halogen-containing organosulfur compound (31):
¹H-NMR (CDCl₃, TMS, δ (ppm)): 1.73-2.15 (6H,m), 2.25-2.39 (2H, m), 2.44-2.54 (1H, m), 2.66 (1H, d), 2.71-2.84 (2H, m), 3.40-3.48 (1H, m), 3.53-3.61 (1H, m), 3.87 (1H, d).
The halogen-containing organosulfur compound (32):
¹H-NMR (CDCl₃, TMS, δ (ppm) ) : 1.68-2.84 (9H,m), 2.84 (1H, d) 3.39-3.49 (1H, m), 3.51-3.66 (1H m), 3.89 (1H, d), 6.10-6.18 (2H, m).

### Reference Production Example 32

To a solution of 2.81 g of the the halogen-containing organosulfur compound (28) in 10 ml of tetrahydrofuran were added 0.75 g of pyridine and 0.79 g of methoxyamine hydrochloride, and the mixture was stirred at room temperature for 3 hours. To the reaction solution was added 30 ml of an aqueous 1N hydrochloric acid solution, followed by extraction with 50 ml of ethyl acetate twice. Organic layers were combined, washed with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was subjected to silica gel column chromatography to obtain 2.86 g of 2-[4-(methoxyimino) cyclohexyl]-2-(3,3,3-trifluoropropylsulfonyl)acetonitrile (hereinafter, referred to as the halogen-containing organosulfur compound (33)) represented by the formula: 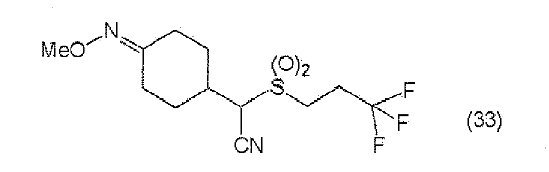 ¹H-NMR (CDCl₃, TMS, δ (ppm) ) : 1.50-1.71 (2H,m), 1.80-1.91 (1H, m), 1.95-2.08 (1H, m), 2.16-2.27 (1H, m), 2.30-2.44 (1H, m), 2.49-2.57 (1H, m), 2.62-2.71 (1H, m), 2.72-2.86 (2H, m), 3.32-3.48 (2H, m), 3.52-3.61 (1H, m), 3.83 (3H, s), 3.87 (1H, d).

### Reference Production Example 33

To a solution of 0.20 g of the halogen-containing organosulfur compound (28) in 2 ml of pyridine was added 0.06 g of hydroxylamine hydrochloride, and the mixture was stirred at room temperature for 3 hours. To the reaction solution was added 50 ml of hexane, followed by concentration under reduced pressure. The residue was subjected to silica gel column chromatography to obtain 0.093 g of 2-[4-(hydroxyimino)cyclohexyl]-2-(3,3,3-trifluoropropylsulfonyl)acetonitrile (hereinafter, referred to as the halogen-containing organosulfur compound (34)) represented by the formula: 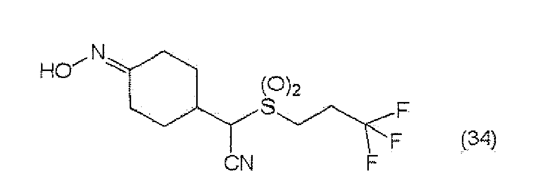 ¹H-NMR (CD₃OD, TMS, δ (ppm)): 1.29-1.48 (2H, m), 1.69-1.85 (1H, m), 1.87-2.01 (1H, m), 2.07-2.27 (2H, m), 2.29-2.38 (1H, m), 2.51-2.59 (1H, m), 2.67-2.81 (2H, m), 3.18-3.22 (1H, m), 3.26-3.34 (2H, m), 4.47 (1H, br.s).

### Reference Production Example 34

According to Reference Production Example 33 except that 0.008 g of ethoxyamine hydrochloride was used in place of hydroxylamine hydrochloride, 0.095 g of 2-[4-(ethoxyimino)cyclohexyl]-2-(3,3,3-trifluoropropylsufonyl)acetonitrile (hereinafter, referred to as the halogen-containing organosulfur compound (35)) represented by the formula: 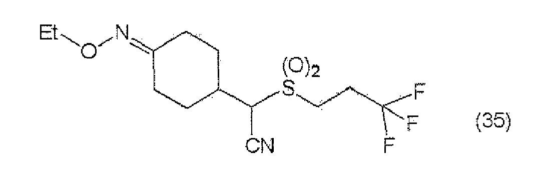 was obtained.
¹H-NMR (CDCl₃, TMS, δ (ppm) ) : 1.25 (3H, t), 1.51-1.72 (2H, m), 1.79-1.92 (1H, m), 1.95-2.07 (1H m), 2.17-2.27 (1H, m), 2.31-2.43 (1H, m), 2.49-2.59 (1H m), 2.61-2.71 (1H, m), 2.72-2.86 (2H, m), 3.35-3.49 (2H, m), 3.51-3.61 (1H, m), 3.85 (1H d), 4.05 (2H, q).

### Reference Production Example 35

According to Reference Production Example 33 except that 0.01 g of t-butoxyamine hydrochloride was used in place of hydroxylamine hydrochloride, 0.19 g of 2-[4-(t-butoxyimino)cyclohexyl]-2-(3,3,3-trifluoropropylsulfonyl)acetonitrile (hereinafter, referred to as the halogen-containing organosulfur compound (36)) represented by the formula: 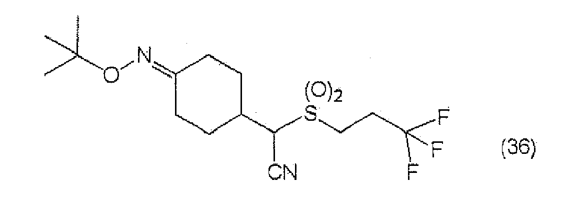 was obtained.
¹H-NMR (CDCl₃, TMS, δ (ppm) 1.26 (9H, s), 1.49-1.70 (2H, m), 1.75-1.88 (1H, m), 1.91-2.06 (1H, m), 2.14-2.25 (1H, m), 2.27-2.42 (1H, m), 2.51-2.59 (1H, m), 2.59-2.70 (1H, m), 2.71-2.88 (2H, m), 3.35-3.48 (2H, m), 3.52-3.68 (1H, m), 3.86-3.88 (1H, m).

### Reference Production Example 36

According to Reference Production Example 33 except that 0.009 g of O-allylhydroxylamine hydrochloride was used in place of hydroxylamine hydrochloride, 0.061 g of 2-[4-(O-allylhydroxylimino)cyclohexyl)-2-(3,3,3-trifluoropropylsulfonyl)acetonitrile (hereinafter, referred to as the halogen-containing organosulfur compound (37)) represented by the formula: 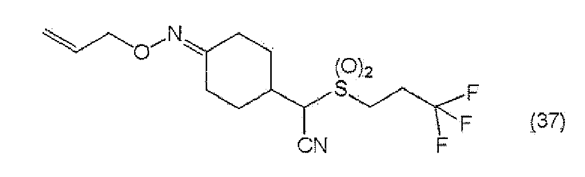 was obtained.
¹H-NMR (CDCl₃, TMS, δ (ppm) ) : 1.52-1.72 (2H, m), 1.79-1.94 (1H, m), 1.96-2.09 (1H, m), 2.16-2.28 (1H, m), 2.31-2.42 (1H, m), 2.50-2.59 (1H m), 2.61-2.72 (1H, m), 2.72-2.86 (2H, m), 3.38-3.48 (2H, m), 3.52-3.61 (1H, m), 3.85-3.88 (1H, m), 4.52-4.55 (2H, m), 5.19-5.33 (2H, m), 5.92-6.04 (2H, m).

### Reference Production Example 37

According to Reference Production Example 33 except that 0.009 g of O-benzylhyroxylamine hydrochloride was used in place of hydroxylaminde hydrochloride, 0.10 g of 2-[4-(0-benzylhydroxylimino)cyclohexyl]-2-(3,3,3-trifluoropropylsulfonyl)acetonitrile (hereinafter, referred to as the halogen-containing organosulfur compound (38)) represented by the formula: 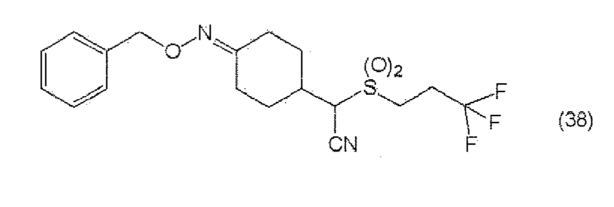 was obtained.
¹H-NMR (CDCl₃, TMS, δ (ppm)): 1.49-1.72 (2H, m), 1.81-2.08 (2H, m), 2.16-2.27 (1H, m), 2.30-2.43 (1H, m), 2.50-2.58 (1H, m), 2.61-2.70 (1H, m), 2.72-2.83 (2H, m), 3.38-3.48 (2H, m), 3.51-3.61 (1H, m), 3.84-3.87 (1H, m), 5.07 (2H, s), 7.28-7.34 (1H, m), 7.35-7.36 (4H, m).

### Reference Production Example 38

To a solution of 0.20 g of the halogen-containing organosulfur compound (28) in 2 ml of pyridine was added 0.06 g of O-carboxymethylhydroxylamine hydrochloride, and the mixture was stirred at room temperature for 3 hours. To the reaction solution was added 30 ml of an aqueous 1N hydrochloric acid solution, followed by extraction with 30 ml of ethyl acetate twice. The resulting organic layer was dried over sodium chloride, filtered, and then concentrated under reduced pressure. The residue was subjected to silica gel column chromatography to obtain 0.094 g of 2-[4-(O-carboxymethylhydroxylimino)cyclohexyl]-2-(3,3,3-trifluoropropylsulfonyl)acetonitrile (hereinafter, referred to as the halogen-containing organosulfur compound (39)) represented by the formula: 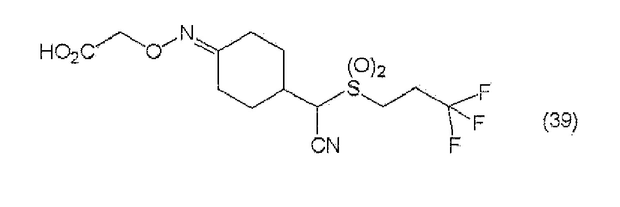 ¹H-NMR (CDCl₃, TMS, δ (ppm)): 1.56-1.72 (2H, m), 1.88-2.11 (2H, m), 2.17-2.29 (1H m), 2.31-2.43 (1H, m), 2.47-2.56 (1H, m), 2.60-2.70 (1H, m), 2.73-2.84 (2H, m), 3.38-3.43 (4H, m), 4.57 (2H, s).

### Reference Production Example 39

A solution of 0.16 g of the halogen-containing organosulfur compound (33) in 3 ml of dimethyl sulfoxide was cooled to 0°C under a nitrogen atmosphere. To the mixture was added 0.05 g of 60% sodium hydride dispersion in paraffin liquid, and the mixture was stirred for 30 minutes. Thereto was added 0.11 g of methyl iodide, and the mixture was stirred at room temperature overnight. To the reaction solution was added 10 ml of an aqueous 1N hydrochloric acid solution, followed by extraction with 30 ml of ethyl acetate twice. Organic layers were combined, and washed with 10 ml of an aqueous saturated sodium hydrogen carbonate solution and 10 ml of an aqueous saturated sodium chloride solution. The resulting organic layer was dried over sodium sulfate, filtered, and then concentrated under reduced pressure. The residue was subjected to silica gel column chromatography to obtain 0.13 g of 2-[4-(methoxyimino)cyclohexyl]-2-(3,3,3-trifluoropropylsulfonyl)propionitrile (hereinafter, referred to as the halogen-containing organosulfur compound (40)) represented by the formula: 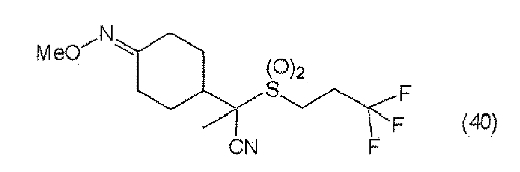 ¹H-NMR (CDCl₃, TMS, δ (ppm)): 1.31-1. 64 (2H,m), 1.75 (3H, s) , 1.76-1.91 (1H, m), 2.13-2.32 (3H, m), 2.48-2.62 (2H, m), 2.69-2.87 (2H, m), 3.32-3.47 (2H, m), 3.55-3.64 (1H, m), 3.83 (3H, s).

### Reference Production Example 40

According to Reference Production Example 39 except that 0.13 g of ethyl iodide was used in place of methyl iodide, 0.13 g of 2-[4-(methoxyimino)cyclohexyl]-2-(3,3,3-trifluoropropylsulfonyl)butyronitrile (hereinafter, referred to as the halogen-containing organosulfur compound (41)) represented by the formula: 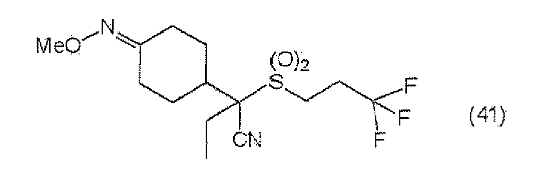 was obtained.
¹H-NMR (CDCl₃,TMS, δ (ppm)): 1.27 (3H, t), 1.43-1.69 (2H, m), 1.73-1.87 (1H m), 2.10-2.31 (5H, m), 2.49-2.61 (2H, m), 2.71-2.87 (2H, m), 3.33-3.47 (2H, m), 3.55-3.66 (1H, m), 3.83 (3H, s).

### Reference Production Example 41

According to Reference Production Example 39 except that 0.14 g of 1-iodopropane was used in place of methyl iodide, 0.055 g of 2-[4-(methoxyimino)cyclohexyl]-2-(3,3,3-trifluoropropylsulfonyl)pentanenitrile (hereinafter, referred to as the halogen-containing organosulfur compound (42)) represented by the formula: 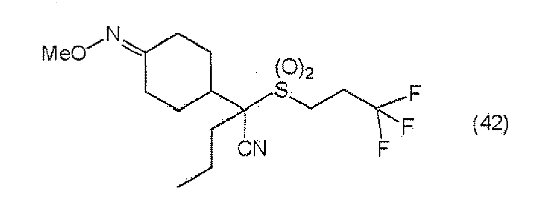 was obtained.
¹H-NMR (CDCl₃, TMS, δ (ppm) 1.06 (3H, t), 1.44-1.71 (4H, m), 1.73-1.86 (1H, m), 1.94-2.13 (2H, m), 2.15-2.31 (3H, m), 2.48-2.61 (2H, m), 2.73-2.83 (2H, m), 3.32-3.46 (2H, m), 3.55-3.65 (1H, m), 3.83 (3H, s).

### Reference Production Example 42

According to Reference Production Example 39 except that 0.10 g of 3-bromopropene was used in place of methyl iodide, 0.14 g of 2-[4-(methoxyimino)cyclohexyl]-3-methyl-2-(3,3,3-trifluoropropylsufonyl)-4-pentenenitrile (hereinafter, referred to as the halogen-containing organosulfur compound (43)) represented by the formula: 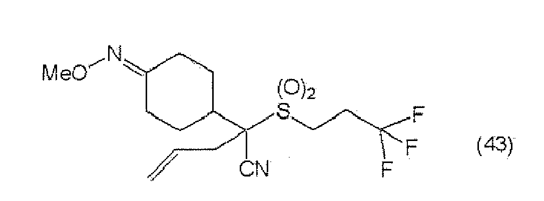 was obtained.
¹H-NMR (CDCl₃, TMS, δ (ppm)): 1.41-1.68 (2H, m), 1.73-1.87 (1H, m), 2.12-2.33 (3H, m), 2.47-2.82 (5H, m), 2.86-2.91 (1H, m), 3.31-3.48 (2H, m), 3.61-3.68 (1H, m), 3.83 (3H, s), 5.40-5.49 (2H, m), 5.87-6.02 (1H, m).

### Reference Production Example 43

According to Reference Production Example 39 except that 0.10 g of 3-bromopropyne was used in place of methyl iodide, 0.14 g of 2-[4-(methoxyimino)cyclohexyl]-3-methyl-2-(3,3,3-trifluoropropylsulfonyl)-4-pentynenitrile (hereinafter, referred to as the halogen-containing organosulfur compound (44)) represented by the formula: 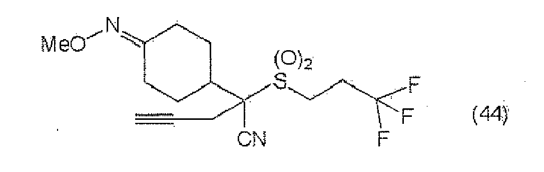 was obtained.
¹H-NMR (CDCl₃, TMS, δ (ppm)): 1.29-1.47 (1H, m), 1.50-1.68 (1H, m), 1.74-1.91 (1H, m), 2.13-2.35 (3H, m), 2.43-2.45 (1H, m), 2.49-2.61 (1H, m), 2.73-2.86 (3H, m), 2.91-3.11 (2H, m), 3.32-3.47 (1H, m), 3.73-3.89 (2H, m), 3.83 (3H, s).

### Reference Production Example 44

A solution of 0.45 g of the halogen-containing organosulfur compound (33) in 5 ml of tetrahydrofuran was cooled to 0°C under a nitrogen atmosphere. Thereto 0.10 g of 60% sodium hydride dispersion in paraffin liquid was added, and the mixtrue was stirred for 30 minutes. Then 0.20 g of N-chlorosuccinimide was added, and the mixture was stirred at room temperature overnight. To the reaction solution was added 10 ml of an aqueous 1N hydrochloric acid solution, followed by extraction with 30 ml of ethyl acetate twice. Organic layers were combined, washed with 10 ml of an aqueous saturated sodium hydrogen carbonate solution and 10 ml of an aqueous saturated sodium chloride solution. The resulting organic layer was dried over sodium sulfate, filtered, and then concentrated under reduced pressure. The residue was subjected to silica gel column chromatography to obtain 0.39 g of 2-chloro-2-[4-(methoxyimlno)-cyclohexyl]-2-(3,3,3-trifluoropropylsulfonyl)acetonitrile (hereinafter, referred to as the halogen-containing organosulfur compound (45)) represented by the formula: 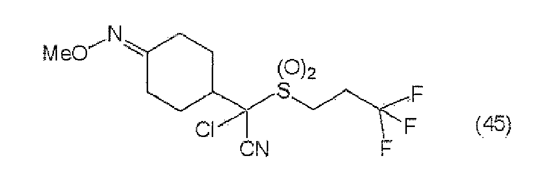 ¹H-NMR (CDCl₃, TMS, δ (ppm)): 1.52-1.91 (3H,m), 2.16-2.29 (1H, m), 2.34-2.48 (2H, m), 2.52-2.63 (1H, m), 2.74-2.89 (3H, m), 3.36-3.46 (1H, m), 3.64-3.82 (2H, m), 3.84 (3H, s).

### Reference Production Example 45

A mixture of 1.00 g of (3,3,3-trifluoropropylsulfonyl) acetonitrile, 30 ml of tetrahydrofuran, 0.12 g of DL-proline and 1.01 g of cyclopentanone was heated and stirred for 6 hours under the reflux condition,. The reaction mixture was cooled to 0°C, and 0.42 g of sodium borohydride was added thereto. The mixture was stirred at room temperature for 6 hours. After the reaction mixture was cooled to 0°C, 10 ml of water and 30 ml of ethyl acetate were added. While the mixture was stirred, 50 ml of 1N hydrochloric acid was added dropwise thereto, followed by extraction with 30 ml of ethyl acetate twice. Organic layers were combined, washed successively with 30 ml of an aqueous saturated sodium hydrogen carbonate solution and 30 ml of an aqueous saturated sodium chloride solution, dried over sodium sulfate, filtered, and then concentrated under reduced pressure. The residue was subjected to silica gel column chromatography to obtain 1.01 g of 2-cyclopentyl-2-(3,3,3-trifluoropropylsulfonyl) acetonitrile (referred to as the halogen-containing organosulfur compound (46)) represented by the following formula (46). 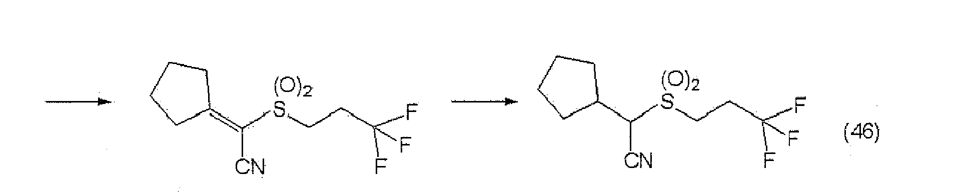 ¹H-NMR (CDCl₃, TMS, δ (ppm) 1.46-1.56 (1H, m), 1.59-1.71 (3H, m), 1.73-1.85 (2H, m), 2.02-2.17 (2H, m), 2.66-2.86 (3H, m), 3.38-3.56 (2H, m), 4.03 (1H, d).

### Reference Production Example 46

According to Reference Production Example 45 except that 1.14 g of cyclohexanone was used in place of cyclopentanone, 1.01 g of 2-cyclohexyl-2-(3,3,3-trifluoropropylsulfonyl) acetonitrile (hereinafter, referred to as the halogen-containing organosulfur compounds (47)) represented by the following formula (47) was obtained. 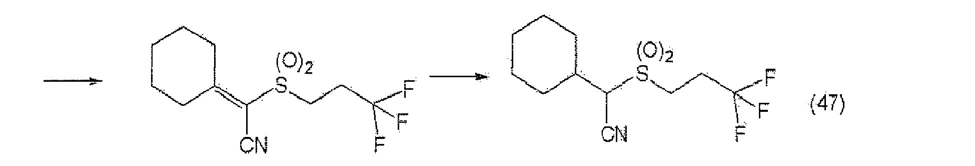 ¹H-NMR (CDCl₃, TMS, δ (ppm)): 1.16-1.28 (1H, m), 1.30-1.47 (4H, m), 1.69-1.77 (1H m), 1.79-1.88 (3H, m), 2.15-2.22 (1H, m), 2.39-2.49 (1H, m), 2.70-2.84 (2H, m), 3.37-3.46 (1H, m), 3.48-3.56 (1H, m), 3.80 (1H, d).

### Reference Production Example 47

According to Reference Production Example 45 except that 1.23 g of cycloheptanone was used in place of cyclopentanone, 1.24 g of 2-cyclohexyl-2-(3,3,3-trifluoropropylsulfonyl)acetonitrile (hereinafter, referred to as the halogen-containing organosulfur compound (48)) represented by the following formula (48) was obtained. 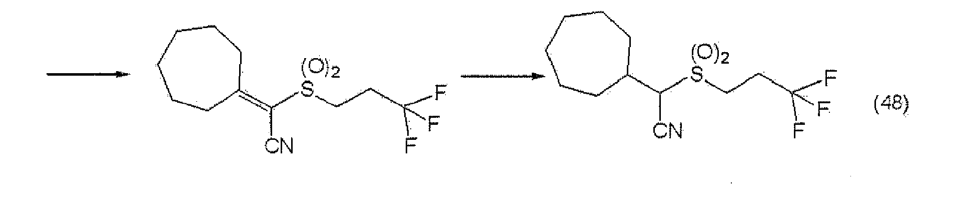 ¹H-NMR (CDCl₃, TMS, δ (ppm)): 1.50-1.86 (11H, m), 2.14-2.22 (1H, m), 2.56-2.62 (1H m), 2.70-2.83 (2H, m), 3.36-3.44 (1H, m), 3.48-3.56 (1H, m), 3.84 (1H, d).

### Reference Production Example 48

According of Reference Production Example 45 except that 1.34 g of 4-methylcyclohexanone was used in place of cyclopentanone, 1.12 g of 2-(4-methylcyclohexyl)-2-(3,3,3-trifluoropropylsulfonyl)acetonitrile (hereinafter, referred to as the halogen-containing organosulfur compound (49)) represented by the following formula (49) was obtained. The resulting halogen-containing organosulfur compound (49) was a 6/4 isomer mixture. 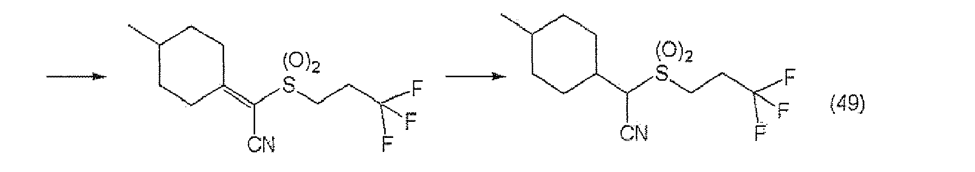 The main isomer of the halogen-containing organosulfur compound (49):
¹H-NMR (CDCl₃, TMS, δ (ppm)): 0.98 (3H, d), 1.00-1.12 (1H, m), 1.32-1.97 (8H, m), 2.45-2.54 (1H m), 2.71-2.87 (2H, m), 3.37-3.59 (2H, m), 3.92 (1H, d).
Minor isomer of the halogen-containing organosulfur compound (49):
¹H-NMR (CDCl₃, TMS, δ (ppm) ): 0.91 (3H, d), 1.32-1.97 (8H, m), 2.14-2.23 (1H, m), 2.33-2.43 (1H m), 2.71-2.87 (2H, m), 3.37-3.59 (2H, m), 3.82 (1H, d).

### Reference Production Example 49

According to Reference Production Example 45 except that 1.24 g of 4,4-dimetylcyclohexanone was used in place of cyclopentanone, 0.98 g of 2-(4,4-dimethylcyclohexyl)-2-(3,3,3-trifluoropropylsulfonyl)acetonitrile (hereinafter, referred to as the halogen-containing organosulfur compound (50)) represented by the following formula (50) was obtained. 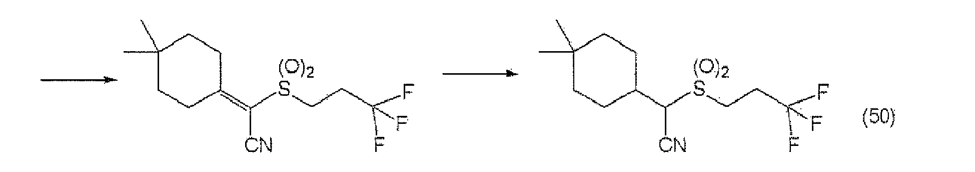 ¹H-NMR (CDCl₃, TMS, δ (ppm)): 0.93 (3H, s), 0.94 (3H, s), 1.25-1.36 (2H, m), 1.45-1.69 (5H, m), 1.94-2.03 (1H, m), 2.30-2.39 (1H, m), 2.69-2.83 (2H, m), 3.37-3.46 (1H, m), 3.48-3.56 (1H, m), 3.84 (1H, d).

### Reference Production Example 50

A mixture of 3.31 g of (3,3,3-trifluoropropylsulfonyl) acetonitrile, 60 ml of tetrahydrofuran, 0.19 g of DL-proline and 2.03 g of 4-cyanocyclohexanone was heated and stirred for 6 hours under the reflux condition. After the reaction mixture was cooled to 0°C, 0.62 g of sodium borohydride was added thereto. The mixture was stirred at room temperature for 6 hours. After the reaction mixture was cooled to 0°C, 30 ml of water and 50 ml of ethyl acetate were added thereto. While the mixture was stirred, 90 ml of IN hydrochloric acid was added dropwise, followed by extraction with 50 ml of ethyl acetate three times. An organic layer was washed with 50 ml of an aqueous saturated sodium hydrogen carbonate solution and 50 ml of an aqueous saturated sodium chloride solution, dried over sodium sulfate, filtered, and then concentrated under reduced pressure. The residue was subjected to silica gel column chromatography to obtain 0.51 g of a trans form (hereinafter, referred to as the halogen-containing organosulfur compound (51t)) and 0.59 of a cis form (hereinafter, referred to as the halogen-containing organosulfur compound (51c)) of 2-(4-cyanocyclohexyl)-2-(3,3,3-trifluoropropylsulfonyl) acetnitrile represented by the following formula (51). 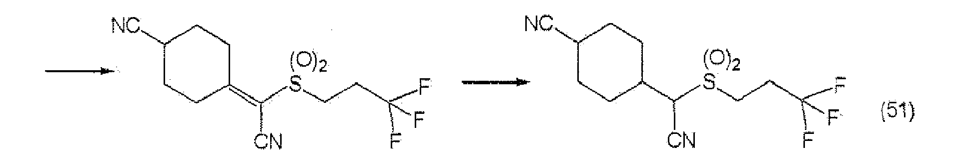 The halogen-containing organosulfur compound (51c): ¹H-NMR (CDC1_{3,} TMS, δ (ppm)): 1.40-1.54 (2H, m), 1.64-1.79 (2H, m), 1.89-1.99 (1H, m), 2.21-2.30 (2H, m), 2.30-2.38 (1H, m), 2.41-2.54 (2H, m), 2.69-2.84 (2H, m), 3.37-3.47 (1H m), 3.51-3.59 (1H, m), 3.83 (1H, d).
The halogen-containing organosulfur compound (51t):
¹H-NMR (CDC1₃, TMS, δ(ppm)): 1.64-1.88 (4H, m), 1.95-2.02 (1H, m), 2.09-2.19 (2H, m), 2.19-2.29 (1H, m), 2.41-2.51 (1H, m), 2.51-2.69 (2H, m), 3.00-3.05 (1H, m), 3.41-3.50 (1H, m), 3.54-3.63 (1H, m), 3.83 (1H, d).

### Reference Production Example 51

To a solution of 0.34 g of the halogen-containing organosulfur compound (27) in 10 ml of acetonitrile were added 0.21 g of 1,2-ethanedithiol and 0.05 g of tetrabutylammonium tribromide, and the mixture was stirred at room temperature for 1 hour. To the reaction mixture were added 100 ml of ethyl acetate and then 50 ml of an aqueous saturate sodium hydrogen carbonate solution. The solution was stirred for 1 hour and then extracted with 50 ml of ethyl acetate twice. Organic layers were combined, washed with 50 ml of an aqueous saturated sodium hydrogen carbonate solution and 50 ml of an aqueous saturated sodium chloride solution, dried over sodium sulfate, filtered, and then concentrated under reduced pressure. The residue was subjected to silica gel column chromatography to obtain 0.36 g of 2-(1,4-dithiaspiro[4.5]dec-8-yl)-2-(3,3,3-trifluoropropylsulfonyl)acetonitrile (hereinafter, referred to as the halogen-containing organosulfur compound (52)) represented by the formula: 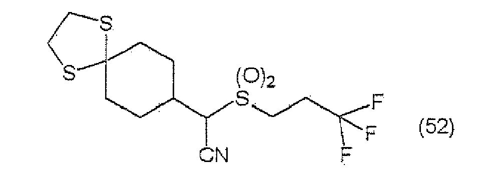 ¹H-NMR (CDC1₃, TMS, δ (ppm)): 1.67-1.83 (2H,m), 1.88-1.97 (1H, m), 1.98-2.09 (2H, m), 2.17-2.30 (3H,m), 2.36-2.48 (1H, m), 2.67-2.85 (2H, m), 3.24-3.36 (4H, m), 3.48-3.50 (1H, m), 3.60-3.83 (1H, m), 3.86 (1H, d).

### Reference Production Example 52

### Step 52-1

To a suspension of 22.85 g of potassium thioacetate in 200 ml of methanol was added dropwise 54.79 g of 1-iodo-3,3,4,4,4-pentafluorobutane at 0°C over 30 minutes under a nitrogen atmosphere, and the mixture was stirred at room temperature for 1 hour. At this time, the reaction mixture was analyzed by thin layer chromatography (TLC), and thereby the formation of 3,3,4,4,4-pentafluorobutyl thioacetate was confirmed. After the mixture was cooled to 0°C, 40.52 g of a 28% solution of sodium methoxide in methanol was added dropwise over 15 minutes thereto. The mixture was stirred at room temperature for 1 hour. To the mixture was added 16.61 g of chloroacetonitrile at 0°C, and the mixture was stirred at room temperature for 3 hours. A reaction vessel was cooled in an ice bath, and an aqueous 1N hydrochloric acid solution was added to the reaction mixture. Methanol was distilled off under reduced pressure. The residual reaction mixture was extracted with 200 ml of t-butyl methyl ether twice. Organic layers were combined, washed with an aqueous saturated sodium chloride solution, dried over sodium sulfate, filtered, and then subjected to reduced pressure to distill off the solvent. The residue was subjected to silica gel column chromatography to obtain 17.81 g of (3,3,4,4,4-pentafluorabutylthio)acetonitrile. 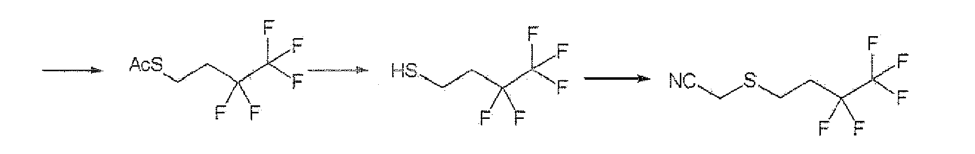 ¹H-NMR (CDC1₃, TMS, δ (ppm)): 2.35-2.52 (2H,m), 2.94-3.03 (2H, m), 3.36 (2H, s).

Alternatively, 3,3,4,4,4-pentafluorobutyl thioacetate was synthesized according to Step 27-1 of Reference Production Example 27 except that 1.22 g of 1-iodo-3,3,4,4,4-pentafluorobatane was used in place of 1-iodo-3,3,3-trifluoropropane.

### Step 52-2

To a suspension of 17.81 g of (3,3,4,4,4-pentafluorabutylthio)acetanitrile and 0.28 g of sodium tungstate dihydrate in 30 ml of water was added 8.94 ml of 31% aqueous hydrogen peroxide while the suspension was stirred. The temperature of the mixture was raised to 65°C, and 8.94 ml of 31% aqueous hydrogen peroxide was added thereto. The mixture was stirred at 70°C for 1 hour. In the middle of the reaction, the formation of a deduced sulfoxide compound was confirmed by thin layer chromatography (TLC) analysis. The reaction mixture was cooled to room temperature, and 30 ml of an aqueous sodium sulfite solution was added thereto, followed by extraction with 150 ml of ethyl acetate three times. Organic layers were combined, washed with an aqueous saturated sodium chloride solution, dried over sodium sulfate, filtered, and then concentrated under reduced pressure. The residue was crystallized from chloroform: hexane=1:2 to obtain 17.84 g of (3,3,4,4,4-pentafluorobutylsulfonyl)acetonitrile represented by the formula: 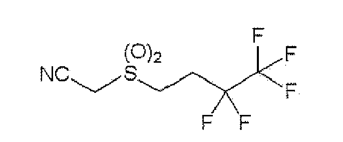 ¹H-NMR (CDC1₃, TMS, δ (ppm)): 2.66-2.80 (2H, m), 3.53-3.58 (2H, m), 4.09 (2H, s).

### Step 52-3

A mixture of 7.74 g of (3,3,4,4,4-pentafluorobutylsulfonyl) acetonitrile, 100 ml of toluene, 0.23 g of DL-proline and 4.81 g of 1,4-cyclohexanedionemonoethylene ketal was heated and stirred for 3 hours under the reflux condition. After 20 ml of toluene was distilled off, the reaction mixture was cooled to room temperature. To the reaction mixture was added 100 ml of tetrahydrofuran. After cooled to 0°C, to the reaction mixture was added 1.17 g of sodium borohydride. The mixture was stirred at room temperature for 6 hours and then cooled to 0°C, and 100 ml of water and 100 ml of ethyl acetate were added thereto. To the mixture was added dropwise 100 ml of 1N hydrochloric acid while the mixture was stirred, followed by extraction with 100 ml of ethyl acetate twice. An organic layer was washed with 100 ml of an aqueous saturated sodium hydrogen carbonate solution, 100 ml of an aqueous saturated sodium chloride solution and then 100 ml of water, dried over sodium sulfate, filtered, and then concentrated under reduced pressure. The residue was subjected to silica gel column chromatography to obtain 5.00 g of 2- (1,4-dioxaspiro[4.5]dec-8-yl)-2-(3,3,4,4,4-pentafluorobutylsulfonyl)acetonitrile (hereinafter, referred to as the halogen-containing organosulfur compound (53)) represented by the following formula (53). 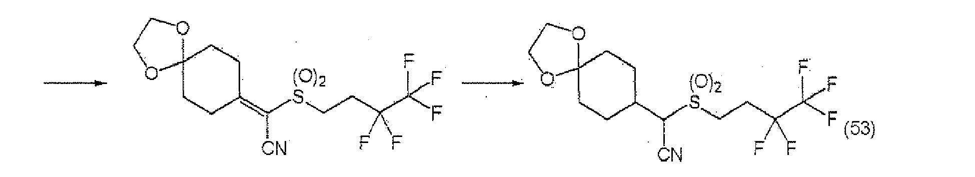 ¹H-NMR (CDC1₃, TMS, δ (ppm)): 1.58-1.91 (7H, m), 2.13-2.22 (1H, m), 2.39-2.51 (1H, m), 2.58-2.82 (2H, m), 3.40-3.50 (1H, m), 3.53-3.63 (1H, m), 3.87 (1H, d), 3.93-3.98 (4H, m).

### Reference Production Example 53

A mixture of 5.00 g of the halogen-containing organosulfur compound (53), 14 ml of acetic acid and 6 ml of water was heated to 70°C and stirred for 10 hours. After the reaction mixture was cooled to room temperature, 100 ml of ethyl acetate was added. The mixture was slowly added to 100 ml of an aqueous saturated sodium hydrogen carbonate solution. The solution was stirred for 1 hour, followed by extraction with 100 ml of ethyl acetate twice. Organic layers were combined, washed with 100 ml of an aqueous saturated sodium hydrogen carbonate solution and 100 ml of an aqueous saturated sodium chloride solution, dried over sodium sulfate, filtered, and then concentrated under reduced pressure. The residue was subjected to silica gel column chromatography to obtain 3.56 g of 2-(4-oxocyclohexyl)-2-(3,3,4,4,4-pentafluorobutylsulfonyl)acetonitrile (hereinafter, referred to as the halogen-containing organosulfur compound (54)) represented by the formula: 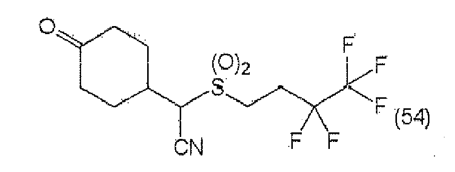 ¹H-NMR (CDC1₃, TMS, δ (ppm)): 1.84-1.97 (2H, m), 2.17-2.26 (1H, m), 2.40-2.61 (5H, m), 2.81-2.85 (2H, m), 3.44-3.54 (1H, m), 3.59-3.70 (1H, m), 3.99 (1H, d).

### Reference Production Example 54

To a solution of 0.35 g of the halogen-containing organosulfur compound (54) in 10 ml of tetrahydrofuran were added 0.75 g of pyridine and 0.79 g of methoxyamine hydrochloride, and the mixture was stirred at room temperature for 3 hours. To the reaction mixture was added 30 ml of an aqueous IN hydrochloric acid solution, followed by extraction with 50 ml of ethyl acetate twice. Organic layers were combined, and washed with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was subjected to silica gel column chromatography to obtain 0.34 g of 2-[4-(methoxyimino)cyclohexyl]-2-(3,3,4,4,4-pentafluorobutylsulfonyl)acetonitrile (hereinafter, referred to as the halogen-containing organosulfur compound (55)) represented by the formula: 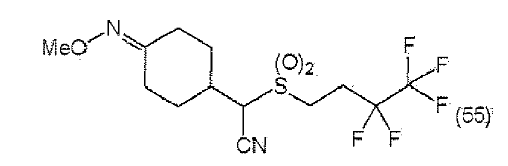 ¹H-NMR (CDC1₃, TMS, δ (ppm) ): 1.55-1.72 (2H, m) , 1.80-1.92 (1H, m), 1.95-2.11 (1H, m), 2.17-2.28 (1H, m), 2.31-2.45 (1H, m), 2.48-2.58 (1H, m), 2.61-2.80 (3H, m), 3.33-3.40 (1H, m), 3.42-3.50 (1H, m), 3.55-3.65 (1H, m), 3.83 (3H, s), 3.89 (1H, d).

### Reference Production Example 55

To a solution of 2.88 g of the halogen-containing organosulfur compound (15) in 10 ml of pyridine was added 1.91 g of p-toluenesulfonyl chloride, and the mixture was stirred at room temperature for 3 hours. To the reaction solution was added 30 ml of an aqueous LN hydrochloric acid solution, followed by extraction with 50 ml of ethyl acetate twice. Organic layers were combined, washed with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was dissolved in 20 ml of toluene. To the solution were added 1.50 g of sodium iodide and 1.52 g of 1,8-diazabicyclo[5.4.0]undec-7-ene, and the mixture was heated to 110°C and stirred for 10 hours. To the reaction solution was added 30 ml of an aqueous IN hydrochloric acid solution, followed by extraction with 50 ml of ethyl acetate twice. Organic layers were combined, and washed successively with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was subjected to silica gel column chromatography to obtain 1.01 g of 1-methylene-4-(3,3,3-trifluoropropylsulfonylmethyl)-cyclohexane (hereinafter, referred to as the halogen-containing organosulfur compound (56)) represented by the formula: 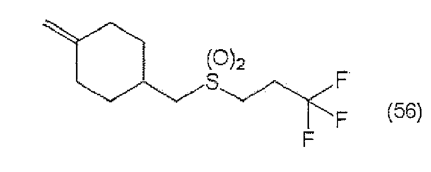 ¹H-NMR (CDC1₃, TMS, δ (ppm)): 1.20-1.31 (2H, m) , 2.03-2.17 (4H, m), 2.22-2.36 (3H, m), 2.62-2.74 (2H, m) , 2.95 (2H, d) , 3.16-3.21 (2H, m), 4.66 (2H, s).

### Reference Production Example 56

To a solution of 0.60 g of the halogen-containing organosulfur compound (56) in 4 ml of dichloromethane were added 0.86 g of bromoform, 0.44 g of sodium hydroxide and 0.02 g of benzyltriethylammonium chloride, and the mixture was stirred at 30°C for 4 hours under an ultrasound irradiation condition. To the reaction mixture was added 20 ml of an aqueous 2N hydrochloric acid solution, followed by extraction with 30 ml of ethyl acetate twice. Organic layers were combined, washed successively with 30 ml of an aqueous saturated sodium hydrogen carbonate solution and 30 ml of an aqueous saturated sodium chloride solution, dried over sodium sulfate, filtered, and then concentrated under reduced pressure. The residue was subjected to silica gel column chromatography to obtain 0.31 g of 1,1-dibromo-6-(3,3,3-trifluoropropylsulfonylmethyl) spiro [2.5] octane (hereinafter, referred to as the halogen-containing organosulfur compound (57)) represented by the formula: 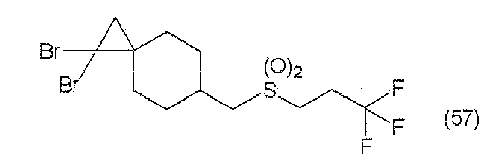 ¹H-NMR (CDC1₃, TMS, δ (ppm)): 1.23-1.34 (2H, m), 1.39 (2H, s) , 1.59-1.67 (2H, m), 1.85-1.95 (2H, m), 2.07-2.15 (2H, m), 2.16-2.25 (1H, m), 2.63-2.75 (2H, m), 2.99 (2H, d), 3.17-3.21 (2H, m).

### Reference Production Example 57

According to Reference Production Example 56 except that 0.71 g of chloroform was used in place of bromoform, 0.41 g of 1,1-dichloro-6-(3,3,3-trifluoropropylsulfonylmethyl)-spiro [2.5] octane (hereinafter, referred to as the halogen-containing organosulfur compound (58)) represented by the formula: 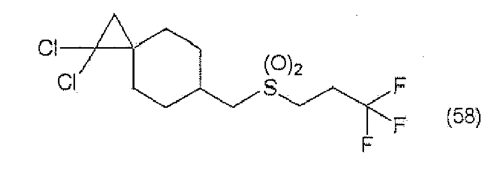 was obtained.
¹H-NMR (CDC1₃, TMS, δ (ppm) ): 1.24-1.35 (2H, m), 1.53-1.62 (4H, m), 1.84-1.93 (2H, m), 2.08-2.15 (2H, m), 2.16-2.26 (1H, m), 2.63-2.75 (2H, m), 2.99 (2H, d), 3.18-3.22 (2H, m).

### Reference Production Example 58

To a solution of 0.46 g of the halogen-containing organosulfur compound (54) in 10 ml of tetrahydrofuran were added 0.86 g of pyridine and 0.86 g of an aqueous 30% ethoxyamine hydrochloride solution, and the mixture was stirred at room temperature for 3 hours. To the reaction mixture was added 30 ml of an aqueous IN hydrochloric aicd solution, followed by extraction with 50 ml of ethyl acetate twice. Organic layers were combined, and washed with 50 ml of an aqueous saturated sodium hydrogen carbonate solution and 50 ml of an aqueous saturated sodium chloride solution. The resulting organic layer was dried over sodium sulfate, filitered, and then concentrated under reduced pressure. The residue was subjected to silica gel column chromatography to obtain 0.34 g of 2-[4-(ethoxyimino)cyclohexyl]-2-(3,3,4,4,4-pentafluorobutylsulfonyl)acetonitrile (hereinafter, referred to as the halogen-containing organosulfur compound (59)) represented by the formula: 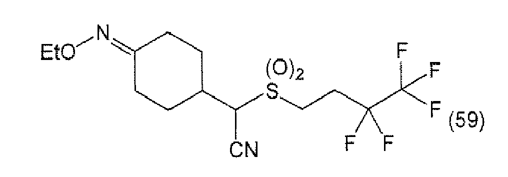 ¹H-NMR (CDC1₃,TMS, δ (ppm)): 1.25 (3H, t), 1.55-1. 73 (2H, m), 1.76-1.92 (1H, m), 1.94-2.09 (1H, m), 2.16-2.29 (1H, m), 2.31-2.44 (1H, m), 2.48-2.57 (1H, m), 2.62-2.79 (3H, m), 3.36-3.51 (2H, m), 3.54-3.65 (1H, m), 3.88 (1H, d), 4.08 (2H, q).

### Reference Production Example 59

### Step 59-1

According to Step 52-1 of Reference Production Example 52 except that 4.19 g of 1-iodo-3-(trifluoromethyl)-3,4,4,4-tetrafluorobutane was used in palce of 1-iodo-3,3,4,4,4-pentafluorobutane, 4.77 g of (3-(trifluoromethyl)-3,4,4,4-pentafluorobutylthio)acetonitrile was obtained.

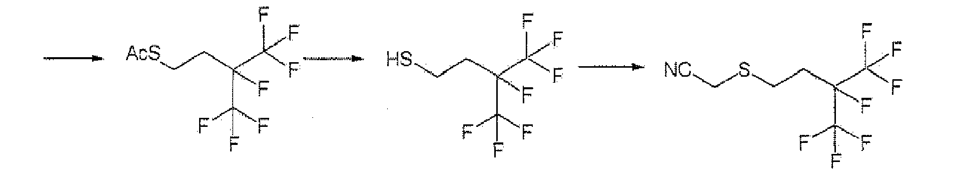

### Step 59-2

To a suspension of 16.14 g of a double salt of 2KHSO₅·KHSO₄·K₂SO₄ (Oxone, registered trade mark) in 50 ml of water was added dropwise a solution of 4.77 g of (3-(trifluoromethyl)-3,4,4,4-pentafluorobutylthio)acetonitrile in 50 ml of methanol at room temperature over 60 minutes under a nitrogen atmosphere, and the mixture was stirred for 2 hours. To the reaction mixture was added 50 ml of an aqueous 10% sodium sulfite solution, follwed by extraction with 100 ml of ethyl acetate twice. Organic layers were combined, washed with 50 ml of an aqueous 10% sodium sulfite solution and 50 ml of an aqueous saturated sodium chloride solution, dried over sodium sulfate, filtered, and then concentrated under resuced pressure. The residue was subjected to silica gel column chromatography to obtain 4.00 g of (3- (trifluoromethyl) -3, 4, 4, 4-pentafluorobutylsulfonyl)acetonitrile represented by the formula: 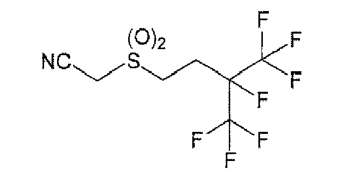 ¹H-NMR (CDC1₃, TMS, δ (ppm)): 2.70-2.81 (2H, m), 3.50-3.55 (2H, m), 4.08 (2H, s).

### Step 59-3

A mixture of 4.00 g of (3-(trifluoromethyl)-3,4,4,4-pentafluorobutylsulfonyl) acetonitrile, 50 ml of toluene, 0.15 g of DL-proline and 2.28 g of 1,4-cyclohexanedionemonoethylene ketal was heated and stirred for 3 hours under the reflux condition. After 30 ml of the toluene was distilled off, the reaction mixture was cooled to 0°C. To the reaction mixture were added 0.25 g of sodium borohydride and 2 ml of N,N-dimethylformamide. The mixture was stirred at room temperature for 6 hours, and then cooled to 0°C, and 50 ml of water and 50 ml of ethyl acetate were added thereto. To the mixture was added dropwise 20 ml of 1N hydrochloric acid while the mixture was stirred, followed by extraction with 100 ml of ethyl acetate twice. An organic layer was washed with 100 ml of an aqueous saturated sodium hydrogen carbonate solution and 100 ml of an aqueous saturated sodium chloride solution, dried over sodium sulfate, filtered, and then concentrated under reduced pressure. The residue was subjected to silica gel column chromatography to obtain 4.41 g of 2-(1,4-dioxaspiro[4,5]dec-8-yl)-2-(3-(trifluoromethyl)-3,4,4,4-pentafluorobutylsulfonyl)acetonitrile (hereinafter, referred to as the halogen-containing organosulfur compound (60)) represented by the following formula (60). 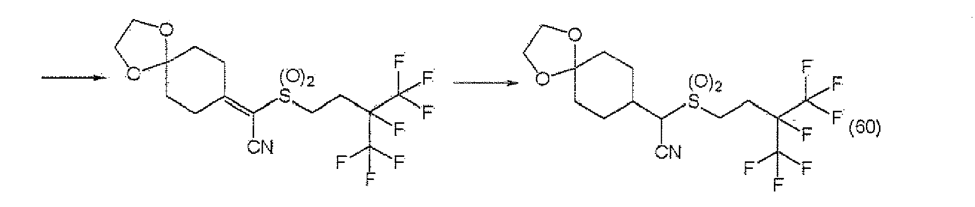 ¹H-NMR (CDC1₃, TMS, δ (ppm)): 1.51-2.22 (8H, m), 2.40-2.51 (1H, m), 2.64-2.83 (2H, m), 3.37-3.48 (1H, m), 3.49-3.61 (1H, m), 3.86-3.89 (1H, m), 3.90-3.99 (4H, m).

### Reference Production Example 60

A mixture of 4.41 g of the halogen-containing organosulfur compound (60), 14 ml of acetic acid, 6 ml of water, 1.50 g of methoxyamine hydrochloride and 1.47 g of sodium acetate was heated to 100°C and stirred for 10 hours. The reaction mixture was cooled to room temperature, and 100 ml of ethyl acetate was added thereto. The mixture was slowly added to 100 ml of an aqueous saturated sodium hydrogen carbonate solution. The mixture was stirred for 1 hour, followed by extraction with 100 ml of ethyl acetate twice. Organic layers were combined, washed with 100 ml of an aqueous saturated sodium hydrogen carbonate solution and 100 ml of an aqueous saturated sodium chloride solution, dried over sodium sulfate, filtered, and then concentrated under reduced pressure. The residue was subjected to silica gel column chromatography to obatin 3.61 g of 2-(4-methoxyiminocyclohexyl)-2-(3-(trifluoromethyl)-3,4,4,4-pentafluorobutylsulfonyl)acetonitrile (hereinafter, referred to as the halogen-containing organosulfur compound (61)) represented by the following formula (61). 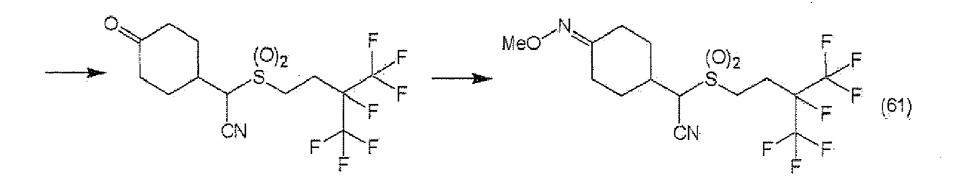 ¹H-NMR (CDC1₃, TMS, δ (ppm) ) : 1.52-1.72 (2H,m), 1.81-1.91 (1H, m), 1.96-2.08 (1H, m), 2.16-2.28 (1H, m), 2.31-2.44 (1H, m), 2.50-2.57 (1H, m), 2.62-2.82 (3H, m), 3.33-3.48 (2H, m), 3.53-3.63 (1H, m), 3.83 (3H, s), 3.89 (1H, d).

### Reference Production Example 61

### Step 61-1

A mixture of 42.65 g of 3,3,4,4,5,5,6,6,6-nonafluorohexyl-1-toluenesulfonate, 11.65 g of potassium thioacetate and 100 ml of N,N-dimethylformamide was stirred at 80°C for 4 hours under a nitrogen atmosphere. A reaction vessel was cooled in an ice bath. To the reaction mixture was added an aqueous 1N hydrochloric acid solution, and the mixture was extracted with 200 ml of ethyl acetate twice. Organic layers were combined, washed with an aqueous saturated sodium chloride solution, dried over sodium sulfate, filtered, and then concentrated under reduced pressure to remove the solvent. The residue was subjected to silica gel column chromatography to obtain 18.91 g of 3,3,4,4,5,5,6,6,6-nonafluorohexyl thioacetate represented by the formula: 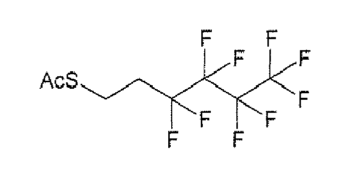 ¹H-NMR(CDC1₃, TMS, δ (ppm)): 2.28-2.45 (5H,m),3.04-3.13(2H, m).

### Step 61-2

A solution of 18.91 g of 3,3,4,4,5,5,6,6,6-nonafluorohexyl thioacetate in 60 ml of tetrahydrofuran was cooled to 0°C. Thereto 11.32 g of a 28% solution of sodium methoxide in methanol was added dropwise over 15 minutes, and then stirred at room temperature for 1 hour. To the mixture was added 4.40 g of chloroacetonitrile at 0°C, and then stirred at room temperature for 3 hours. A reaction vessel was cooled in an ice bath. To the reaction mixture was added an aqueous saturated sodium chloride solution, and the mixture was extracted with 100 ml of t-butyl methyl ether twice. Organic layers were combined, washed with an aqueous saturated sodium chloride solution, dried over sodium sulfate, filtered, and then concentrated under reduced pressure. The residue was subjected to silica gel column chromatography to obtain 15.70 g of (3,3,4,4,5,5,6,6,6-nonafluorohexylthio)acetonitrile. 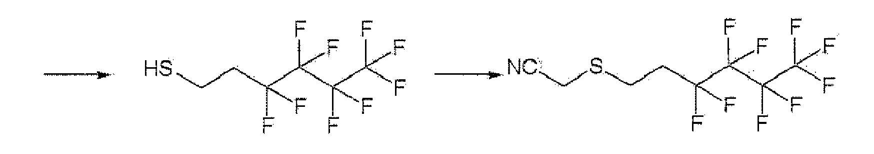 ¹H-NMR(CDC1₃, TMS, δ (ppm)) : 2.40-2.59 (2H, m), 2.93-3.06 (2H, m), 3.38 (2H, s).

### Step 61-3

To a suspension of 15.10 g of a double salt of 2KHSO₅·KHSO₄·K₂SO₄ (Oxone, registered trade mark) in 100 ml of water was added dropwise a solution of 15.70 g of (3,3,4,4,5,5,6,6,6-nonafluorohexylthio)acetonitrile in 100 ml of methanol at -20°C over 60 minutes under a nitrogen atmosphere, and the mixture was stirred for 2 hours. To the reaction mixture was added 50 ml of an aqueous 10% sodium sulfite solution, follwed by extraction with 100 ml of ethyl acetate twice. Organic layers were combined, washed with 50 ml of an aqueous 10% sodium sulfite solution and 50 ml of an aqueous saturated sodium chloride solution, dried over sodium sulfate, filtered, and then concentrated under resuced pressure. The residue was subjected to silica gel column chromatography to obtain 12.56 g of (3,3,4,4,5,5,6,6,6-nonafluorohexylsulfinyl)acetonitrile represented by the formula: 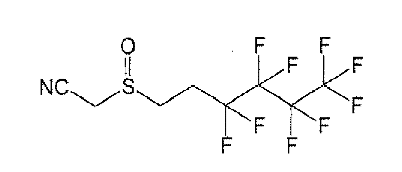 ¹H-NMR (CDC1₃, TMS, δ (ppm)) : 2.56-2.79 (2H, m), 3.10-3.29 (2H, m), 3.63-3.84 (2H, m).

### Step 61-4

To a suspension of 9.21 g of a double salt of 2KHSO₅·KHSO₄·K₂SO₄ (Oxone, registered trade mark) in 50 ml of water was added dropwise a solution of 6.80 g of (3,3,4,4,5,5,6,6,6-nonafluorohexylsulfinyl)acetonitrile in 50 ml of methanol at room temperature over 60 minutes under a nitrogen atmosphere, and the mixture was stirred overnight. To the reaction mixture was added 25 ml of an aqueous 10% sodium sulfite solution, follwed by extraction with 100 ml of ethyl acetate twice. Organic layers were combined, washed with 25 ml of an aqueous 10% sodium sulfite solution and 50 ml of an aqueous saturated sodium chloride solution, dried over sodium sulfate, filtered, and then concentrated under resuced pressure. The residue was subjected to silica gel column chromatography to obtain 5.4 g of (3,3,4,4,5,5,6,6,6-nonafluorohexylsulfonyl)acetonitrile represented by the formula: 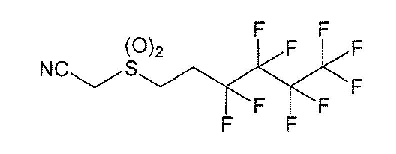 ¹H-NMR (CDC1₃, TMS, δ (ppm)): 2.69-2.83 (2H, m), 3.54-3.60 (2H, m), 4.09 (2H, s).

### Step 61-5

A mixture of 5.40 g of (3,3,4,4,5,5,6,6,6-nonafluorohexylsulfonyl)acetonitrile, 60 ml of toluene, 0.18 g of DL-proline and 2.77 g of 1,4-cyclohexanedione monoethylene ketal was heated and stirred for 3 hours under the reflux condition. After 40 ml of toluene was distilled off, the reaction mixture was cooled to room temperature. The mixture was cooled to 0°C and then 0.61 g of sodium borohydride was added thereto. To the reaction mixture was added 3 ml of N,N-dimethylformamide. The mixture was stirred at room temperature for 6 hours and then cooled to 0°C. Thereto 50 ml of water and 50 ml of ethyl acetate were added. While the reaction mixture was stirred, 20 ml of an aqueous IN hydrochloric acid solution was added dropwise, followed by extraction with 100 ml of ethyl acetate twice. An organic layer was washed with 100 ml of an aqueous saturated sodium hydrogen carbonate solution, 100 ml of an aqueous saturated sodium chloride solution and 100 ml of water, dried over sodium sulfate, filtered, and concentrated under reduced pressure. The residue was dissolve to 14 ml of acetic acid and 6 ml of water. The mixture was heated to 100°C and stirred for 8 hours. After the reaction mixture was cooled to room temperature, 100 ml of ethyl acetate was added thereto. The mixture was added slowly into 100 ml of an aqueous saturated sodium hydrogen carbonate solution. The solution was stirred for 1 hour, followed by extraction with 100 ml of ethyl acetate twice. Organic layers were combined, washed with 100 ml of an aqueous saturated sodium hydrogen carbonate solution and 100 ml of an aqueous saturated sodium chloride solution, dried over sodium sulfate, filtered, and concentrated under reduced pressure. The residue was subjected to silica gel column chromatography to obtain 2.94 g of 2-(4-oxocyclohexyl) -2- (3, 3, 4, 4, 5, 5, 6, 6, 6-nonafluorohexylsulfonyl)acetonitrile (hereinafter, referred to as the halogen-containing organosulfur compound (62)) represented by the formula: 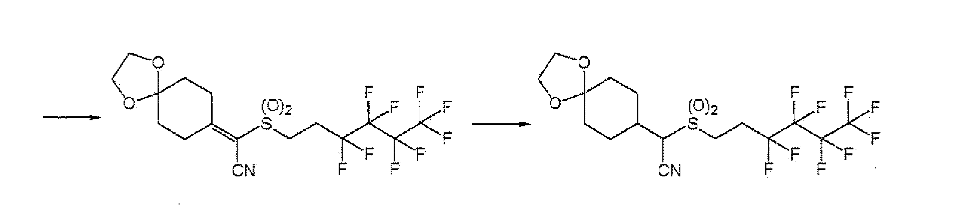 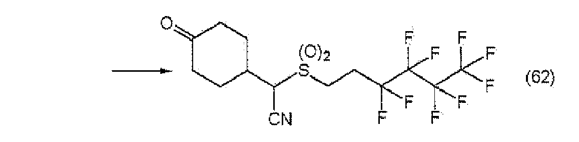 ¹H-NMR(CDC1₃, TMS, δ (ppm) ) : 1.83-1.98 (2H, m) , 2.17-2.26 (1H, m), 2.38-2.59 (5H, m), 2.67-2.97 (3H, m), 3.44-3.54 (1H, m), 3.60-3.70 (1H, m), 4.00 (1H, d).

### Reference Production Example 62

To a solution of 2.69 g of the halogen-containing organosulfur compound (62) in 12 ml of tetrahydrofuran were added 0.52 g of pyridine and 0.55 g of methoxyamine hydrochloride, and the mixture was stirred at room temperature for 3 hours. To the reaction solution was added 30 ml of an aqueous 1N hydrochloric acid solution, followed by extraction with 50 ml of ethyl acetate twice. Organic layers were combined, washed with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was subjected to silica gel column chromatography to obtain 2.86 g of 2-[4-(methoxyimino)cyclohexyl]-2-(3,3,4,4,5,5,6,6,6-nonafluorohexylsulfonyl)acetonitrile (hereinafter, referred to as the halogen-containing organosulfur compound (63)) represented by the formula: 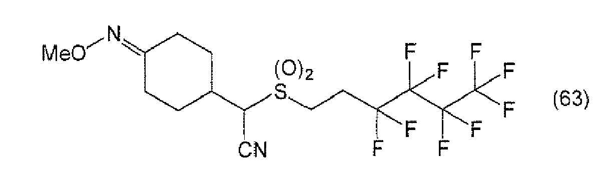 ¹H-NMR (CDC1₃, TMS, δ (ppm)): 1.49-1.73 (2H, m), 1.77-1.91 (1H, m), 1.95-2.09 (1H, m), 2.17-2.27 (1H, m), 2.31-2.43 (1H, m), 2.49-2.57 (1H, m), 2.62-2.86 (3H, m), 3.32-3.39 (1H, m), 3.42-3.53 (1H, m), 3.55-3.66 (1H, m), 3.84 (3H, s), 3.90 (1H, d).

### Reference Production Example 63

### Step 63-1

A mixture of 23.80 g of 1-iodo-4,4,4-trifluorobutane, 100 ml of N,N-dimethylformamide and 11.42 g of potassium thioacetate was stirred at 80°C for 4 hours under a nitrogen atmosphere. A reaction vessel was cooled in an ice bath. To the reaction mixture was added an aqueous 1N hydrochloric acid solution, and the mixture was extracted with 100 ml of t-butyl methyl ether twice. Organic layers were combined, washed with an aqueous saturated sodium chloride solution, dried over sodium sulfate, filtered, and then concentrated under reduced pressure. The residue was subjected to silica gel column chromatography to obtain 18.20 g of 4,4,4-trifluorobutyl thioacetate represented by the formula: 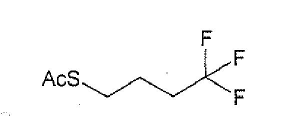 ¹H-NMR(CDC1₃, TMS, δ (ppm)): 1.82-1.92 (2H, m), 2.08-2.23 (2H, m), 2.35 (3H, s) , 2.88-2.99 (2H, m).

### Step 63-2

A solution of 18.91 g of 4,4,4-trifluorobutyl thioacetate in 60 ml of tetrahydrofuran was cooled to 0°C. Thereto 19.29 g of a 28% solution of sodium methoxide in methanol was added dropwise over 15 minutes, and then stirred at room temperature for 1 hour. To the mixture was added 7.50 g of chloroacetonitrile at 0°C, and then stirred at room temperature for 3 hours. A reaction vessel was cooled in an ice bath. To the reaction mixture was added an aqueous saturated sodium chloride solution, and the mixture was extracted with 200 ml of t-butyl methyl ether twice. Organic layers were combined, washed with an aqueous saturated sodium chloride solution, dried over sodium sulfate, filtered, and then concentrated under reduced pressure. The residue was subjected to silica gel column chromatography to obtain 17.82 g of (4,4,4-trifluorobutylthio)acetonitrile. 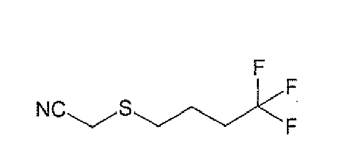 ¹H-NMR(CDC1₃, TMS, δ (ppm)): 1.91-1.99 (2H, m), 2.17-2.32 (2H, m), 2.80-2.87 (2H, m).

### Step 63-3

To a suspension of 67.50 g of a double salt of 2KHSO₅·KHSO₄·K₂SO₄ (Oxone, registered trade mark) in 100 ml of water was added dropwise a solution of 17.82 g of (4,4,4-trifluorobutylthio)acetonitrile in 100 ml of methanol at room temperature over 60 minutes under a nitrogen atmosphere, and the mixture was stirred overnight. To the reaction mixture was added 50 ml of an aqueous 10% sodium sulfite solution, follwed by extraction with 200 ml of ethyl acetate twice. Organic layers were combined, washed with 50 ml of an aqueous 10% sodium sulfite solution and 50 ml of an aqueous saturated sodium chloride solution, dried over sodium sulfate, filtered, and then concentrated under resuced pressure. The residue was subjected to silica gel column chromatography to obtain 22.34 g of (4,4,4-trifluorobutylsulfonyl)acetonitrile represented by the formula: 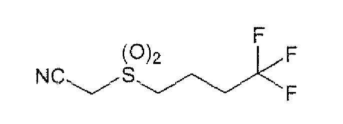 ¹H-NMR (CDC1₃, TMS, δ (ppm)): 2.11-2.28 (2H, m), 2.32-2.46 (2H, m), 3.31-3.43 (2H, m), 4.03 (2H, s).

### Step 63-4

A mixture of 24.10 g of (4,4,4-trifluorobutylsulfonyl) acetonitrile, 200 ml of toluene, 1.11 g of DL-proline and 16.58 g of 1,4-cyclohexanedionemonoethylene ketal was heated and stirred for 5 hours under the reflux condition. After 100 ml of the toluene was distilled off, the reaction mixture was cooled to 0°C. To the reaction mixture were added 1.89 g of sodium borohydride and 5 ml of N,N-dimethylformamide. The mixture was stirred at room temperature for 12 hours, and then cooled to 0°C, and 200 ml of water and 200 ml of ethyl acetate were added thereto. To the mixture was added dropwise 100 ml of 1N hydrochloric acid while the mixture was stirred, followed by extraction with 200 ml of ethyl acetate twice. An organic layer was washed with 100 ml of an aqueous saturated sodium hydrogen carbonate solution and 100 ml of an aqueous saturated sodium chloride solution, dried over sodium sulfate, filtered, and then concentrated under reduced pressure. The residue was subjected to silica gel column chromatography to obtain 22.03 g of 2-(1,4-dioxaspiro[4,5]dec-8-yl)-2-(4,4,4-trifluorobutylsulfonyl)acetonitrile (hereinafter, referred to as the halogen-containing organosulfur compound (64)) represented by the following formula (64). 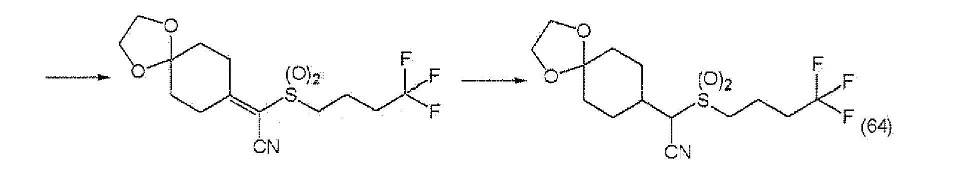 ¹H-NMR (CDC1₃, TMS, δ (ppm)): 1.56-2.49 (13H, m), 3.27-3.43 (2H, m), 3.81 (1H d), 3.90-4.00 (4H, m).

### Reference Production Example 64

A mixture of 22.03 g of the halogen-containing organosulfur compound (64), 70 ml of acetic acid and 30 ml of water was heated to 100°C and stirred for 8 hours. After the reaction mixture was cooled to room temperature, 300 ml of ethyl acetate was added thereto. The mixture was added slowly into 300 ml of an aqueous saturated sodium hydrogen carbonate solution. The solution was stirred for 1 hour, followed by extraction with 200 ml of ethyl acetate twice. Organic layers were combined, washed with 300 ml of an aqueous saturated sodium hydrogen carbonate solution and 300 ml of an aqueous saturated sodium chloride solution, dried over sodium sulfate, filtered, and concentrated under reduced pressure. The residue was subjected to silica gel column chromatography to obtain 15.82 g of 2-(4-oxocyclohexyl)-2-(4,4,4-trifluorobutylsulfonyl)acetonitrile (hereinafter, referred to as the halogen-containing organosulfur compound (65)) represented by the formula: 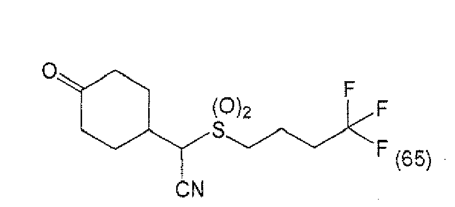 ¹H-NMR(CDC1₃, TMS, δ (ppm)): 1.81-1.99 (2H, m), 2.17-2.64 (10H, m), 2.82-2.95 (1H, m), 3.30-3.89 (2H, m), 3.93 (1H, d).

### Reference Production Example 65

To a solution of 3.11 g of the halogen-containing organosulfur compound (65) in 10 ml of tetrahydrofuran were added 0.87 g of pyridine and 0.92 g of methoxyamine hydrochloride, and the mixture was stirred at room temperature for 3 hours. To the reaction solution was added 30 ml of an aqueous 1N hydrochloric acid solution, followed by extraction with 50 ml of ethyl acetate twice. Organic layers were combined, washed with 50 ml of an aqueous saturated sodium hydrogen carbonate solution and 50 ml of an aqueous saturated sodium chloride solution. The resulting organic layer was dried over sodium sulfate, filtered, and then concentrated under reduced pressure. The residue was subjected to silica gel column chromatography to obtain 3.26 g of 2-[4-(methoxyimino) cyclohexyl]-2-(4,4,4-trifluorobutylsulfonyl) acetonitrile (hereinafter, referred to as the halogen-containing organosulfur compound (66)) represented by the formula: 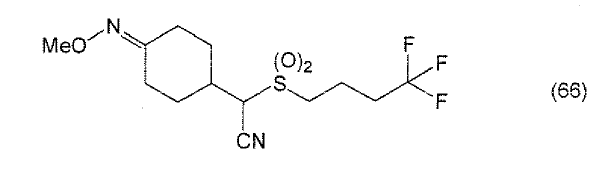 ¹H-NMR(CDC1₃, TMS, δ (ppm)) : 1.49-2.08 (4H, m), 2.14-2.44 (6H, m), 2.47-2.57 (1H, m), 2.59-2.70 (1H, m), 3.28-3.45 (3H, m), 3.80-3.85 (4H, m).

### Reference Production Example 66

According to Reference Production Example 35 except that 1.72 g of ethoxyamine hydrochloride was used in place of hydroxylamine hydrochloride, 2.87 g of 2-[4-(ethoxyimino) cyclohexyl]-2-(4,4,4-trifluorobutylsulfonyl) acetonitrile (hereinafter, referred to as the halogen-containing organosulfur compound (67)) represented by the formula: 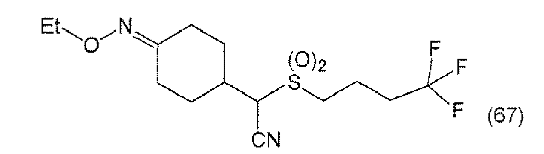 ¹H-NMR (CDC1₃,TMS, δ (ppm) ): 1.25 (3H, t), 1.46-2.07 (4H, m), 2.14-2.43 (6H, m), 2.48-2.56 (1H, m), 2.59-2.70 (1H, m), 3.28-3.44 (3H, m), 3.83 (1H d), 4.05 (2H, q).

### Reference Production Example 67

According to Reference Production Example 29 except that 8.5g of the halogen-containing organosulfur compound (28) and 25 g of tungsten hexachloride was used, 6.1 g of 2-(4,4-dichlorocyclohexyl)-2-(3,3,3-trifluoropropylsulphonyl)acetonitrile (hereinafter, referred to as the halogen-containing organosulfur compound (68)) represented by the formula: 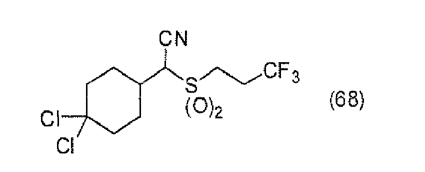 was obtained.
1H-NMR (CDC1₃, TMS, 5 (ppm): 1.85-2.03 (3H, m), 2.18-2.33 (3H, m), 2.45-2.54 (1H, m), 2.62-2.68 (2H, m), 2.74-2.86 (2H, m), 3.39-3.47 (1H, m), 3.54-3.63 (1H, m), 3.88 (1H, d).

More preferable examples of the halogen-containing organosulfur compound represented by the formula (I) include compounds represented by the following formula (II): 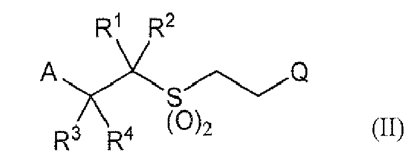 wherein R¹, R², R³, R⁴, A and Q are as defined above. Sepeicifc examples thereof include 2-[4-(methoxyimino)cyclohexyl]-2-(3,3,3-trifluoropropylsulfonyl)acetonitrile (the halogen-containing organosulfur compound (33)), 2-[4-(ethoxyimino) cyclohexyl]-2-(3,3,3-trifluoropropylsulfonyl)acetonitrile (the halogen-containing organosulfur compound (35)), 2-[4-(methoxyimino)cyclohexyl]-3-methyl-2-(3,3,3-trifluoropropylsulfonyl)-4-pentenenitrile (the halogen-containing organosulfur compound (43)), 2-[4-(methoxyimino)cyclohexyl]-3-methyl-2-(3,3,3-trifluoropropylsulfonyl)-4-pentynenitrile (the halogen-containing organosulfur compound (44)), 2-chloro-2-[4-(methoxyimino)cyclohexyl]-2-(3,3,3-trifluoropropylsulfonyl)acetonitrile (the halogen-containing organosulfur compound (45)), 2-[4-(methoxyimino)cyclohexyl]-2-(3,3,4,4,4-pentafluorobutylsulfonyl) acetonitrile (the halogen-containing organosulfur compound (55)) and 2-[4-(ethoxyimino) cyclohexyl]-2-(3,3,4,4,4-pentafluorobutylsulfonyl)acetonitrile (the halogen-containing organosulfur compound (59)).

The insecticidal component of the present composition is not limited to the above described compounds.
The present composition can contain one or more of the above described insecticidal components.

The present composition can control various animal ectoparasites depending on insecticidal components contained in the present composition. Examples of animal ectoparasites which the present composition can control include Siphonaptera insects, such as cat flea *(Ctenocephalides felis),* dog flea *(Ctenocephalides canis),* oriental rat flea *(Xenopsylla cheopis),* human flea *(Pulex irritans),* chigoe *(Tunga penetrans)* and European rat flea *(Nosopsyllus* fasciatus); Anoplura insects, such as Head louse *(Pediculus humanus capitis),* crab louse *(Pthirus pubis*) , short-nosed cattle louse *(Haematopinus eurysternus),* sheep louse *(Dalmalinia ovis),* hog louse *(Haematopinus suis),* long-nosed cattle louse *(Linognathus vituli),* cattle biting louse *(Bovicola bovis),* poultry shaft louse *(Menopon gallinae) ,* poultry body louse *(Menacanthus stramineus),* little blue cattle louse *(Solenopotes capillatus) ,* Haematopinus spp., Linognathus spp., Pediculus spp., Phtirus spp. and Solenopotes spp.; Acarina insects, such as bush tick *(Haemaphysalis longicornis) , Haemaphysalis flava, Dermacentor taiwanicus,* American dog tick *(Dermacentor variahilis), Ixodes ovatus, Ixodes persulcatus,* black legged tick (Ixodes *scapulars),* lone star tick *(Amblyomma americanum) , Boophilus microplus, Rhipicephalus sanguineus, Ixodes holocyclus,* western black legged tick (Ixodes pacificus), *Dermacentor andersoni, Ambryomma maculatum,* ear mite *(Octodectes cynotis),* Psoroptes spp., Chorioptes spp., Otodectes spp., Sarcoptes spp., Notoedres spp., Knemidocoptes spp., Sacroptes scabiei, Demodex spp., folicle mite *(Demodex canis),* northern fowl mite (Ornithonyssus sylviarum), poultry red mite *(Dermanyssus gallinae),* Trombicula spp., *Leptotrombidium* akamushi, *Ornithodorus hermsi, Ornithodorus turicata, Ornithanyssus* bacoti, Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Cytodites spp. and Laminosioptes spp.; Heteroptera insects, such as common bedbug *(Cimex lectularius),* tropical bedbug (Cimex *hemipterus), Reduvius senilis,* Triatoma spp. Rhodnius spp., Panstrongylus spp., and *Arilus critatus;* and Mallophage (Amblycera and Ischnocera) insects, such as Trimenopon spp., Menopon spp., Trinoton spp., Bovicola spp., Werneckiella spp., Lepikentron spp., Trichodectes spp. and Felicola spp.. Preferred are Siphonaptera, Anoplura and Acarina insects.

Examples of a target animal to which the present composition is administered include animals which become hosts of the above-mentioned animal ectoparasites and generally include, in addition to a human being, homeothermic animals and heterothermic animals being reared as livestock or pets.
Examples of the homeothermic animals include a human being and nonhuman mammals. Examples of the nonhuman mammals include a cow, a sheep, a goat, a pig, a camel, a deer, a horse, a rabbit, a dog, a cat, a ferret, a buffalo, a donkey, a fallow deer, a reindeer, a mouse, a rat, a hamster, a squirrel and a monkey, and fur-bearing animals such as a mink, a chinchilla and a raccoon; and avian species such as a chicken, a goosey, a turkey, a duck, a pigeon, a parrot and a quail. Examples of the heterothermic animals include reptiles, such as a tortoise, a sea turtle, *Trachemys scripta, Mauremys japonica,* a lizard, a iguana, a chameleon, a gecko lizard, a rock snake, a Japanese four-lined snake and a cobra. The target animal is preferably a homeothermic animal, more preferably a mammal such as a dog, a cat, a cow, a horse, a pig or a sheep, more preferably a dog or a cat.

An effective amount of the present composition can be administered to an animal to control an animal ectoparasite therapeutically, suppressively, preventively and protectively. The present composition can systemically or topically suppress an animal ectoparasite, and can be used during all stages or one stage of infestation of an animal ectoparasite.

The present composition may be a simple mixture of an insecticidal component and an adipate. The present composition is usually used in the form of a formulation such as a liquid formulation, and is preferably used as an oral formulation, an external formulation for skin or an injectable formulation.
The formulation can be produced by a known method, if necessary, using conventional additives or auxiliary agents as described below.

In the present composition, the total content of the insecticidal component and the adipate is 0.1 to 100% by weight, preferably 10 to 100% by weight, more preferably 30 to 100% by weight, depending on the form of the formulation.

When the present composition is administered in the form of an oral formulation to an animal, examples of the formulation include animal feed, an animal feed premix, an animal feed concentrate, a pill, a liquid formulation, a paste, a suspension, a water-based formulation, a gel, a tablet, a bolus, and a capsule.

When the present composition is administered in the form of an external formulation for skin to an animal, examples of the formulation include a dipping formulation, a dust, a powder, a spray, a shampoo, a liquid formulation, an ointment and an oil-in-water or water-in-oil type emulsion. The present composition can be administered by spot-on treatment, pour-on treatment, immersion, spraying, bathing, washing, rubbing, or dispersion.

When the present composition is administered in the form of an injectable formulation to an animal, the present composition can be injected intraruminally, intramuscularly, intravenously or subcutaneously. Preferable injection methods include a spot-on treatment method and a pour-on treatment method. The spot-on treatment method usually comprises dropping or applying a liquid type formulation to the skin of the head part, shoulder blade or dorsal region of a host animal. The pour-on treatment method usually comprises pouring a liquid type formulation along the middorsal line of a host animal body.

The dosage amount of the present composition to an animal is usually 1 to 5000 mg, preferably 10 to 1000 mg, more preferably 50 to 500 mg per kg of the living body weight of a. target animal, although it may be varied depending on the kind of a target animal or an ectoparasite to be controlled.

The present composition may contain only an insecticidal component and an adipate, or if necessary, may further contain an auxiliary agent such as an auxiliary solvent, an antioxidant, a colorant, a light stabilizer or a thickener.

Examples of the auxiliary agent include alcohols and glycols such as ethanol, isopropyl alcohol, benzyl alcohol, ethylene glycol, propylene glycol and phenoxyethanol; ethers such as ethylene glycol dimethyl ether, diethylene glycol monomethyl ether, diethylene glycol monoethyl ether, propylene glycol monomethyl ether, dipropylene glycol monomethyl ether, dipropylene glycol monoethyl ether and 3-methoxy-3-methyl-1-butanol; amides such as N,N-dimethylformamide and N,N-dimethylacetamide; alkylpyrrolidones such as N-methyl-2-pyrrolidane and N-octyl-2-pyrrolidone; fatty acid esters such as isopropyl myristate; y-butyrolactone, propylene carbonate, water, paraffin oils, silicone oils, triglycerides, surfactants, and their mixtures.

Examples of the surfactant include nonionic surfactants, amphoteric surfactants, anionic surfactants and cationic surfactants. Examples of the nonionic surfactant include sorbitan fatty acid esters such as sorbitan stearate and sorbitan oleate; glycerin fatty acid esters such as glyceryl stearate, glyceryl isostearate, glyceryl oleate, polyglyceryl stearate, polyglyceryl isostearate and polyglyceryl oleate; polyoxyethylene alkyl ethers such as polyoxyethylene lauryl ether, polyoxyethylene cetyl ether, polyoxyethylene stearyl ether, polyoxyethylene oleyl ether and polyoxyethylene styryl phenyl ether; polyoxyethylene sorbitan fatty acid esters such as polyoxyethylene sorbitan coconut oil fatty acid ester, polyoxyethylene sorbitan oleate and polyoxyethylene sorbitan stearate; polyoxyethylene sorbitol fatty acid esters such as polyoxyethylene sorbitol tetraoleate; polyoxyethylene hydrogenated castor oil and alkylphenol polyglycol ethers. Examples of the amphoteric surfactant include betaine such as lauryl betaine and stearyl betaine; imidazoline derivatives such as di-sodium N-lauryl-p-iminodipropionate; and lecithin. Examples of the anionic surfactant include alkyl sulfates such as sodium lauryl sulfate and triethanolamine lauryl sulfate; polyoxyethylene alkyl ether sulfates such as sodium polyoxyethylene lauryl ether sulfate and triethanolamine polyoxyethylene lauryl ether sulfate; alkylbenzenesulfonic acid salts such as sodium dodecylbenzenesulfonate; and polyoxyethylene alkyl ether phosphoric acid salts such as sodium di(polyoxyethylene)lauryl ether phosphate and sodium di;palyoxyethylene)oleyl ether phosphate. Examples of the cationic surfactant include alkylammonium salts such as cetyltrimethylammonium chloride and distearyldimethylammonium chloride.

Examples of the silicone oil include dimethyl silicone oil, high polymerization degree dimethyl silicone oil, cyclic silicone oil, polyether-modified silicone oil, amino-modified silicone oil and methyl phenyl silicone oil.

Examples of the antioxidant include BHT (butylated hydroxytoluene) and BHA (butylated hydroxyanisole). Examples of the colorant include food tar dyes such as Food Red No.2 (Amaranth), Food Red No.3 (Erythrosine), Food Yellow No.4 (Tartrazine), Food Green No.3 (Fast green FCF) and Food Blue No.1 (Brilliant Blue FGF). Examples of the light sensitizer include benzophenone compounds. Examples of the thickener include bentonite, colloidal silicic acids, cellulose derivatives, starch derivatives, polyacrylates, natural polymers, alginates and gelatin.

Animal ectoparasites can be controlled by using the present composition.

### EXAMPLES

Hereinafter, the present invention will be described in detail with reference to Formulation Examples and Test Examples which the present invention is not limited to.
First, Formulation Examples of the present composition will be described. In Examples, the "part(s)" means part(s) by mass unless otherwise specified.

### Formulation Example 1

To 20 g of one compound selected from the halogen-containing organosulfur compounds (1) to (68) is added 80 g of diisopropyl adipate and then mixed to obtain a liquid formulation.

### Formulation Example 2

To 20 g of one compound selected from the halogen-containing organosulfur compounds (1) to (68) is added 80 g of diisobutyl adipate and then mixed to obtain a liquid formulation.

### Formulation Example 3

To a mixture of 20 g of one compound selected from the halogen-containing organosulfur compounds (1) to (68), 1 g of pyriproxyfen and 0.3 g of BHT is added 39.35 g of diisopropyl adipate and 39.35 g of diethylene glycol monoethyl ether, and then and then mixed to obtain a liquid formulation.

### Formulation Example 4

To a mixture of 20 g of one compound selected from the halogen-containing organosulfur compounds (1) to (68), 1 g of pyriproxyfen and 0.3 g of BHT is added 39.35 g of diisobutyl adipate and 39.35 g of diethylene glycol monoethyl ether, then mixed to obtain a liquid formulation.

### Formulation Example 5

To a mixture of 20 g of one compound selected from the halogen-containing organosulfur compounds (1) to (68), 1 g of pyriproxyfen, 10 g of silicone oil (dimethyl silicone oil) and 0.3 g of BHT are added 34.35 g of diisapropyl adipate and 34.35 g of dipropylene glycol monoethyl ether, and then mixed to obtain a liquid formulation.

### Formulation Example 6

To a mixture of 20 g of one compound selected from the halogen-containing organosulfur compounds (1) to (68), 1 g of pyriproxyfen, 10 g of silicone oil (dimethyl silicone oil) and 0.3 g of BHT are added 34.35 g of diisobutyl adipate and 34.35 g of dipropylene glycol monoethyl ether, and then mixed to obtain a liquid formulation.

### Formulation Example 7

To a mixture of 20 g of one compound selected from the halogen-containing organosulfur compounds (1) to (68), 1 g of pyriproxyfen, 10 g of silicone oil (polyether-modified silicone oil) and 0.3 g of BHT are added 34.35 g of diisopropyl adipate and 34.35 g of dipropylene glycol monoethyl ether, and then mixed to obtain a liquid formulation.

### Formulation Example 8

To a mixture of 20 g of one compound selected from the halogen-containing organosulfur compounds (1) to (68), g of pyriproxyfen, 10 g of silicone oil (polyether-modified silicone oil) and 0.3 g of BHT are added 34.35 g of diisobutyl adipate and 34.35 g of dipropylene glycol monoethyl ether, and then mixed to obtain a liquid formulation.

### Formulation Example 9

To a mixture of 20 g of one compound selected from the halogen-containing organosulfur compounds (1) to (68), 1 g of pyriproxyfen and 0.3 g of BHT are added 36.85 g of diisopropyl adipate, 36.85 g of dipropylene glycol monoethyl ether and 5 g of triglyceride, and then mixed to obtain a liquid formulation.

### Formulation Example 10

To a mixture of 20 g of one compound selected from the halogen-containing organosulfur compounds (1) to (68), 1 g of pyriproxyfen and 0.3 g of BHT are added 36.85 g of diisobutyl adipate, 36.85 g of dipropylene glycol monoethyl ether and 5 g of triglyceride, and then mixed to obtain a liquid formulation.

### Formulation Example 11

To a mixture of 20 g of one compound selected from the halogen-containing organosulfur compounds (1) to (68), 1 g of pyriproxyfen and 0.3 g of BHT are added 39.35 g of diisopropyl adipate and 39.35 g of dipropylene glycol monoethyl ether, and then mixed to obtain a liquid formulation.

### Formulation Example 12

To a mixture of 20 g of one compound selected from the halogen-containing organosulfur compounds (1) to (68), 1 g of pyriproxyfen and 0.3 g of BHT are added 39.35 g of diisobutyl adipate and 39.35 g of dipropylene glycol monoethyl ether, and then mixed to obtain a liquid formulation.

### Formulation Example 13

To a mixture of 20 g of one compound selected from the halogen-containing organosulfur compounds (1) to (68), 1 g of pyriproxyfen and 0.3 g of BHT are added 39.35 g of diisopropyl adipate and 39.35 g of γ-butyrolactone, and then mixed to obtain a liquid formulation.

### Formulation Example 14

To a mixture of 20 g of one compound selected from the halogen-containing organosulfur compounds (1) to (68), 1 g of pyriproxyfen and 0.3 g of BHT are added 39.35 g of diisobutyl adipate and 39.35 g of γ-butyrolactone, and then mixed to obtain a liquid formulation.

### Formulation Example 15

To a mixture of 20 g of one compound selected from the halogen-containing organosulfur compounds (1) to (68), 10 g of metofluthrin, 1 g of pyriproxyfen and 0.3 g of BHT are added 34.35 g of diisopropyl adipate and 34.35 g of y-butyrolactone, and then mixed to obtain a liquid formulation.

### Formulation Example 16

To a mixture of 20 g of one compound selected from the halogen-containing organosulfur compounds (1) to (68) , 10 g of metofluthrin, 1 g of pyriproxyfen and 0.3 g of BHT are added 34.35 g of diisobutyl adipate and 34.35 g of γ-butyrolactone, and then mixed to obtain a liquid formulation.

### Formulation Example 17

To a mixture of 5 g of one compound selected from the halogen-containing organosulfur compounds (1) to (68) and 4 g of metofluthrin are added 37.5 g of diisopropyl adipate and 37.5 g of N,N-dimethylformamide, and then mixed. To the mixture are added 10 g of polyoxyethylene styryl phenyl ether and 6 g of calcium dodecylbenzenesulfonate, and then stirred and mixed thoroughly to obtain an emulsifiable concentrate.

### Formulation Example 18

To a mixture of 5 g of one compound selected from the halogen-containing organosulfur compounds (1) to (68) and 4 g of metofluthrin, are added 37.5 g of diisobutyl adipate and 37.5 g of N,N-dimethylformamide, and then mixed. To the mixture are added 10 g of polyoxyethylene styryl phenyl ether and 6 g of calcium dodecylbenzenesulfonate, and then stirred and mixed thoroughly to obtain an emulsifiable concentrate.

### Formulation Example 19

To 0.5 g of one compound selected from the halogen-containing organosulfur compounds (1) to (68) are added 10 g of diisopropyl adipate, 50 g of Nikkol TEALS-42 (a product name of a 42% aqueous solution of triethanolamine lauryl sulfate manufactured by Nikko Chemicals Co., Ltd.) and 20 g of propylene glycol, and then stirred and mixed thoroughly to obtain a uniform solution. Then, 19.5 g of water is added thereto, and further stirred and mixed thoroughly to obtain a shampoo formulation as a uniform solution.

### Formulation Example 20

To 0.5 g of one compound selected from the halogen-containing organosuifur compounds (1) to (68) are added 10 g of diisobutyl adipate, 50 g of Nikkol TEALS-42 (a product name of a 42% aqueous solution of triethanolamine lauryl sulfate manufactured by Nikko Chemicals Co., Ltd.) and 20 g of propylene glycol, and then stirred and mixed thoroughly to obtain a uniform solution. Then, 19.5 g of water is added thereto, and further stirred and mixed thoroughly to obtain a shampoo formulation as a uniform solution.

### Formulation Example 21

To 0.1 g of one compound selected from the halogen-containing organosulfur compounds (1) to (68) is added 0.4 g of diisopropyl adipate, and mixed. The mixture was stuffed in a capsule of hydroxypropyl methyl cellulose to obtain a capsule formulation.

### Formulation Example 22

To 0.1 g of one compound selected from the halogen-containing organosulfur compounds (1.) to (68) is added 0.4 g of diisobutyl adipate, and mixed. The mixture was stuffed in a capsule of hydroxypropyl methyl cellulose to obtain a capsule formulation.

### Formulation Example 23

To 20 g of one compound selected from the halogen-containing organosulfur compounds (1) to (68) are added 10 g of diisopropyl adipate and 70 g of diethylene glycol monoethyl ether, and then mixed to obtain a liquid formulation.

### Formulation Example 24

To 20 g of one compound selected from the halogen-containing organosulfur compounds (1) to (68) are added 10 g of diisobutyl adipate and 70 g of diethylene glycol monoethyl ether, and then mixed to obtain a liquid formulation.

### Formulation Example 25

To 20 g of one compound selected from the halogen-containing organosulfur compounds (1) to (68) are added 40 g of diisopropyl adipate and 40 g of diethylene glycol monoethyl ether, and then mixed to obtain a liquid formulation.

### Formulation Example 26

To 20 g of one compound selected from the halogen-containing organosulfur compounds (1) to (68) are added 40 g of diisobutyl adipate and 40 g of diethylene glycol monoethyl ether, and then mixed to obtain a liquid formulation.

### Formulation Example 27

To 20 g of one compound selected from the halogen-containing organosulfur compounds (1) to (68) are added 10 g of diisopropyl adipate and 70 g of propylene carbonate, and then mixed to obtain a liquid formulation.

### Formulation Example 28

To 20 g of one compound selected from the halogen-containing organosulfur compounds (1) to (68) are added 10 g of diisobutyl adipate and 70 g of propylene carbonate, and then mixed to obtain a liquid formulation.

### Formulation Example 29

To 20 g of one compound selected from the halogen-containing organosulfur compounds (1) to (68) are added 40 g of diisopropyl adipate and 40 g of propylene carbonate, and then mixed to obtain a liquid formulation.

### Formulation Example 30

To 20 g of one compound selected from the halogen-containing organosulfur compounds (1) to (68) are added 40 g of diisobutyl adipate and 40 g of propylene carbonate, and then mixed to obtain a liquid formulation.

### Formulation Example 31

To 20 g of one compound selected from the halogen-containing organosulfur compounds (1) to (68) are added 10 g of diisopropyl adipate and 70 g of isopropyl myristate, and then mixed to obtain a liquid formulation.

### Formulation Example 32

To 20 g of one compound selected from the halogen-containing organosulfur compounds (1) to (68) are added 10 g of diisobutyl adipate and 70 g of isopropyl myristate, and then mixed to obtain a liquid formulation.

### Formulation Example 33

To 20 g of one compound selected from the halogen-containing organosulfur compounds (1) to (68) are added 40 g of diisopropyl adipate and 40 g of isopropyl myristate, and then mixed to obtain a liquid formulation.

### Formulation Example 34

To 20 g of one compound selected from the halogen-containing organosulfur compounds (1) to (68) are added 40 g of diisobutyl adipate and 40 g of isopropyl myristate, and then mixed to obtain a liquid formulation.

The following Test Examples show that the present composition has an excellent controlling effect on an animal ectoparasite.

### Test Example 1

Any one of the halogen-containing organosulfur compounds (33), (35), (43), (44), (45), (55) and (59) was dissolved in diisopropyl adipate such that the concentration of the halogen-containing organosulfur compound was 19.2 mg/ml to obtain the present composition (33), (35), (43), (44), (45), (55) or (59). A filter paper was put on the bottom face of a glass petri dish with 7 cm diameter and treated with 0.1 ml of the present composition. On the filter paper, 10 *Haernaphysalis longicornis* were released and allowed to contact the filter paper for 2 hours. Thereafter, the insects were transferred to another container. The container was allowd to stand still at 25°C. After 24 hours, fatalities of the insects were observed.
As a result, the present compositions (33), (35), (43), (44), (45), (55), and (59) showed a high lethal effect on *Haemaphysalis longicornis.*

### Test Example 2

### Dropwise administration test to dog-parasitizing

### Naemaphysalis longicornis

The day before a dropwise administration test, dogs (beagle) were infected with each 50 ticks *(Haemaphysalis longicornis,* nymphal ticks). The number of ticks parasitizing the dog was counted before dropwise administration.
The halogen-containing organosulfur compound (33) was dissolved in diethylene glycol monoethyl ether (referred to as EDG in Table 1) or diisobutyl adipate in weight ratios (%) as shown in Table 1 to prepare a test solution. The test solution was directly administered dropwise onto the skin of the dog at a dose of 0.1 ml per kg of the body weight after the hair of the neck and back was shoved aside. Dogs of a test group were subjected to the above treatment.
On the other hand, dogs of a placebo group were subjected to dropwise administration of only ethylene glycol monoethyl ether.
The number of living ticks parasitizing the dogs was observed on 1st day and 2nd day after administration. On completion of the observation on 2nd day after administration, all of parasitizing ticks were removed from the dogs. The above test was repeated three times for each group. A parasitization rate and a disinfestation rate were determined according to the following equations.
Calculating methods of a parasitization rate and a disinfestation rate in the initial stage of administration (1st day and 2nd day):
Parasitization rate (%) on Xₜₕ day = [(number of living ticks on Xₜₕ day) / (number of living ticks before administration)] x 100
Disinfestation rate (%) on Xₜₕ day = {[(parasitization rate of test group before administration) - (parasitization rate of test group on Xₜₕ day)] / (parasitization rate of test group before administration)} x 100

### Test results are shown in Table 2.

**Table 1**

| | Weight ratio (% | | |
|---|---|---|---|
| | Compound (33) | EDG | Diisobutyl adipate |
| Formulation 1 | 20 | 80 | 0 |
| Formulation 2 | 20 | 0 | 80 |

**Table 2**

| | Disinfestation rate of ticks (%) | |
|---|---|---|
| | 1st day | 2nd day |
| Formulation 1 | 12 | 49 |
| Formulation 2 | 94 | 96 |

### Test Example 3

### Dropwise administration test to dog-parasitizing

### Dermacentor variabilis

The day before a dropwise administration test, dogs (beagle) were infected with each 50 ticks (Dermacentar *variabilis,* adult ticks). The number of ticks parasitizing the dog was counted before dropwise administration.
The halogen-containing organosulfur compound (33) and pyriproxyfen were dissolved in y-butyrolactone (referred to as BLO in Table 3) and diisopropyl adipate and/or POE sorbitol tetraoleate in weight ratios (%) as shown in Table 3 to prepare a test solution. The test solution was directly administered dropwise onto the skin of the dog at a dose of 0.1 ml per kg of the body weight after the hair of the neck and back was shoved aside. Dogs of a test group were subjected to the above treatment.
On the other hand, dogs of a placebo group were subjected to dropwise administration of only ethylene glycol monoethyl ether.
On 14th day and on 28th day after administration, the dogs were reinfected with each 50 ticks (Dermacentor *variabilis,* adult ticks). On 2nd day after each reinjection, the number of living ticks parasitizing the dogs was observed. On completion of the observation on 2nd day after reinfection, all of parasitizing ticks were removed from the dogs. The above test was repeated three times for each group. A parasitization rate and a disinfestation rate were determined according to the following equations.
Calculating methods of a parasitization rate and a Disinfestation rate after infection on 14th day and 28th day after administration:
Parasitization rate (%) on Xₜₕ day = [(number of living ticks on Xₜₕ day) / (number of tested ticks)] x 100 Disinfestation rate (%) on Xₜₕ day = {[(parasitization rate of placebo group on Xₜₕ day) - (parasitization rate of study group on Xₜₕ day)] / (parasitization rate of placebo group on Xₜₕ day) } x 100
In the case where the parasitization rate of the test group was higher than that of the placebo group, the parasitization rate of the test group was defined to be 0%.

### Test results are shown in Table 4.

**Table 3**

| | Weight ratio (%) | | | | |
|---|---|---|---|---|---|
| | Compound (33) | Pyriproxyfen | BLO | POE sorbitol tetraoleate | Diisopropyl adipate |
| Formulation 3 | 20 | 1 | 69 | 10 | 0 |
| Formulation 4 | 20 | 1 | 29 | 10 | 40 |

**Table 4**

| | Disinfestation rate (%) | |
|---|---|---|
| | *Dermacentor variabilis* | |
| | 16th day | 30th day |
| Formulation 3 | 68 | 44 |
| Formulation 4 | 100 | 84 |

### Test Example 4

### Dropwise administration test to dog-parasitizing

### Haemaphysalis longicornis

The day before a dropwise administration test, dogs (beagle) were infected with each 10 ticks *(Haemaphysalis longicornis,* nymphal ticks). The number of ticks parasitizing the dog was counted before dropwise administration.
The halogen-containing organosulfur compound (33) and pyriproxyfen were dissolved in diethylene glycol monoethyl ether (referred to as EDG in Table 5), diisopropyl adipate and/or diisobutyl adipate in weight ratios (%) as shown in Table 5 to prepare a test solution. The test solution was directly administered dropwise onto the skin of the dog at a dose of 0.1 ml per kg of the body weight after the hair of the neck and back was shoved aside. Dogs of a test group were subjected to the above treatment.
On the other hand, dogs of a placebo group were subjected to dropwise administration of only ethylene glycol monoethyl ether.
On 14th day after administration, the dogs were reinfected with each 100 ticks *(Haemaphysalis longicornis,* nymphal ticks). On 2nd day after reinfection, the number of living ticks parasitizing the dogs was observed. The above test was repeated three times for each group. A parasitization rate and a disinfestation rate were determined according to the following equations.
Calculating methods of a parasitization rate and a disinfestation rate after infection on 14th day after administration:
Parasitization rate (%) on Xₜₕ day = [(number of living ticks on Xₜₕ day) / (number of tested ticks)] x 100 Disinfestation rate (%) on Xₜₕ day ={[ (parasitization rate of placebo group on Xₜₕ day) - (parasitization rate of test group on Xₜᵣ day)] ] / (parasitization rate of placebo group on _{Xth} day) } x 100
In the case where the parasitization rate of the test group was higher than that of the placebo group, the parasitization rate of the test group was defined to be 0%.

### Test results are shown in Table 6.

**Table 5**

| | Weight ratio (%) | | | | |
|---|---|---|---|---|---|
| | Compound (33) | pyriproxyfen | EDG | Diisooropyl adipate | Diisobutyl adapate |
| Formulations 5 | 20 | 1 | 69 | 10 | 0 |
| Formulation 6 | 20 | 1 | 39 | 40 | 0 |
| Formulation 7 | 20 | 1 | 0 | 79 | 0 |
| Formulation 8 | 20 | 1 | 69 | 0 | 10 |
| Formulation 9 | 20 | 1 | 39 | 0 | 40 |
| Formulation 10 | 20 | 1 | 0 | 0 | 79 |

**Table 6**

| | tick disinfestation rate(%) |
|---|---|
| | 16th day |
| Formulation 5 | 100 |
| Formulation 6 | 100 |
| Formulation 7 | 100 |
| Formulation 8 | 100 |
| Formulation 9 | 100 |
| Formulation 10 | 100 |

### Test Example 5

### Dropwise administration test to cat-parasitizing

### Ctenocephalides felis

The day before a dropwise administration test, cats were infected with each 50 fleas *(Ctenocephalides felis,* adult fleas). The number of fleas parasitizing the dog was counted before dropwise administration.
The halogen-containing organosulfur compound (33) and pyriproxyfen were dissolved in diethylene glycol monoethyl ether (referred to as EDG in Table 7), (diisopropyl adipate and dibutylhydroxytoluene (referred to as BHT in Table 7) in weight ratios (%) as shown in Table 7 to prepare a test solution. The test solution was directly administered dropwise onto the skin of the cat at a dose of 0.1 ml per kg of the body weight after the hair of the neck and back was shoved aside. Cats of a test group were subjected to the above treatment.
On the other hand, cats of a placebo group were subjected to dropwise administration of only ethylene glycol monoethyl ether.
On 14th day and on 28th day after administration, the cats were reinfected with each 50 fleas (Ctenocephalides felis, adult fleas). On 1st day and 2nd day after each reinfection, the number of living fleas parasitizing the cats was observed. On completion of the observation on 2nd day after reinfection, all of parasitizing fleas were removed from the cats. The above test was repeated three times for each group. A parasitization rate and a disinfestation rate were determined according to the following equations.
Calculating methods of a parasitization rate and a disinfestation rate in the initial stage of administration (1st day and 2nd day)
Parasitization rate (%) on Xₜₕ day = [(number of living fleas on Xₜₕ day) / (living fleas before administration)] x 100
Disinfestation rate (%) on Xₜₕ day = {[(parasitization rate of test group before administration) - (parasitization rate of test group on Xₜₕ day)] / (parasitization rate of test group before aciministration)) x 100
Calculating methods of a parasitization rate and a disinfestation rate on 14th day and 28th day after administration:
Parasitization rate (%) on Xₜₕ day = [(number of living fleas on Xₜₕ day) / (number of tested fleas)] x 100 Disinfestation rate (%) on Xₜₕ day = {[(parasitization rate of placebo group on Xₜₕ day) - (parasitization rate of test group on Xₜₕ day)] / (parasitization rate of placebo group on Xₜₕ day) } x 100
In the case where the parasitization rate of the test group was higher than that of the placebo group, the parasitization rate of the test group was defined to be 0%.

### Test results are shown in Table 8.

**Table 7**

| | Weight Ratio (%) | | | | |
|---|---|---|---|---|---|
| | Compound(33) | pyriproxyfen | EDG | Diisopropyl adipate | BHT |
| Formulation 11 | 20 | 1 | 39.35 | 39.35 | 0.3 |

**Table 8**

| | Disinfestation rate of *Ctenocephalides felis* (%) | | | | | |
|---|---|---|---|---|---|---|
| | 1st day | 2nd day | 15th day | 16th day | 29th day | 30th day |
| Formulation 11 | 100 | 100 | 100 | 100 | 94 | 97 |

### Test Example 6

The halogen-containing organosulfur compound (33) or (35) was dissolved in a solvent such that the concentration of the halogen-containing organosulfur compound was 63.6 mg/ml to a test solution. A filter paper was put on the bottom face of a glass petri dish with 7 cm diameter and treated with 0.2 ml of the test solution (treatment amount: 2000 mg/m2). On the filter paper, 10 *Haemaphysalis longicornis* were released and allowed to contact the filter paper. The glass petri dish was allowd to stand still at 25°C. After 24 hours, fatalities of the insects were observed.

### Test results are shown in Table 9.

**Table 9**

| | Fatality rate of tick (%) | | |
|---|---|---|---|
| | Example 1 | Example 2 | Comparative Example 1 |
| | diisopropyl adipate | diisobutyl adipate | Propylene Carbonate |
| Compound (33) | 100 | 85 | 25 |
| Compound (35) | 100 | 80 | 20 |
| Only solvent | 0 | 5 | 10 |

### Test Example 7

The halogen-containing organosulfur compound (33) or (35) was mixed with a mixture of diisopropyl adipate and diethylene glycol monoethyl ether such that the concentration of the halogen-containing organosulfur compound was 10%w/w to prepare a test solution. A filter paper was put on the bottom face of a glass petri dish with 9 cm diameter and treated with 0.2 ml of the test solution. On the filter paper, 10 *Haemaphysalis longicornis* were released and allowed to contact the filter paper. The glass petri dish was allowd to stand still at 25°C. After 5 hours, fatalities of the insects were observed.

### Test results are shown in Table 10 and Table 11.

**Table 10**

| Weight ratio (%) | | | | Fatality rate of tick (%) |
|---|---|---|---|---|
| Compound (33) | | diisopropyl adipate | diethylene glycol monoethyl ether | |
| Comparative Example 2 | 10 | 1 | 89 | 30 |
| Example 3 | 10 | 2 | 88 | 60 |
| Example 4 | 10 | 5 | 85 | 90 |
| Example 5 | 10 | 10 | 80 | 100 |
| Example 6 | 10 | 20 | 70 | 100 |

**Table 11**

| Weight ratio (%) | | | | Fatality rate of tick (%) |
|---|---|---|---|---|
| Compound (35) | | diisopropyl adipate | diethylene glycol monoethyl ether | |
| Comparative Example 3 | 10 | 1 | 89 | 40 |
| Example 7 | 10 | 2 | 88 | 95 |
| Example 8 | 10 | 5 | 85 | 95 |
| Example 9 | 10 | 10 | 80 | 100 |
| Example 10 | 10 | 20 | 70 | 100 |

Animal ectoparasites can be controlled efficiently by using the animal ectoparasite control present composition of the present invention.

## Claims

1. An animal ectoparasite control composition containing an insecticidal component and an adipate.

2. The composition according to claim 1, wherein the adipate is at least one selected from the group consisting of diisopropyl adipate and diisobutyl adipate.

3. The composition according to claim 1 or 2, wherein the weight ratio of the insecticidal component to the adipate is 1 : 0.2 to 1 : 500, preferably 1 : 0.2 to 1 : 50, more preferably 1 : 0.5 to 1 : 50.

4. The composition according to any one of claims 1 to 3, wherein the total content of the insecticidal component and the adipate is 0. 1. to 100% by weight, preferably 10 to 100% by weight, more preferably 30 to 100% by weight.

5. The composition according to any one of claims 1 to 4, wherein the insecticidal component is at least one compound selected from the group consisting of halogen-containing organosulfur compounds, pyrethroid compounds, neonicotinoid compounds, organophosphorus compounds, insect growth regulation active compounds, phenylpyrazole compounds, carbamate compounds, and benzoylurea compounds.

6. The composition according to claim 5, wherein the halogen-containing organosulfur compound is represented by the following formula (I): 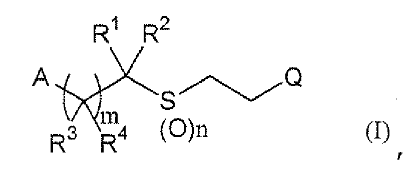 wherein m represents 0 or 1, n represents 0, 1 or 2,
A represents a C3-C7 cycloalkyl group optionally substituted with a group selected from the groups EI to E3, or a C5-C7 cycloalkenyl group optionally substituted with a group selected from the groups E1 to E3;
Q represents a C1-C3 haloalkyl group containing at least one fluorine atom, or a fluorine atom;
R¹ and R³ are the same as or different from each other, and represent a C1-C4 chain hydrocarbon group optionally substituted with a halogen atom, a halogen atom, or a hydrogen atom;
R² and R⁴ are the same as or different from each other, and represent a C1-C4 chain hydrocarbon group optionally substituted with a halogen atom, -C(=G)R⁵, a cyano group, a halogen atom, or a hydrogen atom;
G represents an oxygen atom or a sulfur atom;
R⁵ represents a C1-C4 alkyl group optionally substituted with a halogen atom, a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkylamino group optionally substituted with a halogen atom, a C2-C5 cyclic amino group, or a hydrogen atom;
the group E1 is a group of monovalent substituents consisting of a C1-C6 chain hydrocarbon group optionally substituted with a group selected from the group L, a C3-C6 cycloalkyl group optionally substituted with a halogen atom, -OR⁶, -SR⁶, -S(=O)R⁶, -S(=O)₂R⁶ -C(=O)R⁷, -OC (=O)R⁸ , ₐ halogen atom, a cyano group, and a hydroxyl group;
the group E2 is a group of bivalent substituents of which two valences are derived from one atom, consisting of =O, =NO-R⁶, =C=CH₂, and =C(R¹¹)R¹²;
the group E3 is a group of bivalent substituents of which two valences are derived from different atoms, consisting of a C2-C6 alkylene group optionally substituted with a group selected from the group L, a C4-C6 alkenylene group optionally substituted with a group selected from the group L, -G-T¹_G-, and -G-T¹-G-T²_; wherein T¹ and T² are the same as or different from each other, and represent a methylene group or an ethylene group;
the group L consists of a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, -N(R⁹)R^{1O}, a C2-C5 cyclic amino group, -C(=O)R⁷, -OC(=O)R⁸ and a halogen atom;
R⁶ represents a C1-C4 chain hydrocarbon group optionally substituted with a halogen atom, or a C3-C6 cycloalkyl group optionally substituted with a halogen atom;
R⁷ represents a hydroxyl group, a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkyl)amino group optionally substituted with a halogen atom, a C2-C5 cyclic amino group, a C1-C4 alkyl group optionally substituted with a halogen atom, or a hydrogen atom;
R⁸ represents a C1-C4 alkoxy group optionally substituted with a halogen atom, a C3-C6 alkenyloxy group optionally substituted with a halogen atom, a C3-C6 alkynyloxy group optionally substituted with a halogen atom, an amino group, a C1-C4 alkylamino group optionally substituted with a halogen atom, a di(C1-C4 alkyl)amino group optionally substituted with a halogen atom, a C2-C5 cyclic amino group, a C1-C4 alkyl group optionally substituted with a halogen atom, or a hydrogen atom;
R⁹ and R¹⁰ are the same as or different from each other, and represent a C1-C4 alkyl group optionally substituted with a halogen atom, a C3-C6 alkenyl group optionally substituted with a halogen atom, a C3-C6 alkynyl group optionally substituted with a halogen atom, a C3-C6 cycloalkyl group optionally substituted with a halogen atom, a phenyl group optionally substituted with a halogen atom, or a hydrogen atom; and
R¹¹ and R¹² are the same as or different from each other, and represent a C1-C4 alkoxy group optionally substituted with a halogen atoms, a C1-C4 chain hydrocarbon group optionally substituted with a halogen atom, a halogen atom, or a hydrogen atom.

7. The composition according to claim 6, wherein m is 0, n is 2, R² is a hydrogen atom, a C1-C4 alkyl group or a cyano group.

8. The composition according to any one of claim 6, wherein R² is -C(=G)R⁵; G is an oxygen atom; and R⁵ is an amino group, a C1-C4 alkylamino group optionally substituted with a halogen atom, a di(C1-C4 alkyl)amino group optionally substituted with a halogen atom, or a C2-C5 cyclic amino group.

9. The composition according to claim 6, wherein R¹ is a hydrogen atom or a C1-C4 alkyl group optionally substituted with a halogen atom, or R¹ is a halogen atom.

10. The composition according to claim 6, wherein A is a cyclohexyl group optionally substituted with a group selected from the groups E1 to E3.

11. The composition according to claim 6, wherein the group selected from the groups E1 to E3 is =NO-R⁶ and R6 its a C1-C4 chain hydrocarbon group optionally substituted with a halogen atom.

12. The composition according to claim 6, wherein A is a cyclohexyl group substituted with one or more of halogen atoms.

13. The composition according to any one of claims 1 to 12, which is in the form of a liquid formulation, an oral formulation, an external formulation for skin or an injectable formulation, and is for controlling an animal ectoparasite of Siphonaptera, Anoplura, or Acarina.

14. A method of controlling an animal ectoparasite, which comprises administering an effective amount, preferably 1 to 5000 mg, more preferably 10 to 1000 mg, still more preferably 50 to 500 mg per kg of the living body weight of a target animal of the composition according to any one of claims 1 to 13 to the animal.

15. The method according to claim 14, wherein the administration of the composition to the animal is carried out by a spot-on treatment or a pour-on treatment.

16. The method according to claim 14 or 15, wherein the target animal is a nonhuman mammal, preferably a dog, a cat, a cow, a horse, a pig, or a sheep, more preferably a dog or a cat.
